# EUROPEAN PATENT APPLICATION

(11) **EP 4 378 921 A1**
(43) Date of publication of application: **05.06.2024**
(21) Application number: 22848576.9
(22) Date of filing: 27.07.2022
(51) Int. Cl.: C07C 219/00, C07C 229/00, A61K 48/00

(54) **USE OF FA-TYPE LIPID COMPOUNDS IN PREPARATION OF NUCLEIC ACID DELIVERY REAGENT AND RELATED PRODUCT**

(30) Priority: 27.07.2021 CN 202110862410
(71) Applicant: Institute of Basic Medical Sciences Chinese Academy of Medical Sciences, Beijing 100005 (CN)
(72) Inventor: JIANG, Chengyu, Beijing 100005 (CN); MA, Yiming, Beijing 100005 (CN); XU, Longxin, Beijing 100005 (CN)
(74) Representative: Simmons & Simmons LLP (Munich)
(86) International application number: PCT/CN2022/108245
(87) International publication number: WO 2023/005971

(57) **Abstract**

A use of one or more lipid compounds in the delivery of nucleic acids, and a lipid nucleic acid mixture, pharmaceutical composition or kit comprising the lipid compounds and nucleic acids. The lipid compounds provided above can promote the absorption, particularly oral absorption, of nucleic acids, and promote the entry of nucleic acids into target sites in a subject in need thereof.

## Description

### FIELD

The present invention relates to the field of biology. Specifically, the present application relates to the use of a lipid compound for delivering nucleic acids, wherein the compound, or a plurality of combinations thereof, can facilitate the in vivo absorption of a variety of nucleic acids via oral administration, enter in vivo target sites of a subject in need thereof, and target cells. The compounds related the present application are extracted and found in traditional Chinese medicine, and can also be obtained by synthetic methods.

### BACKGROUND

Over the past few decades, the concept of using nucleic acid molecules as therapeutic agents has evolved from theory to clinical reality. Indeed, nucleic acid molecules possess numerous attributes that render them suitable as therapeutic agents. They can fold into complex conformations, allowing them to interact with proteins, small molecules, or other nucleic acids, and some can even form catalytic centers. Nucleic acid drugs offer precise targeting and are poised to potentially alter the current landscape of Western medicine dominated by compounds and proteins, emerging as the third major type of drugs after small molecule drugs and antibody drugs. For instance, small interfering RNA (siRNA), as an effector molecule of RNA interference (RNAi), holds increasingly broad prospects as a therapeutic drug. Currently, 13 nucleic acid drugs have been approved globally, and a plurality of siRNA drugs have entered clinical trials, indicating promising development prospects. Typically, people collectively refer to siRNA, miRNA, and other non-coding small RNAs as small nucleic acids or small RNA (sRNA). In addition to small RNAs, nucleic acid molecules suitable as drugs include mRNA, antisense nucleic acids, and others. However, because nucleic acid molecules such as RNA degreade easily and their relatively in vivo short half-life, they are generally not considered the optimal choice for therapeutic drugs. Therefore, the urgent challenge for those skilled in the art is how to effectively deliver nucleic acid molecules, including small RNAs, mRNAs, etc., to in vivo target organs and target cells, realizing the biological activity and therapeutic or preventive effects thereof.

### SUMMARY OF THE INVENTION

In one aspect, the present application provides use of a lipid composition in the manufacture of an agent for delivering nucleic acid, wherein the lipid composition comprises one or more compounds of formula (I),

A-L₁-L₃-L₂-B (I)

or salts, hydrates, or solvates thereof:
wherein:
L₁ is absent, straight or branched C₁₋₂₄ alkyl, straight or branched C₂₋₂₄ alkenyl, aryl optionally substituted by alkoxy or hydroxy, or straight or branched C₁₋₂₄ alkyl optionally substituted by aralkyl;
L₂ is absent, straight or branched C₁₋₂₄ alkyl optionally substituted by amine, or straight or branched C₂₋₂₄ alkenyl;
L₃ is 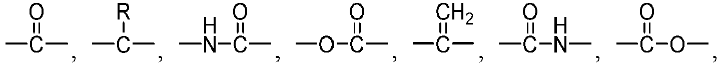 cycloalkyl optionally substituted by oxo, or heterocyclyl optionally substituted by oxo;
A is hydrogen, hydroxy, -COOH, -N(R')₂, straight or branched C₁₋₂₄ alkyl optionally substituted by hydroxy, straight or branched C₂₋₂₄ alkenyl, straight or branched C₂₋₂₄ heteroalkenyl optionally substituted by one or more substituents selected from hydroxy or amine, aryl optionally substituted by alkoxy, aminosulfonyl, or hydroxy, or heterocyclyl optionally substituted by oxo;
B is hydroxy, -CHO, -COOH, -C(O)R', -OC(O)R', straight or branched C₁₋₂₄ alkyl, straight or branched C₂₋₂₄ alkenyl;
R is hydrogen, straight or branched C₁₋₂₄ alkyl;
each R' is independently straight or branched C₁₋₂₄ alkyl.

In some embodiments, the salt of the compound comprises a pharmaceutically acceptable salt.

In another aspect, the the present application also provides use of a lipid composition in the manufacture of an agent for delivering nucleic acid, wherein the lipid composition comprises one or more compounds selected from the group consisting of lipids 501, 502, 503, 505, 519, 520, 521, 522, 525, 526, 527, 528, 529, 530, 537, 542, 543, 572, 573, 574, 578, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830 as shown in table below, or salts, hydrates, or solvates thereof:

| **Lipid Compound No.** | **Product Name** |
|---|---|
| 501 | methyl jasmonate |
| 502 | 6,10-dimethylundeca-5,9-dien-2-one (isomer mixture) |
| 503 | 1-methylcyclopentan-1-ol |
| 505 | tridecan-2-ol |
| 519 | N-(2-hydroxyethyl)tricosanamide |
| 520 | (7Z,10Z,13Z,16Z)-docosa-7,10,13,16-tetraenoic acid |
| 521 | N-(2-hydroxyethyl)tetracosanamide |
| 522 | 6-(6-aminohexanoylamino)hexanoic acid |
| 525 | (E)-4-oxonon-2-enal |
| 526 | (E)-hex-3-en-1-ol |
| 527 | jasmonic acid (isomer mixture) |
| 528 | 2-methylnonane |
| 529 | hexadecanedioic acid |
| 530 | 2-aminohexanedioic acid |
| 537 | capsaicin |
| 542 | (4Z,7Z,10Z,13Z,16Z)-docosa-4,7,10,13,16-pentaenoic acid |
| 543 | 12-Hydroxyjasmonic acid |
| 572 | pentadecanedioic acid |
| 573 | decanamide |
| 574 | pentadecanal |
| 578 | N-(2-hydroxyethyl)hexadecanamide |
| 593 | pentadecan-2-one |
| 594 | tetradecanal |
| 595 | (2S)-2-aminohexanedioic acid |
| 596 | 2-methylpropanedioic acid |
| 597 | dodecanedioic acid-1,6-hexanediamine polymer |
| 598 | 3,3-dimethylpentanedioic acid |
| 599 | hexadecanamide |
| 600 | (E)-octadec-9-en-1-ol |
| 601 | octadecanamide |
| 602 | hexadecanal |
| 603 | 4-hydroxy-4-methyloxan-2-one |
| 604 | decan-2-one |
| 605 | pentadecanoic acid |
| 606 | (E)-2-methylbut-2-enedioic acid |
| 607 | (E)-hex-3-enedioic acid |
| 608 | 2-methylbutanedioic acid |
| 609 | pentadecan-1-ol |
| 610 | pentanamide |
| 611 | dodecanamide |
| 612 | 3-acetyloxolan-2-one |
| 613 | butyl hexanoate |
| 614 | hexyl butanoate |
| 615 | decanal |
| 616 | 2-(3-oxo-2-pentylcyclopentyl)acetic acid |
| 617 | nonyl acetate |
| 618 | (2E,4E)-hepta-2,4-dienal |
| 619 | oct-1-en-3-one |
| 620 | (5Z,8Z,11Z,14Z)-icosa-5,8,11,14-tetraenoic acid |
| 621 | pent-3-en-2-one (isomer mixture) |
| 622 | 3-oxo-N-[(3S)-2-oxooxolan-3-yl]hexanamide |
| 623 | 2-oxobutanedioic acid |
| 624 | icosanoic acid |
| 625 | (2R)-2-aminohexanedioic acid |
| 626 | 2-methylidenebutanedioic acid |
| 627 | propanedioic acid |
| 628 | butanedioic acid |
| 635 | tetradecan-1-ol |
| 636 | octadecan-1-ol |
| 802 | [(2E,6E)-3,7,11-trimethyldodeca-2,6,10-trienyl] dodecanoate |
| 803 | [(2E,6E)-3,7,11-trimethyldodeca-2,6,10-trienyl] decanoate |
| 804 | [(9Z,12Z)-octadeca-9,12-dienyl] hexadecanoate |
| 805 | dodecyl (9Z,12Z,15Z)-octadeca-9,12,15-trienoate |
| 806 | [(Z)-octadec-9-enyl] (9Z,12Z,15Z)-octadeca-9,12,15-trienoate |
| 807 | (5Z,8Z,11Z,14Z)-N-(5-hydroxypentyl)icosa-5,8,11,14-tetraenamide |
| 808 | (5Z,8Z,11Z,14Z)-N-(3-hydroxypropyl)icosa-5,8,11,14-tetraenamide |
| 809 | [(2E,6E)-3,7,11-trimethyldodeca-2,6,10-trienyl] heptanoate |
| 810 | (5Z,8Z,11Z,14Z)-N-[2-(diethylamino)ethyl]icosa-5,8,11,14-tetraenamide |
| 811 | tetradecyl (9Z,12Z)-octadeca-9,12-dienoate |
| 812 | [(9Z,12Z)-octadeca-9,12-dienyl] icosanoate |
| 813 | tridecyl (Z)-hexadec-9-enoate |
| 814 | 3-methylbut-3-enyl icosanoate |
| 815 | 3-methylbut-3-enyl docosanoate |
| 816 | [(Z)-hex-3-enyl] (2R)-2-hydroxy-3-methylbutanoate |
| 817 | docosyl (Z)-tetradec-9-enoate |
| 818 | heptadecyl (Z)-hexadec-9-enoate |
| 819 | tetracosyl (Z)-hexadec-9-enoate |
| 820 | henicosyl (Z)-hexadec-9-enoate |
| 821 | pentadecyl (Z)-hexadec-9-enoate |
| 822 | nonadecyl (Z)-hexadec-9-enoate |
| 823 | docosyl (Z)-hexadec-9-enoate |
| 824 | tetradecyl (9Z,12Z,15Z)-octadeca-9,12,15-trienoate |
| 825 | tricosyl (Z)-hexadec-9-enoate |
| 826 | (5Z,8Z,11Z,14Z)-N-(4-sulfamoylphenyl)icosa-5,8,11,14-tetraenamide |
| 827 | 4-methylheptan-3-yl (Z)-hexadec-9-enoate |
| 828 | 4-methylheptan-3-yl (9Z,12Z)-octadeca-9,12-dienoate |
| 829 | 15-methylhexadecyl tetradecanoate |
| 830 | (2S)-2-(octadecanoylamino)-3-phenylpropanoic acid |

In some embodiments, the lipid composition or the agent may deliver the nucleic acid via oral administration, inhalation, or injection. In some embodiments, the lipid composition or the agent delivers the nucleic acid via oral administration. In some embodiments, the delivery includes in vivo digestive tract delivery.

In some embodiments, the delivery includes in vitro cellular delivery.

In some embodiments, the lipid composition or the agent may be used to prepare a lipid nucleic acid mixture.

The lipid nucleic acid mixture may be prepared by appropriate methods, including, but not limited to, heating, reverse-phase evaporation, or mixing.

In some embodiments, the heating method comprises adding an organic solvent solution of a lipid to an aqueous solution of a nucleic acid to obtain a mixture solution, and heating the mixture solution at a suitable temperature. In some embodiments, the heating method further comprises cooling the heated mixture solution to obtain a mixture of the lipid and the nucleic acid.

In some embodiments, the mixture solution is heated at a temperature selected from 25°C to 100°C, 30°C to 100°C, 40°C to 100°C, 50°C to 100°C, 60°C to 100°C, 70°C to 100°C, 80°C to 100°C, 90°C to 100°C, or 95°C to 100°C. In some embodiments, the mixture solution is heated at a temperature selected from 30°C, 35°C, 37°C, 40°C, 45°C, 50°C, 55°C, 60°C, 65°C, 70°C, 75°C, 80°C, 85°C, 90°C, 95°C, or 100°C.

In some embodiments, the time for heating the mixture solution is about 5 minutes to about 24 hours, about 5 minutes to about 20 hours, about 5 minutes to about 16 hours, about 10 minutes to about 20 hours, about 10 minutes to about 16 hours, about 15 minutes to about 24 hours, about 15 minutes to about 20 hours, about 30 minutes to about 24 hours, about 30 minutes to about 20 hours, about 40 minutes to about 16 hours, about 50 minutes to about 12 hours, about 1 hour to about 8 hours, or about 2 hours to about 4 hours. In some embodiments, the time for heating the mixture solution is about 5 minutes to about 1 hour, about 5 minutes to about 30 minutes, about 5 minutes to about 15 minutes, or about 10 minutes to about 15 minutes. In some embodiments, the time for heating the mixture solution is about 5 minutes, about 10 minutes, about 15 minutes, about 20 minutes, about 25 minutes, about 30 minutes, 40 minutes, 50 minutes, 1 hour, 2 hours, 3 hours, 4 hours, 5 hours, 6 hours, 7 hours, 8 hours, 9 hours, 10 hours, 12 hours, 16 hours, 20 hours, or 24 hours.

In some embodiments, the mixture solution is cooled at a temperature selected from 25°C to -80°C, 20°C to -80°C, 15°C to -80°C, 10°C to -80°C, 4°C to -80°C, 0°C to -80°C, -10°C to -80°C, -20°C to -80°C, -30°C to -80°C, or -40°C to -80°C. In some embodiments, the mixture solution is cooled at a temperature selected from 25°C, 20°C, 15°C, 10°C, 4°C, or 0°C.

In some embodiments, the reverse-phase evaporation method comprises mixing an aqueous solution of a nucleic acid with an organic solvent solution of a lipid compound to obtain a mixture solution. In some embodiments, the reverse-phase evaporation method further comprises removing the organic solvent from the mixture solution followed by hydration to obtain a mixture of the lipid and the nucleic acid. In some embodiments, the mixture solution is ultrasonicated and/or evaporated to remove the organic solvent. In some embodiments, the step to remove the organic solvent from the mixture solution is conducted at a suitable temperature.

In some embodiments, the organic solvent in the mixture solution is removed at a temperature selected from about 25°C to about 70°C, 30°C to about 70°C, about 30°C to about 65°C, about 40°C to about 65°C, about 40°C to about 60°C, or about 50°C to about 60°C. In some embodiments, the organic solvent in the mixture solution is removed at a temperature selected from about 25°C, about 30°C, about 35°C, about 40°C, about 45°C, about 50°C, about 55°C, about 60°C, about 65°C, or about 70°C.

In another aspect, the present application provides a method of delivering nucleic acid to an individual in need thereof, comprising administering a lipid composition and the nucleic acid to the individual orally, via inhalation or injection. In some embodiments, the lipid composition and the nucleic acid are administered in the form of a lipid nucleic acid mixture.

In some embodiments, the nucleic acid comprises DNA or RNA. In some embodiments, the DNA is non-coding DNA (such as antisense DNA) or coding DNA, among others. In some embodiments, the RNA is antisense RNA, mRNA, lncRNA, or small RNA (such as miRNA, siRNA, piRNA, snoRNA, tsRNA), among others.

In some embodiments, the nucleic acid comprises small nucleic acids having a length of 14-32bp, 16-28bp, or 18-24bp.

In certain embodiments, the nucleic acid issingle stranded or double stranded.

In certain embodiments, the nucleic acid has a stem-loop structure.

In some embodiments, the nucleic acid is used for treating diseases.

In some embodiments, the nucleic acid is used for treating cancer, inflammation, fibrotic disease, autoimmune disease or autoinflammatory disease, bacterial infection, behavioral and psychiatric disorder, blood disease, chromosomal disease, congenital and hereditary disease, connective tissue disease, digestive disease, ear nose throat disease, endocrine disease, environmental disease, eye disease, female reproductive disease, fungal infection, heart disease, hereditary cancer syndrome, immune system disorder, kidney and urinary disease, lung disease, male reproductive disease, metabolic disorder, mouth disease, musculoskeletal disease, myelodysplastic syndrome, neonatal screening, nutritional disease, parasitic disease, rare cancer, rare disease, skin disease, or viral infection.

In some embodiments, the nucleic acid is used for treating hepatocellular carcinoma, corneal neovascularization, recurrent or refractory anaplastic astrocytoma (WHO Grade III), or secondary glioblastoma (WHO Grade IV), advanced squamous cell lung cancer, acromegaly, psoriasis, Duchenne muscular dystrophy, advanced non-small cell lung cancer, metastatic castration-resistant prostate cancer, cytomegalovirus retinitis, HIV infection, hepatitis B, hepatitis C, hyperlipoprotein disease, total knee replacement, type 2 diabetes, familial amyloid polyneuropathy (FAP), wet macular degeneration (e.g., neovascular age-related macular degeneration, subfoveal neovascular age-related macular degeneration, exudative age-related macular degeneration), hypercholesterolemia, Crohn's disease, extensive liver fibrosis, infantile spinal muscular atrophy, melanoma, neonatal coronary artery disease, mild allergic asthma, chronic lymphocytic leukemia, and hypertriglyceridemia, small cell obliterans of the liver with renal or lung dysfunction after hematopoietic stem cell transplantation, or hereditary transthyretin amyloidosis.

In another aspect, the present application also provides a pharmaceutical composition comprising a lipid composition and a nucleic acid, wherein the lipid composition comprises one or more compounds of formula (I), or pharmaceutically acceptable salts, hydrates, or solvates thereof. In some embodiments, the lipid composition and the nucleic acid are present in the form of a lipid nucleic acid mixture.

In another aspect, the present application also provides a pharmaceutical composition comprising a lipid composition and a nucleic acid, wherein the lipid composition comprises one or more compounds selected from the group consisting of lipids 501, 502, 503, 505, 519, 520, 521, 522, 525, 526, 527, 528, 529, 530, 537, 542, 543, 572, 573, 574, 578, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830, or salts, hydrates, or solvates thereof. In some embodiments, the lipid composition and the nucleic acid are present in the form of a lipid nucleic acid mixture.

In another aspect, the present application also provides the use of the pharmaceutical compositions of the present application in the manufacture of a medicament for the prevention and/or treatment of diseases that can be prevented and/or treated with nucleic acid, or for in vivo delivering nucleic acid to a subject in need thereof.

In another aspect, the present application also provides a kit comprising one or more compounds of formula (I), or salts, hydrates, or pharmaceutically acceptable solvates thereof, positioned in a first container, and a nucleic acid positioned in a second container.

In another aspect, the present application also provides a kit comprising one or more compounds or pharmaceutically acceptable salts, hydrates, or solvates thereof, positioned in a first container, and a nucleic acid positioned in a second container, wherein the one or more compounds are selected from the group consisting of lipids 501, 502, 503, 505, 519, 520, 521, 522, 525, 526, 527, 528, 529, 530, 537, 542, 543, 572, 573, 574, 578, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830.

In another aspect, the present application provides the use of the kit of the present application in the manufacture of a medicament for the prevention and/or treatment of diseases that can be prevented and/or treated with nucleic acids, or for in vivo delivering nucleic acids to a subject in need thereof.

In another aspect, the present application provides a method of delivering a nucleic acid to a target cell, comprising administering to the target cell the pharmaceutical composition of the present application or a lipid nucleic acid mixture formulated from the kit of the present application.

In another aspect, the present application provides a method of in vivo delivering a nucleic acid to a subject in need thereof, comprising administering to the subject the pharmaceutical composition of the present application or a lipid nucleic acid mixture formulated from the kit of the present application.

It should be understood that within the scope of the present disclosure, each above-mentioned technical feature of the present disclosure and each technical feature specifically described below (e.g., in Examples) can be combined with each other to form a new or preferred technical solution, the details of which will not be elaborated here one by one due to space limitations.

### BRIEF DESCRIPTION OF THE DRAWINGS

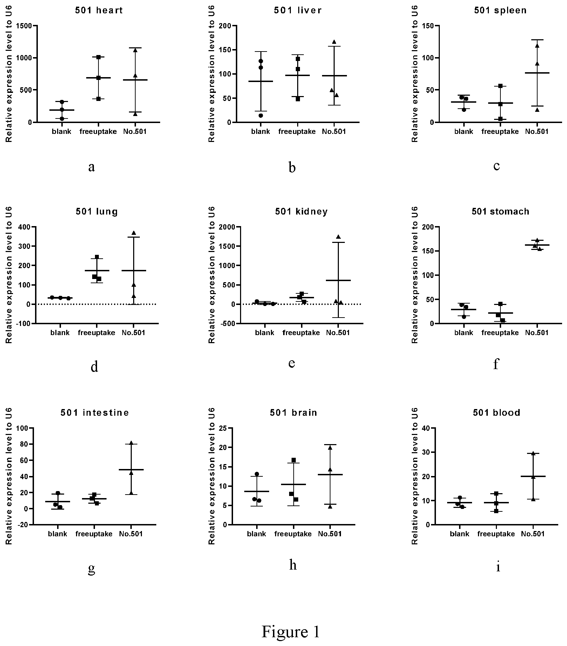

Figure 1 illustrates the results of blank group, free uptake group, and lipid 501 for delivery of PGY-sRNA-26 into heart tissue (a), liver tissue (b), spleen tissue (c), lung tissue (d), kidney tissue (e), stomach tissue (f), intestine tissue (g), brain tissue (h), and blood (i) of mouse.

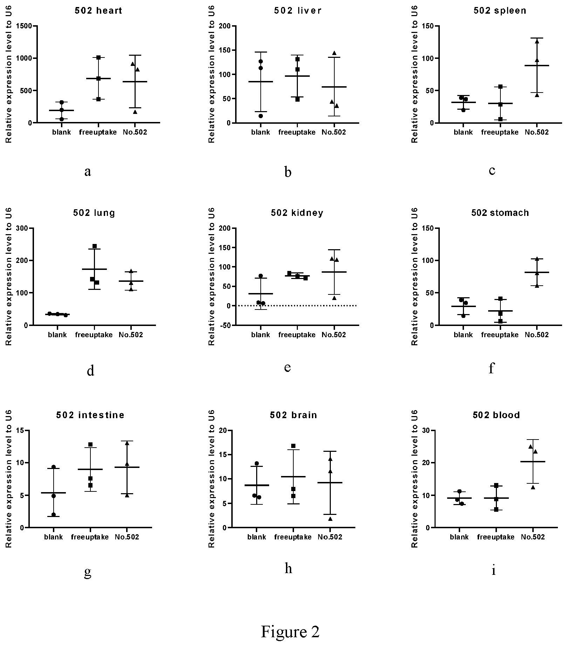

Figure 2 illustrates the results of blank group, free uptake group, and lipid 502 for delivery of PGY-sRNA-26 into heart tissue (a), liver tissue (b), spleen tissue (c), lung tissue (d), kidney tissue (e), stomach tissue (f), intestine tissue (g), brain tissue (h), and blood (i) of mouse.

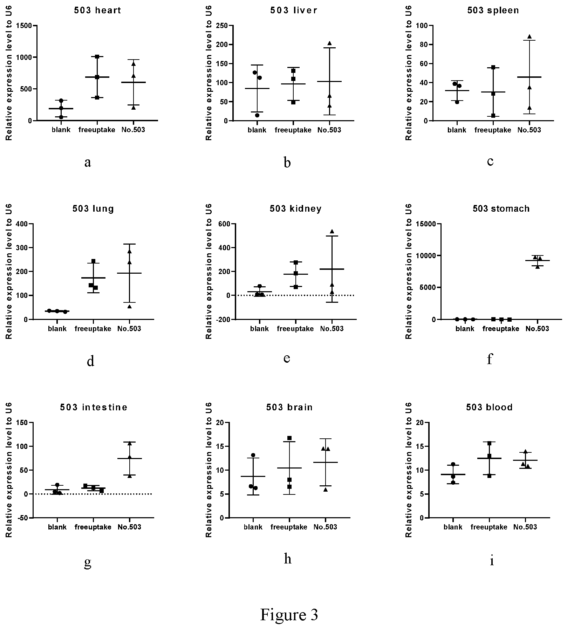

Figure 3 illustrates the results of blank group, free uptake group, and lipid 503 for delivery of PGY-sRNA-26 into heart tissue (a), liver tissue (b), spleen tissue (c), lung tissue (d), kidney tissue (e), stomach tissue (f), intestine tissue (g), brain tissue (h), and blood (i) of mouse.

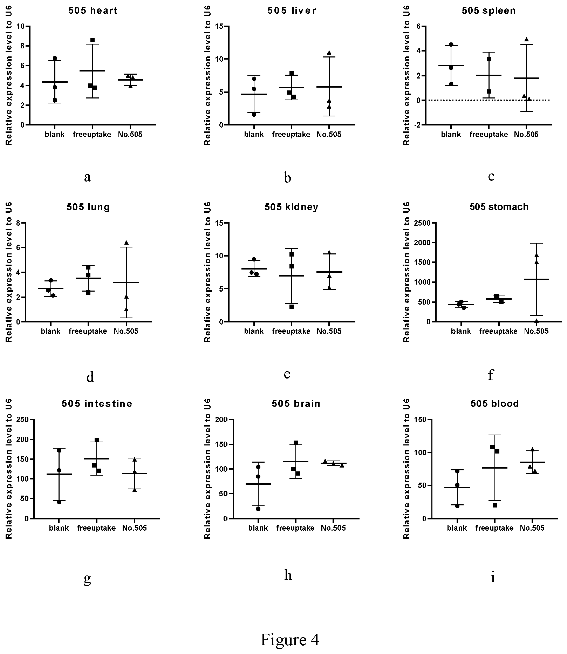

Figure 4 illustrates the results of the blank group, free uptake group, and lipid 505 for delivery of PGY-sRNA-26 into heart tissue (a), liver tissue (b), spleen tissue (c), lung tissue (d), kidney tissue (e), stomach tissue (f), intestine tissue (g), brain tissue (h), and blood (i) of mouse.

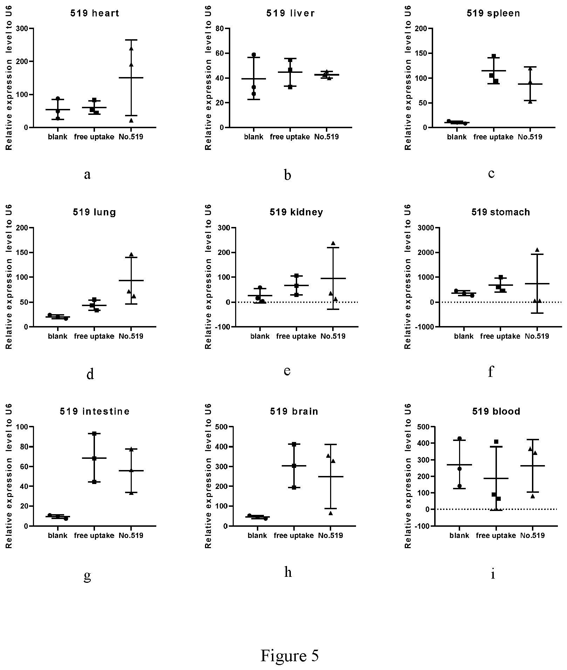

Figure 5 illustrates the results of the blank group, free uptake group, and lipid 519 for delivery of PGY-sRNA-26 into heart tissue (a), liver tissue (b), spleen tissue (c), lung tissue (d), kidney tissue (e), stomach tissue (f), intestine tissue (g), brain tissue (h), and blood (i) of mouse.

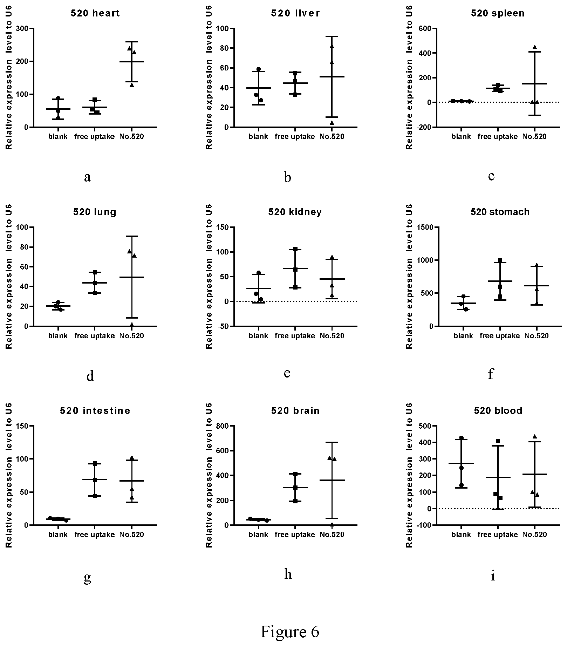

Figure 6 illustrates the results of the blank group, free uptake group, and lipid 520 for delivery of PGY-sRNA-26 into heart tissue (a), liver tissue (b), spleen tissue (c), lung tissue (d), kidney tissue (e), stomach tissue (f), intestine tissue (g), brain tissue (h), and blood (i) of mouse.

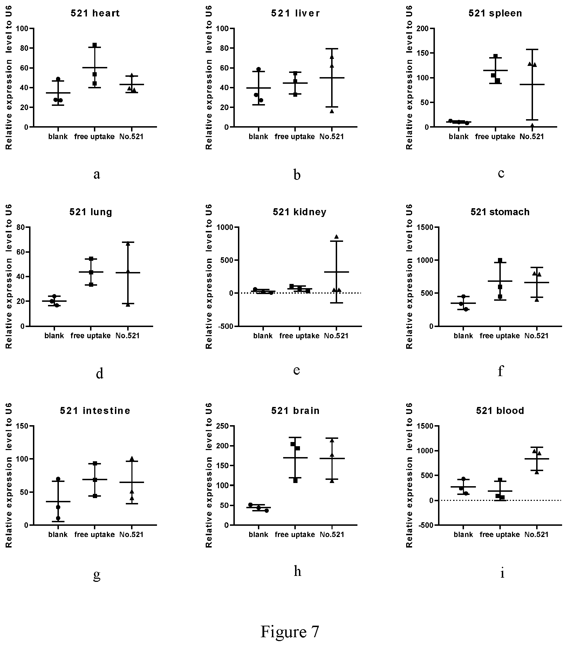

Figure 7 illustrates the results of the blank group, free uptake group, and lipid 521 for delivery of PGY-sRNA-26 into heart tissue (a), liver tissue (b), spleen tissue (c), lung tissue (d), kidney tissue (e), stomach tissue (f), intestine tissue (g), brain tissue (h), and blood (i) of mouse.

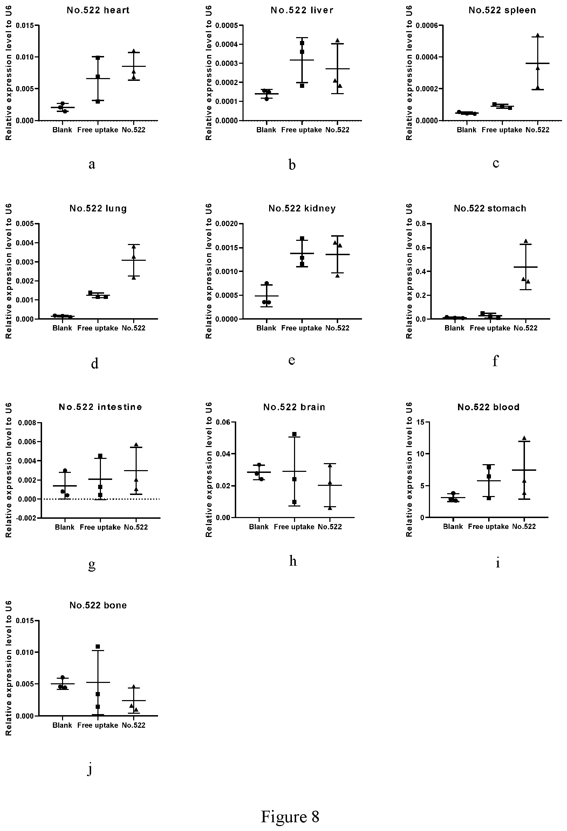

Figure 8 illustrates the results of the blank group, free uptake group, and lipid 522 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

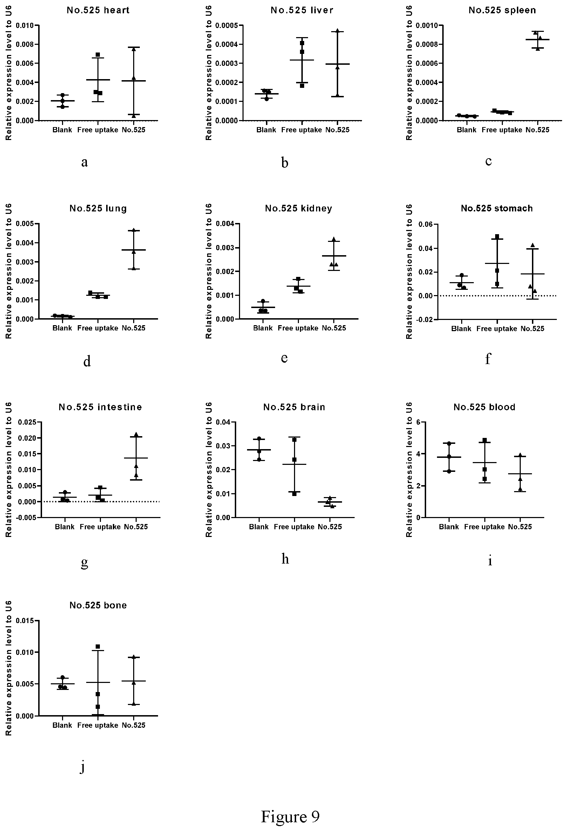

Figure 9 illustrates the results of the blank group, free uptake group, and lipid 525 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

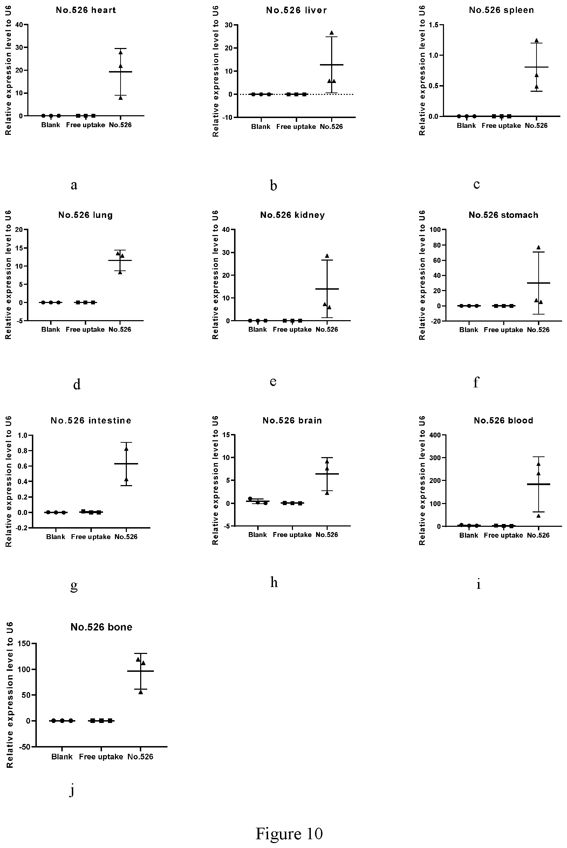

Figure 10 illustrates the results of the blank group, free uptake group, and lipid 526 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

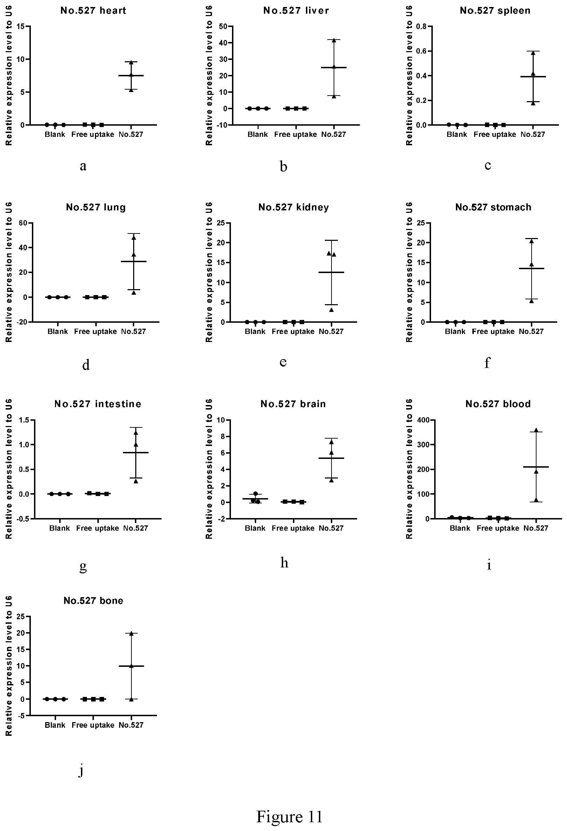

Figure 11 illustrates the results of the blank group, free uptake group, and lipid 527 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

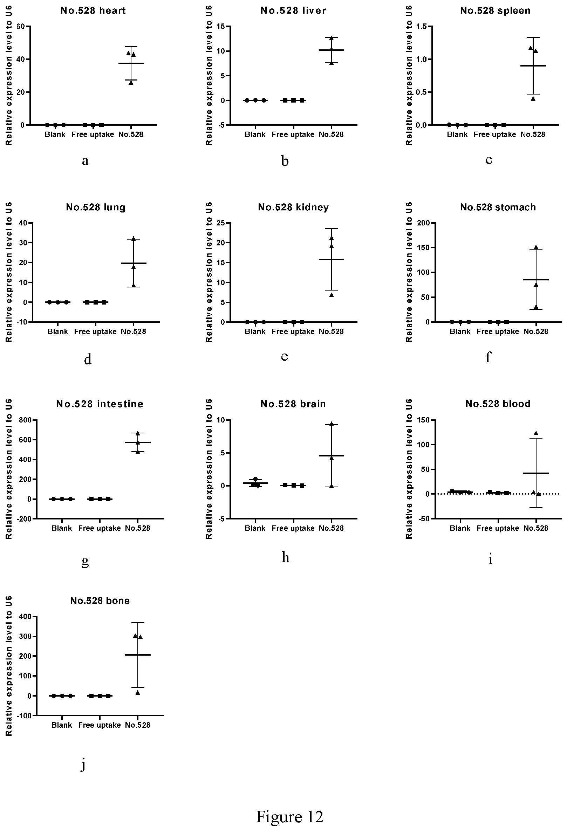

Figure 12 illustrates the results of the blank group, free uptake group, and lipid 528 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

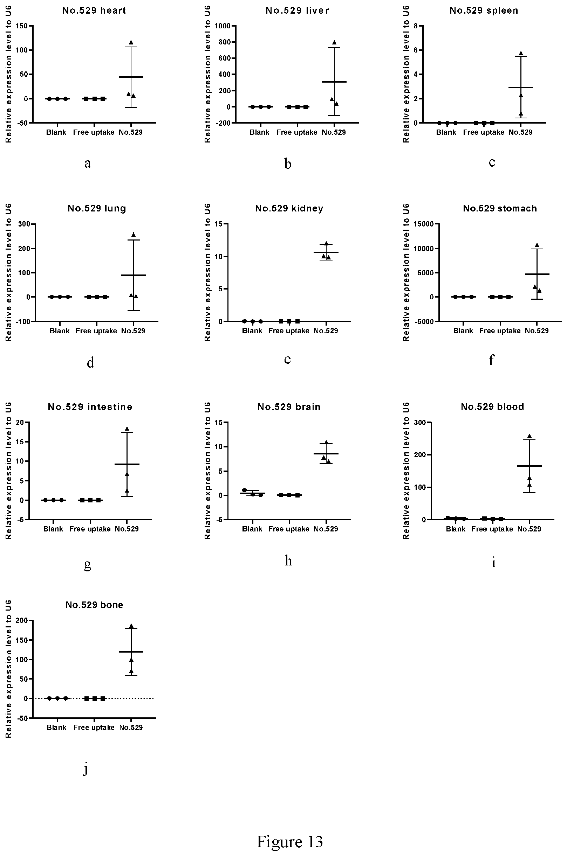

Figure 13 illustrates the results of the blank group, free uptake group, and lipid 529 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

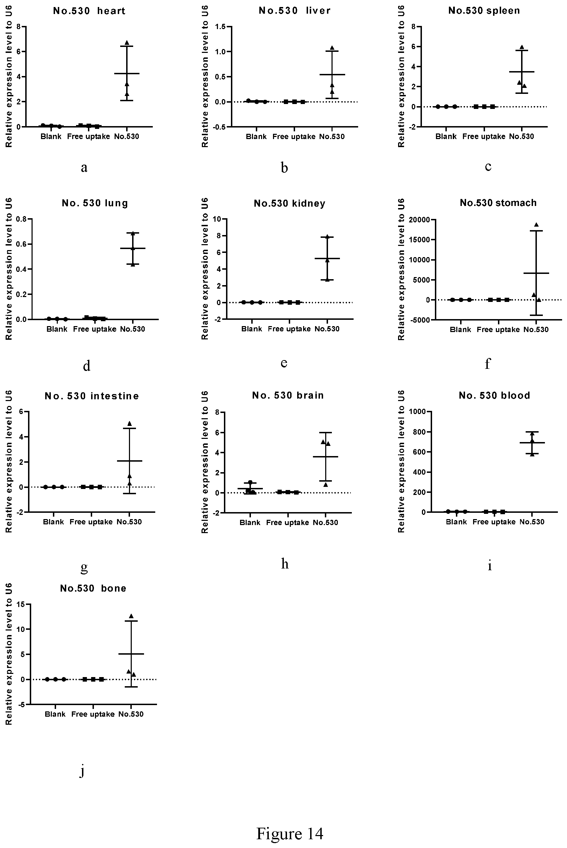

Figure 14 illustrates the results of the blank group, free uptake group, and lipid 530 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

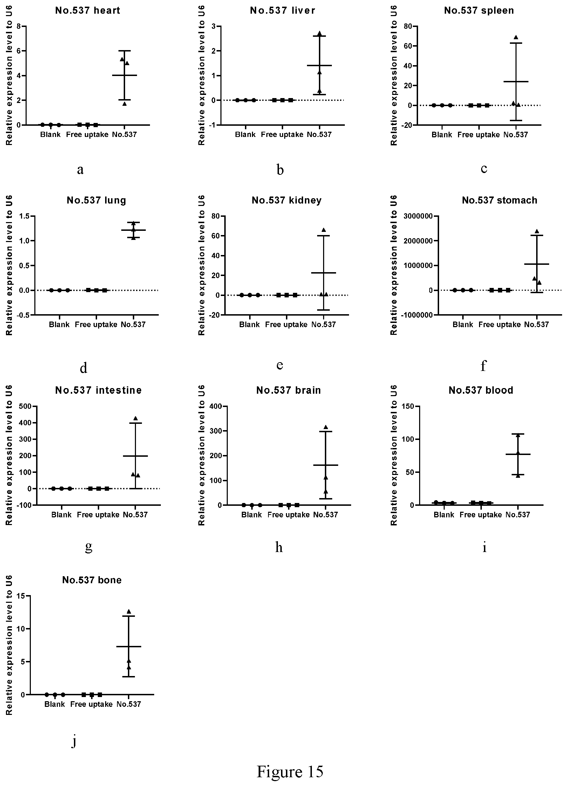

Figure 15 illustrates the results of the blank group, free uptake group, and lipid 537 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

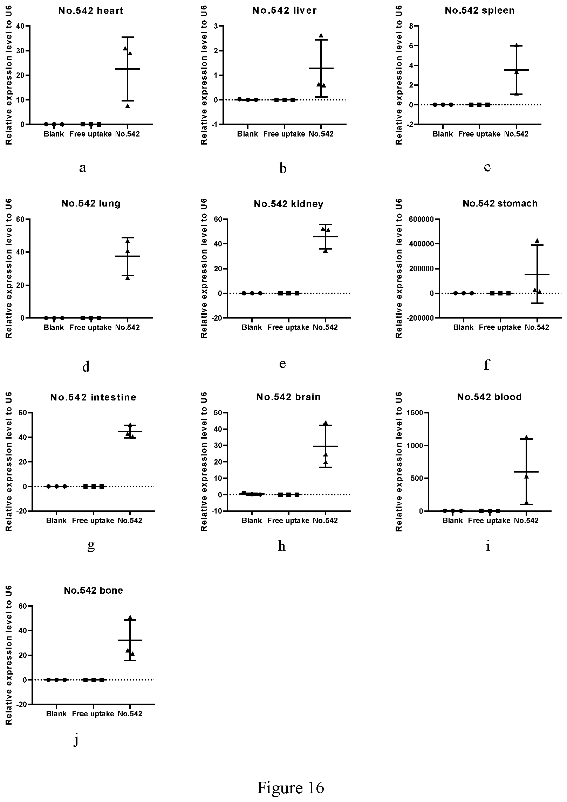

Figure 16 illustrates the results of the blank group, free uptake group, and lipid 542 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

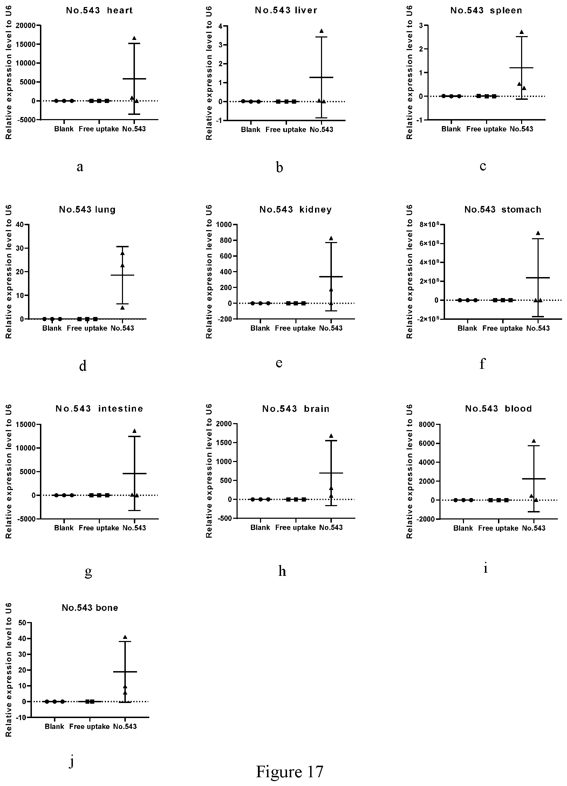

Figure 17 illustrates the results of the blank group, free uptake group, and lipid 543 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

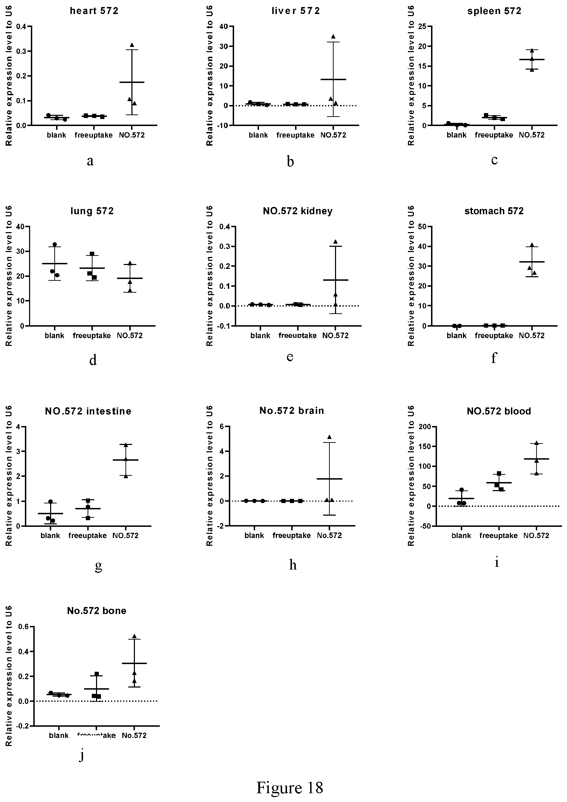

Figure 18 illustrates the results of the blank group, free uptake group, and lipid 572 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

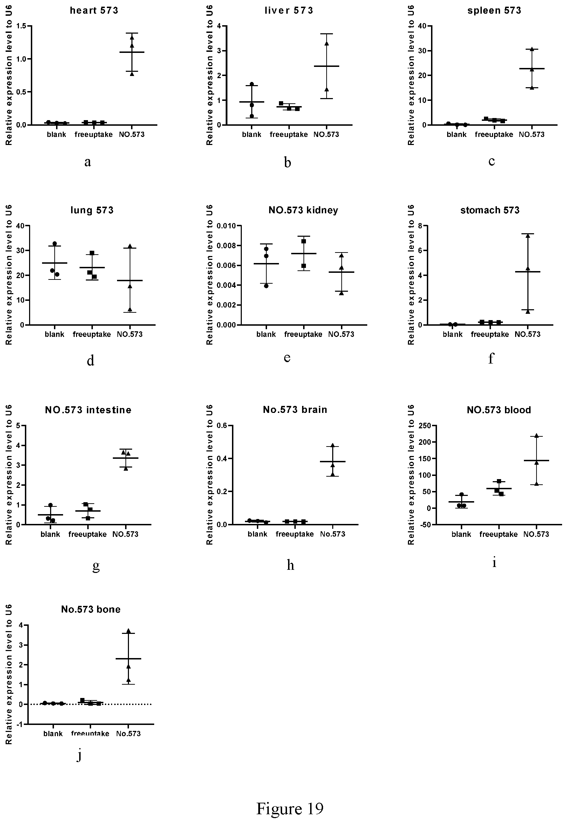

Figure 19 illustrates the results of the blank group, free uptake group, and lipid 573 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

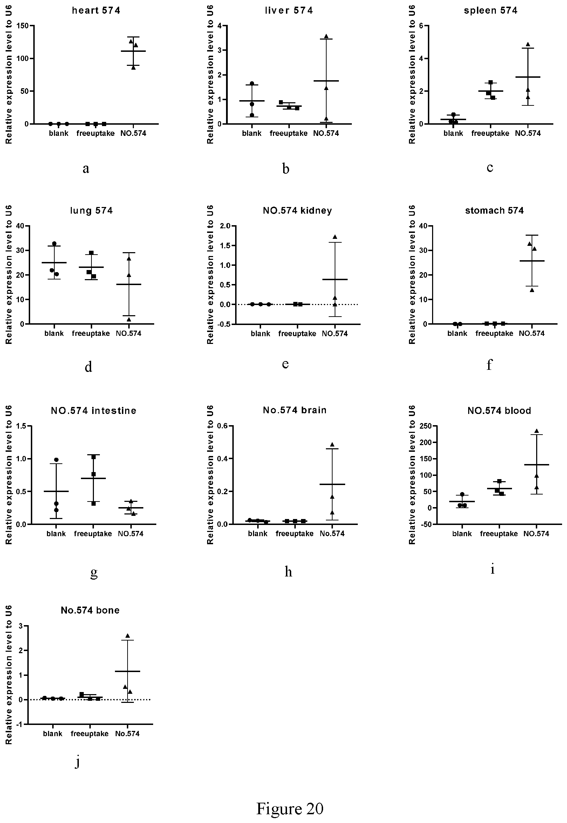

Figure 20 illustrates the results of the blank group, free uptake group, and lipid 574 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

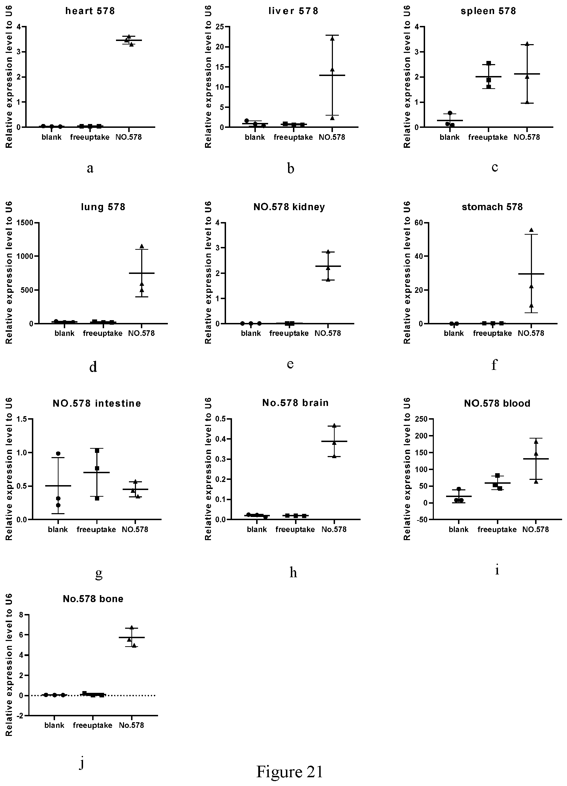

Figure 21 illustrates the results of the blank group, free uptake group, and lipid 578 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

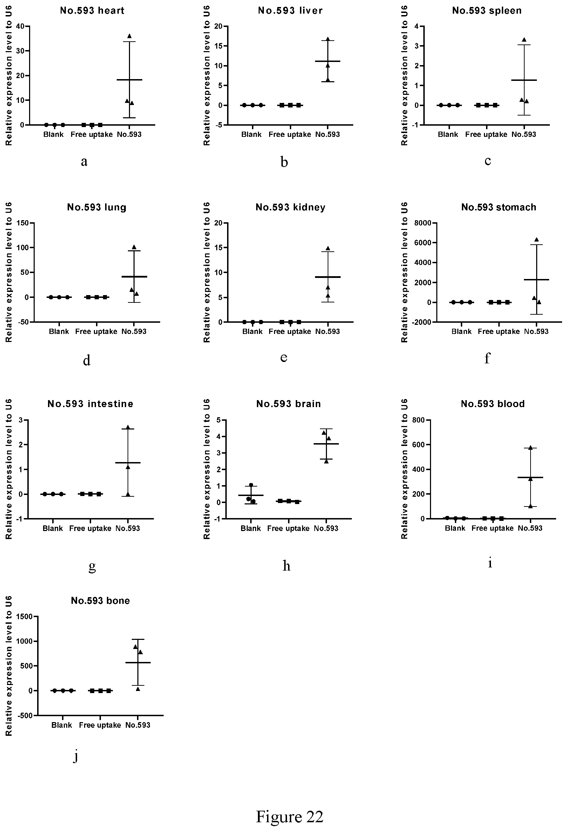

Figure 22 illustrates the results of the blank group, free uptake group, and lipid 593 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

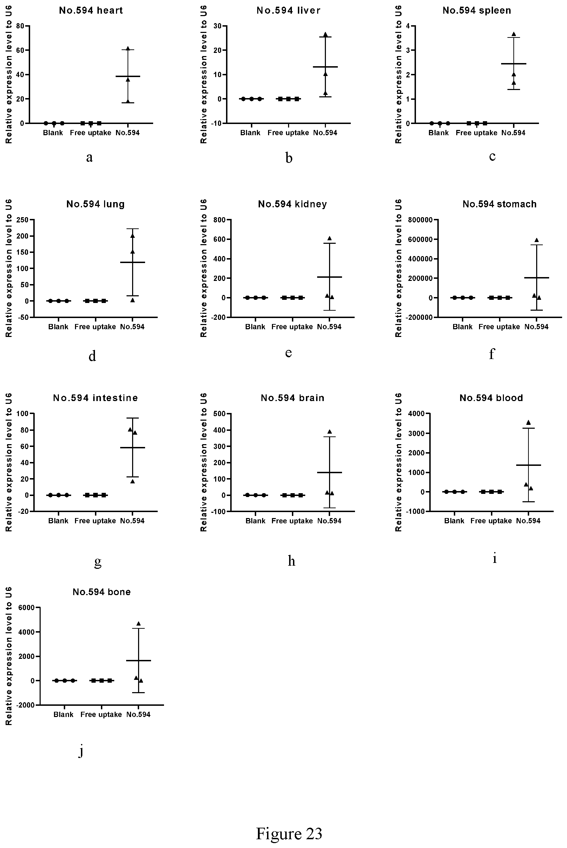

Figure 23 illustrates the results of the blank group, free uptake group, and lipid 594 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

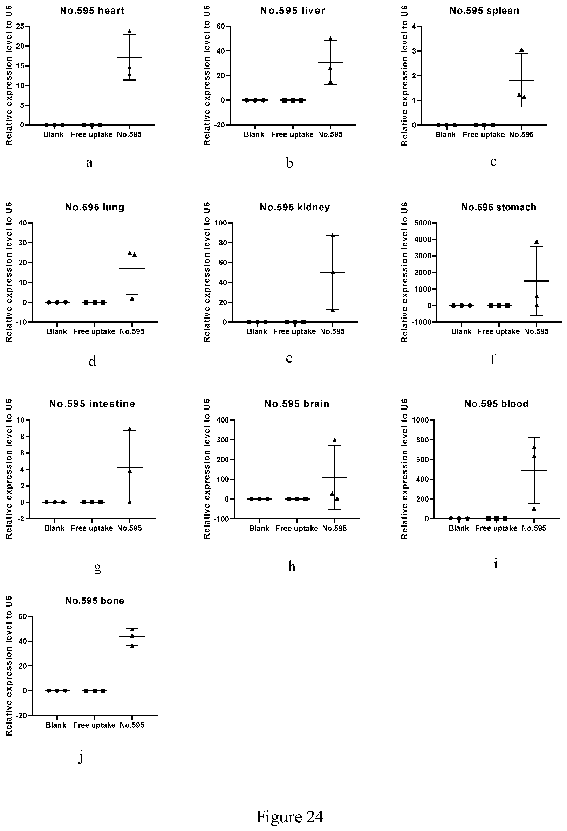

Figure 24 illustrates the results of the blank group, free uptake group, and lipid 595 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

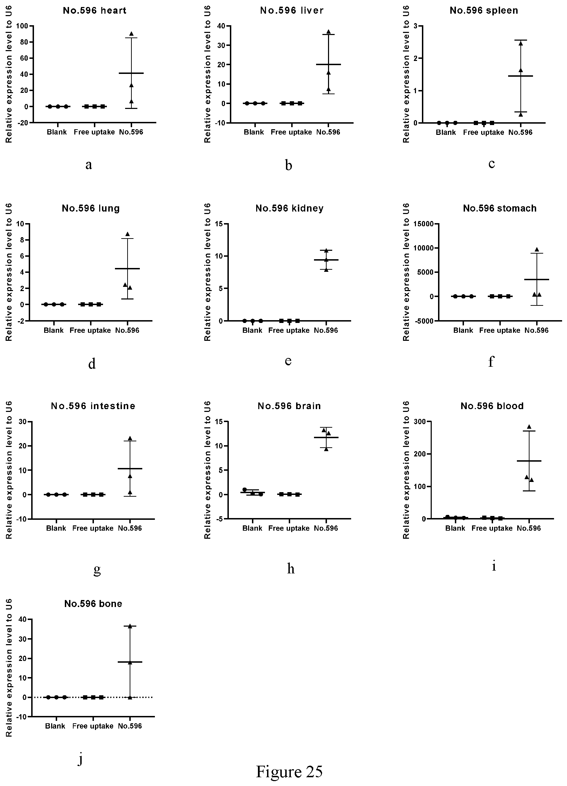

Figure 25 illustrates the results of the blank group, free uptake group, and lipid 596 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

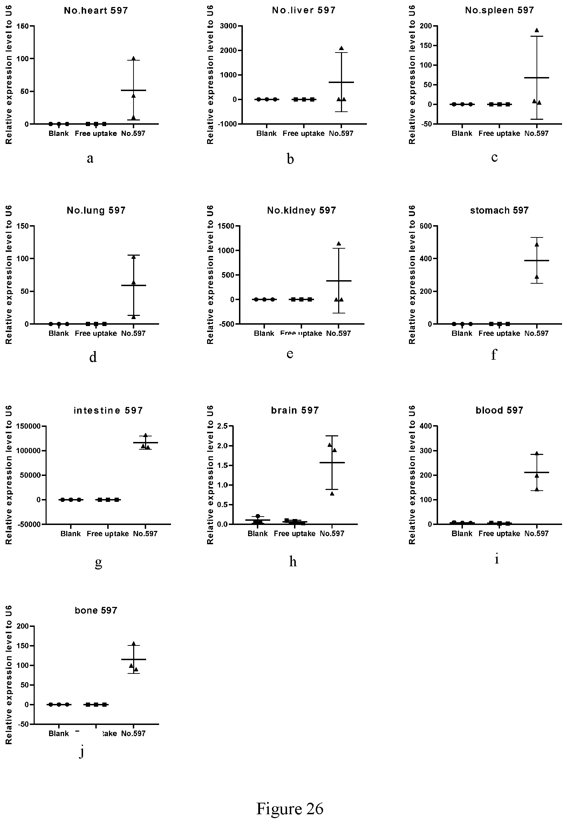

Figure 26 shows the results of the blank group, free uptake group, and lipid 597 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

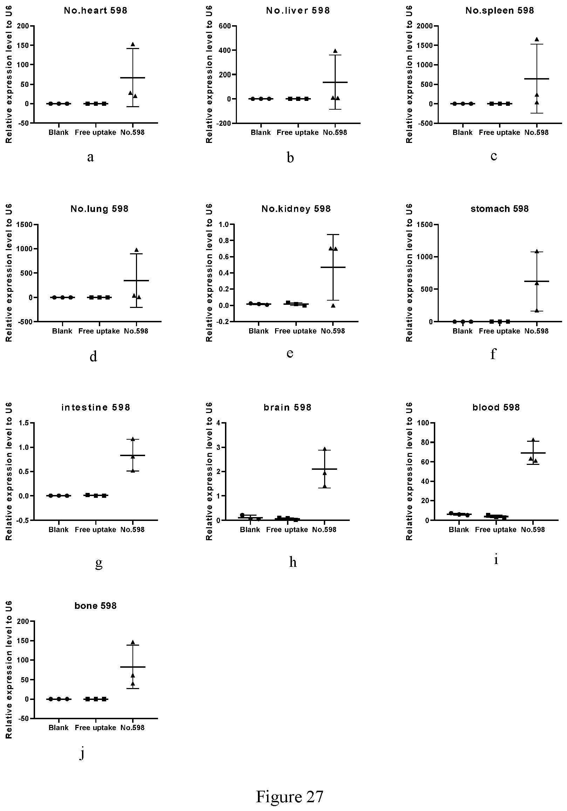

Figure 27 illustrates the results of the blank group, free uptake group, and lipid 598 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

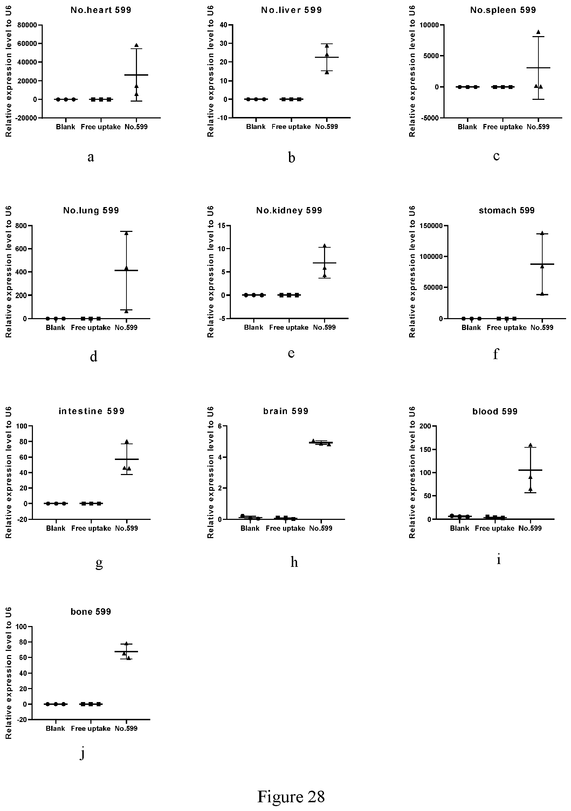

Figure 28 illustrates the results of the blank group, free uptake group, and lipid 599 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

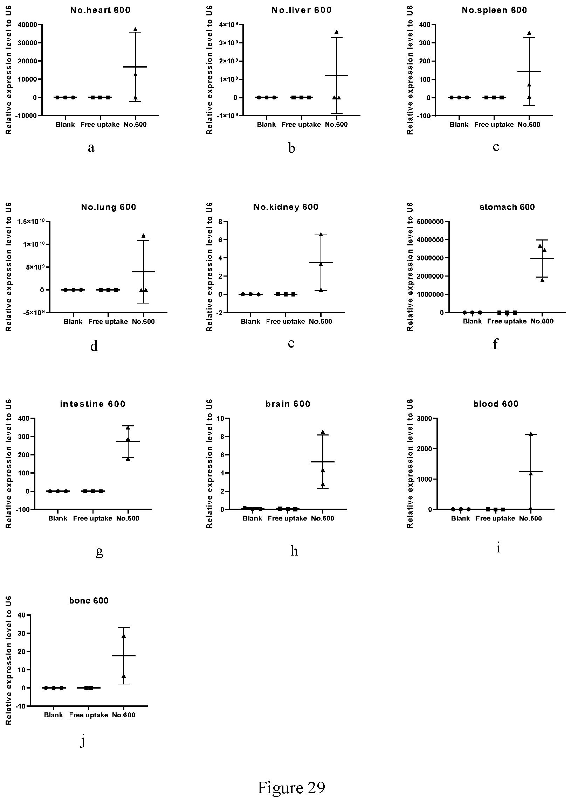

Figure 29 illustrates the results of the blank group, free uptake group, and lipid 600 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

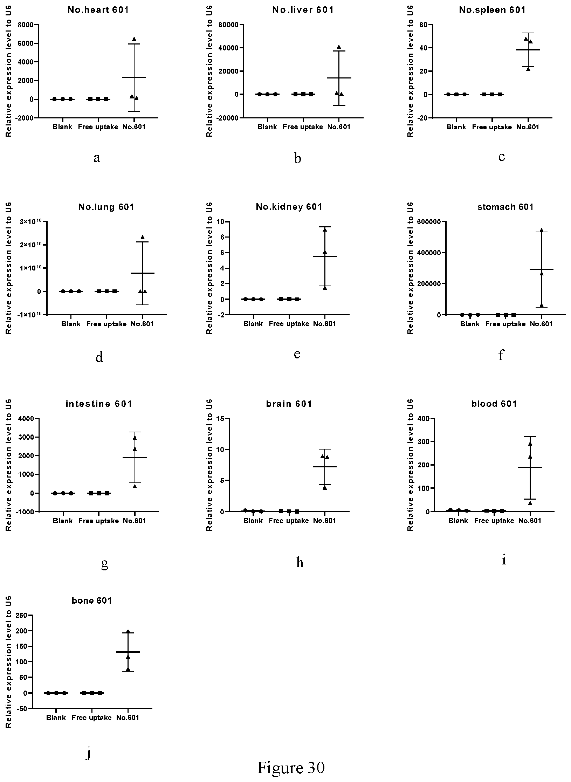

Figure 30 illustrates the results of the blank group, free uptake group, and lipid 601 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

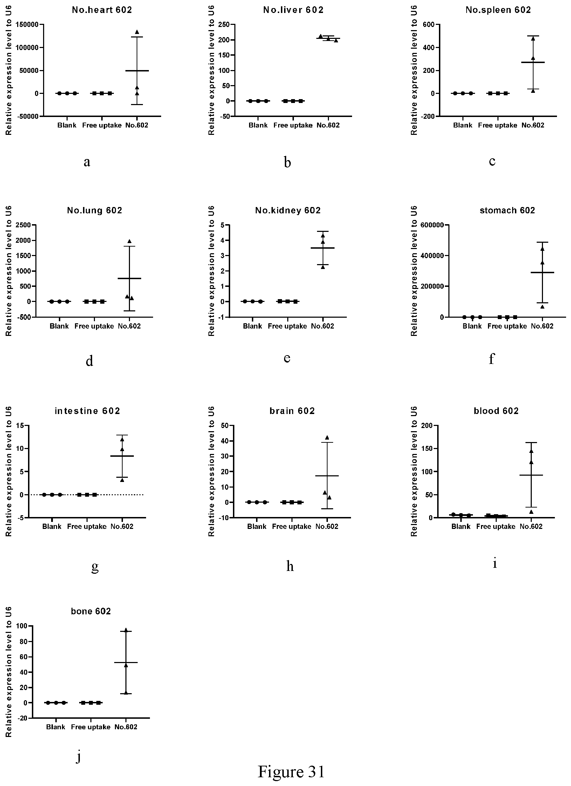

Figure 31 illustrates the results of the blank group, free uptake group, and lipid 602 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

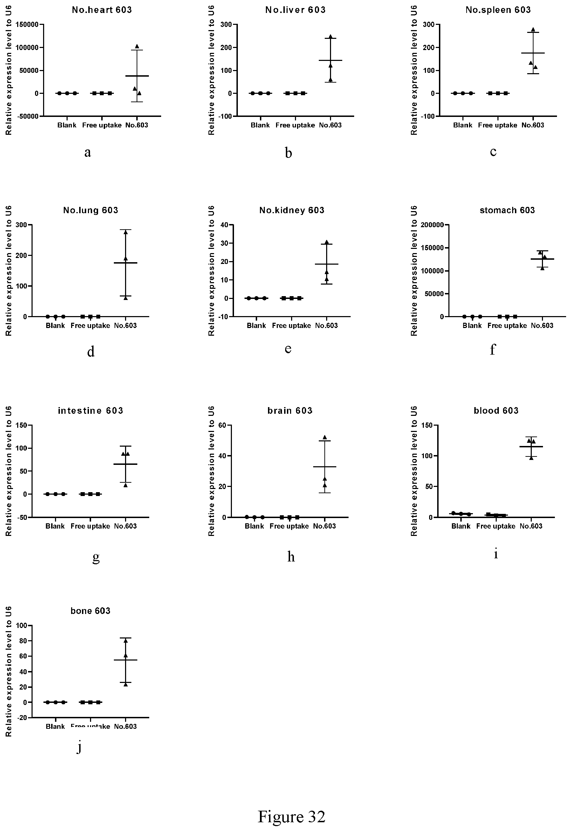

Figure 32 illustrates the results of the blank group, free uptake group, and lipid 603 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

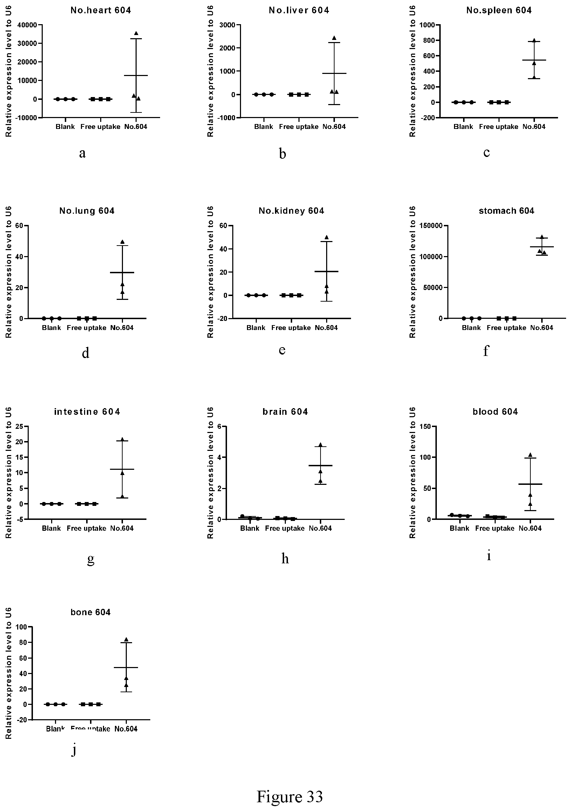

Figure 33 illustrates the results of the blank group, free uptake group, and lipid 604 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

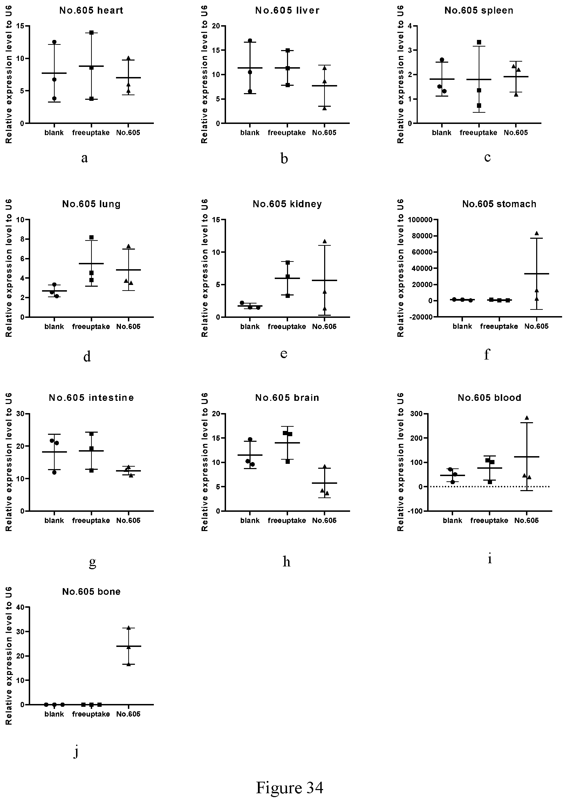

Figure 34 illustrates the results of the blank group, free uptake group, and lipid 605 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

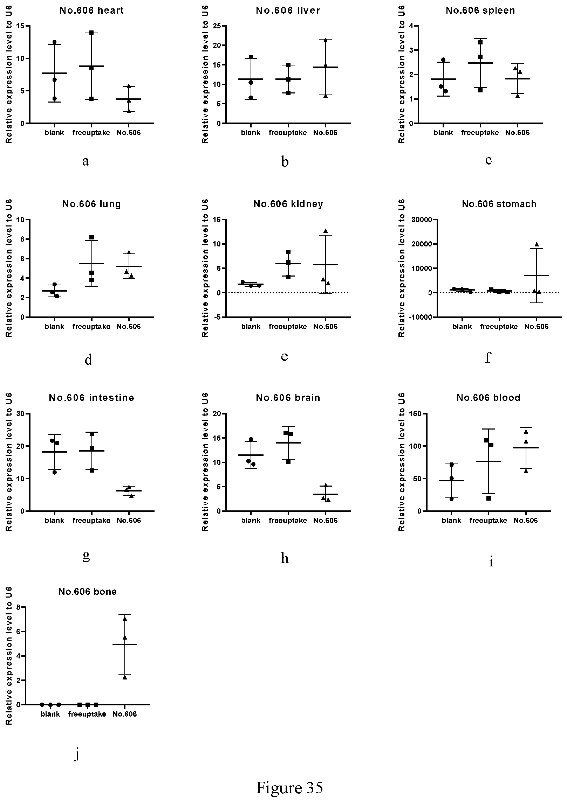

Figure 35 illustrates the results of the blank group, free uptake group, and lipid 606 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

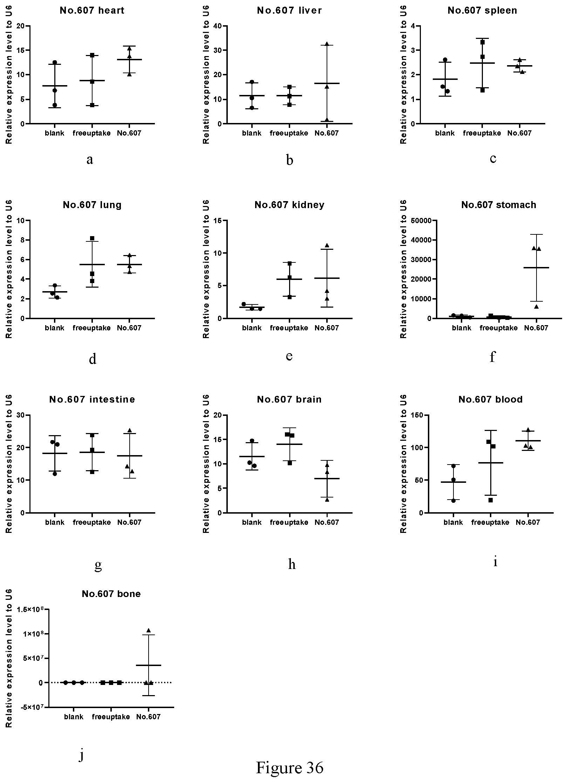

Figure 36 illustrates the results of the blank group, free uptake group, and lipid 607 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

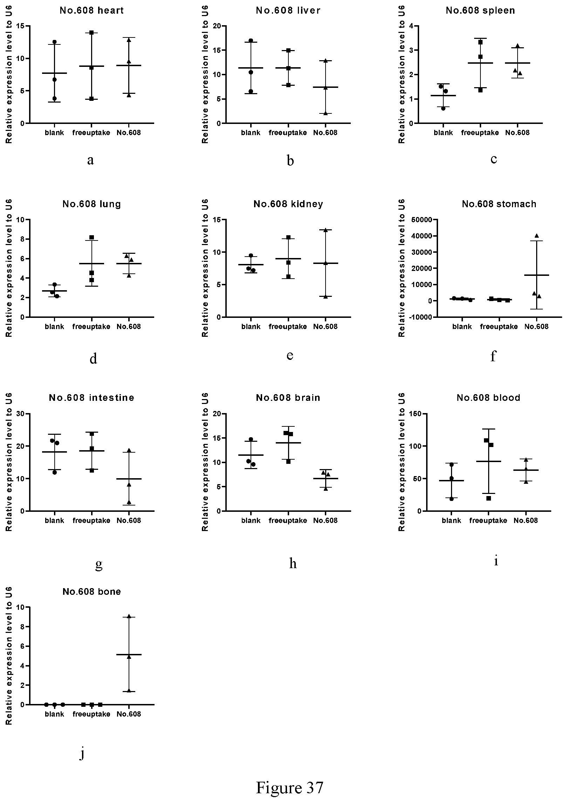

Figure 37 illustrates the results of the blank group, free uptake group, and lipid 608 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

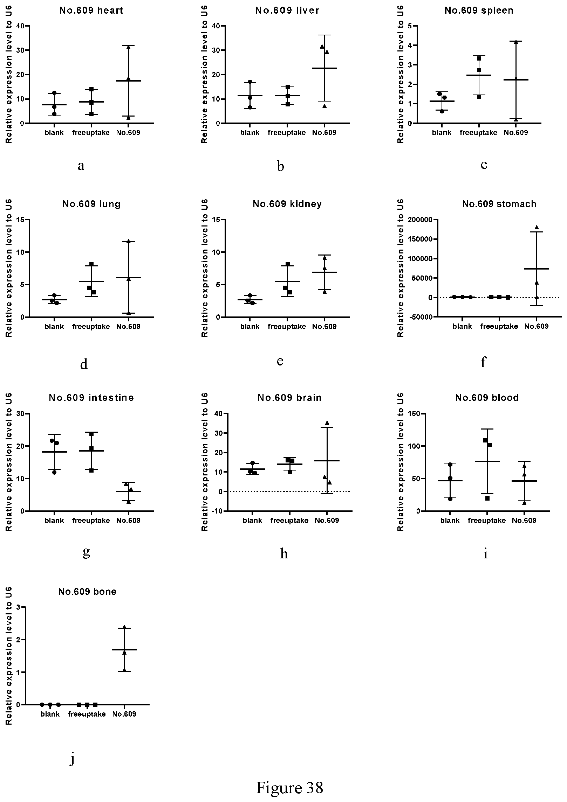

Figure 38 illustrates the results of the blank group, free uptake group, and lipid 609 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

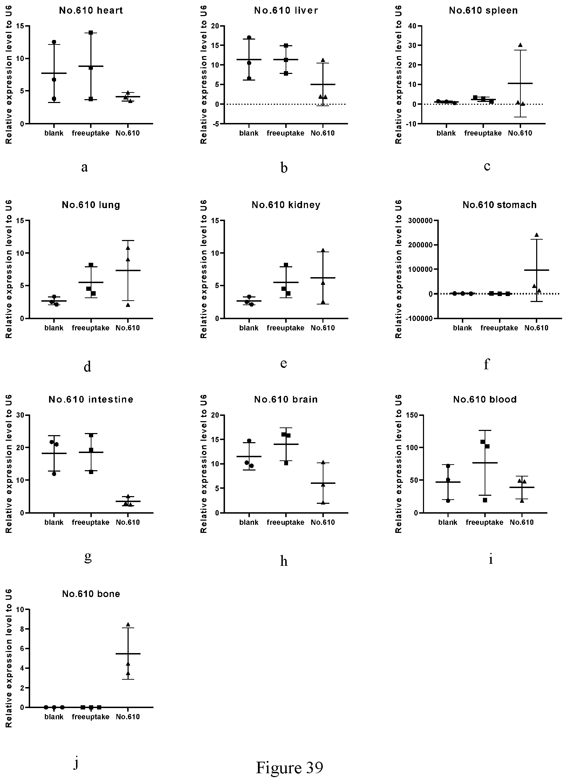

Figure 39 illustrates the results of the blank group, free uptake group, and lipid 610 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

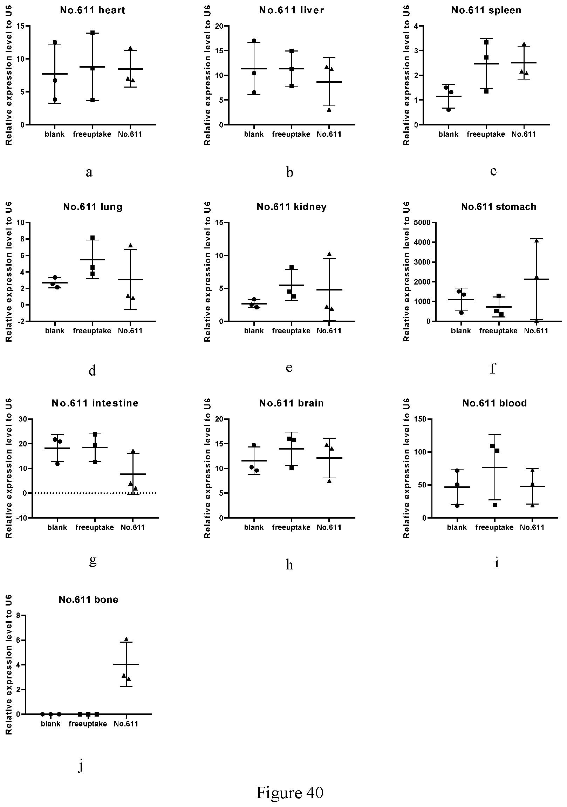

Figure 40 illustrates the results of the blank group, free uptake group, and lipid 611 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

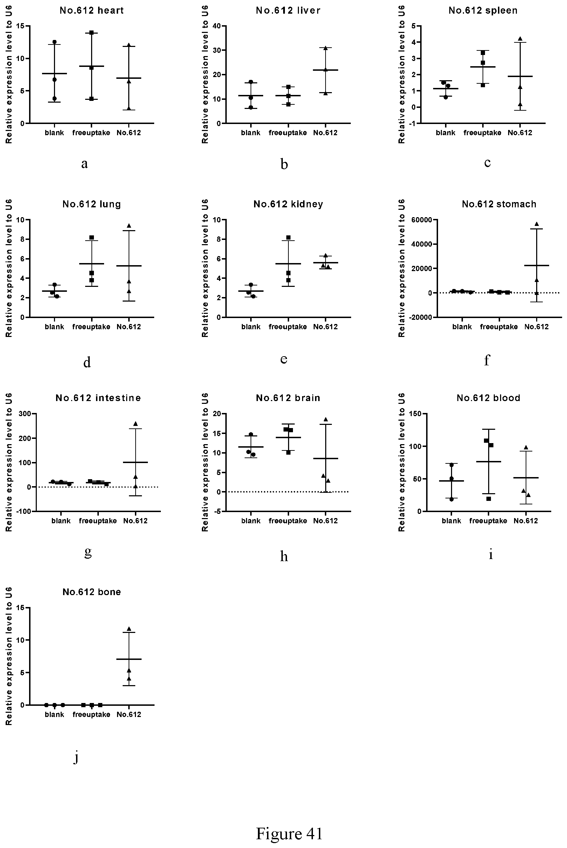

Figure 41 illustrates the results of the blank group, free uptake group, and lipid 612 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

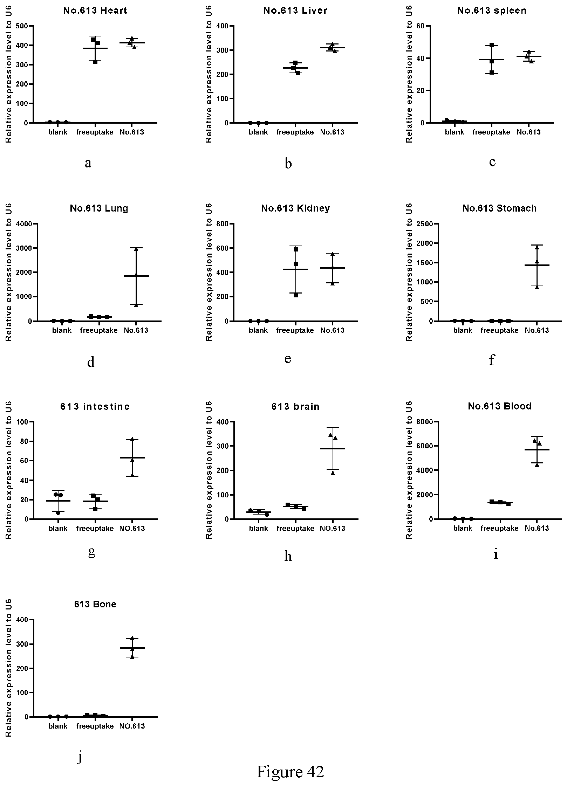

Figure 42 illustrates the results of the blank group, free uptake group, and lipid 613 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

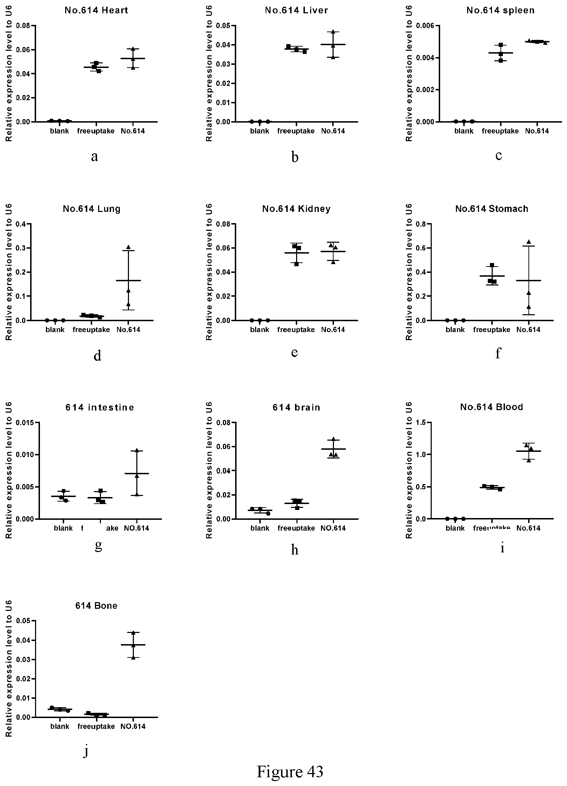

Figure 43 illustrates the results of the blank group, free uptake group, and lipid 614 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

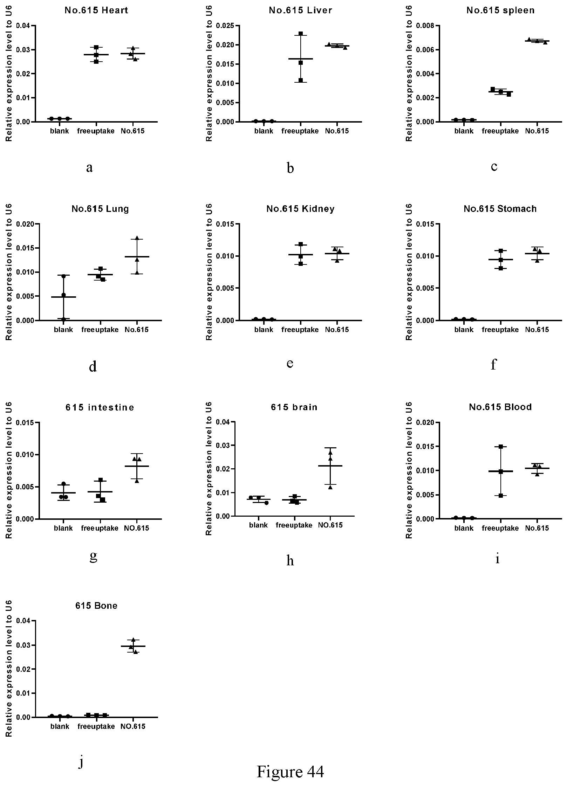

Figure 44 illustrates the results of the blank group, free uptake group, and lipid 615 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

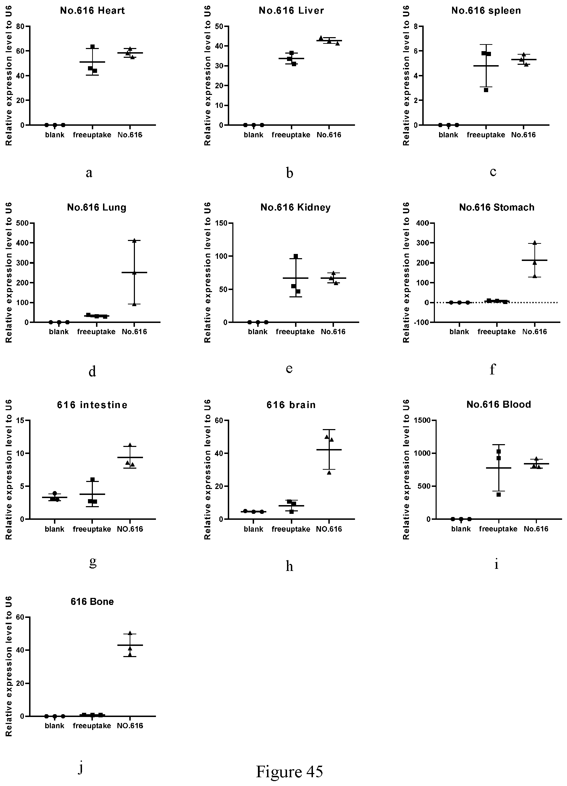

Figure 45 illustrates the results of the blank group, free uptake group, and lipid 616 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

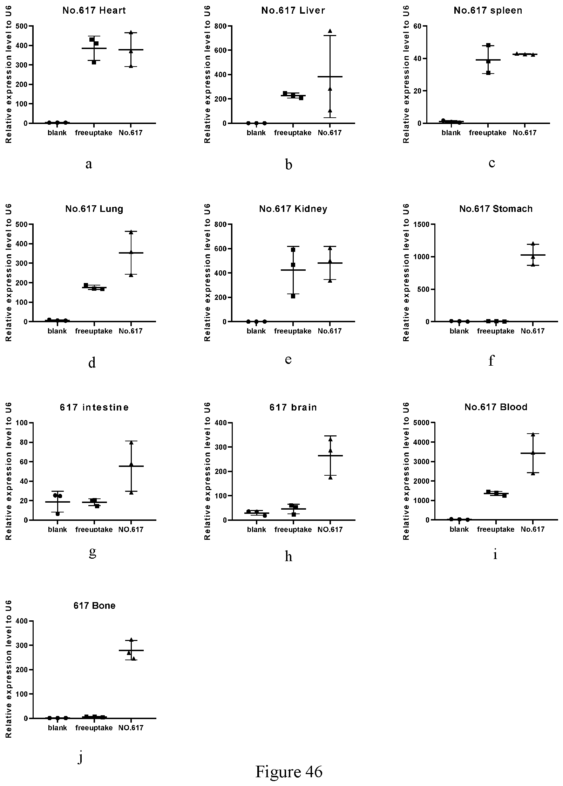

Figure 46 illustrates the results of the blank group, free uptake group, and lipid 617 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

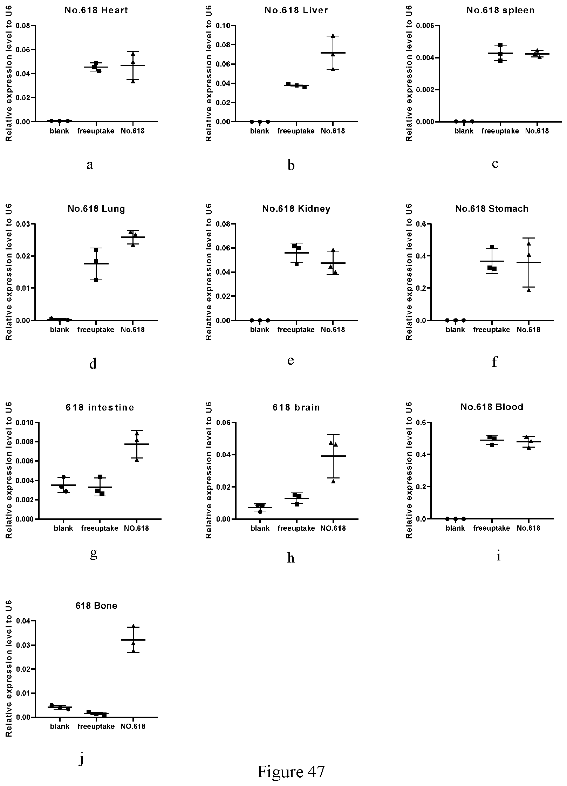

Figure 47 illustrates the results of the blank group, free uptake group, and lipid 618 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

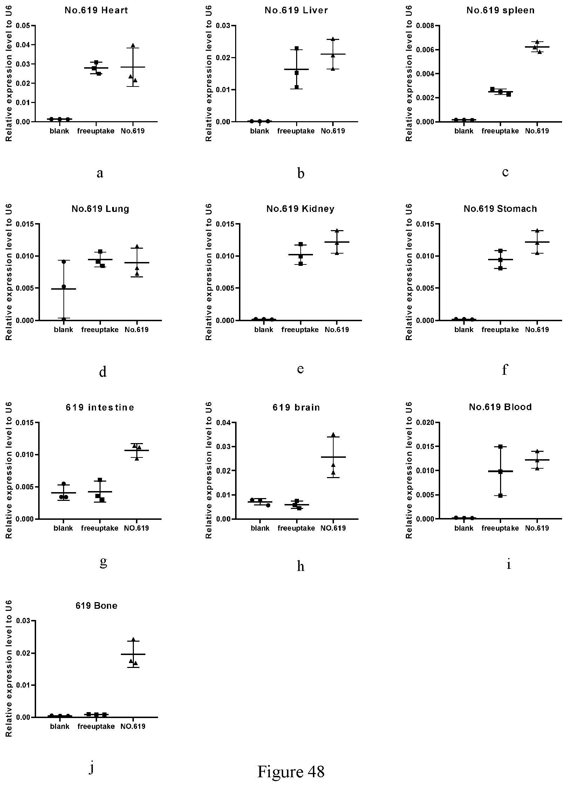

Figure 48 illustrates the results of the blank group, free uptake group, and lipid 619 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

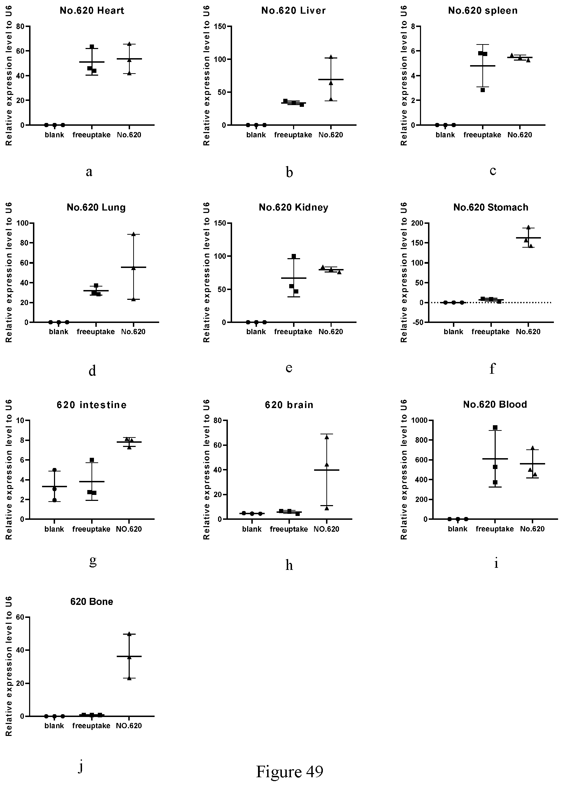

Figure 49 illustrates the results of the blank group, free uptake group, and lipid 620 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

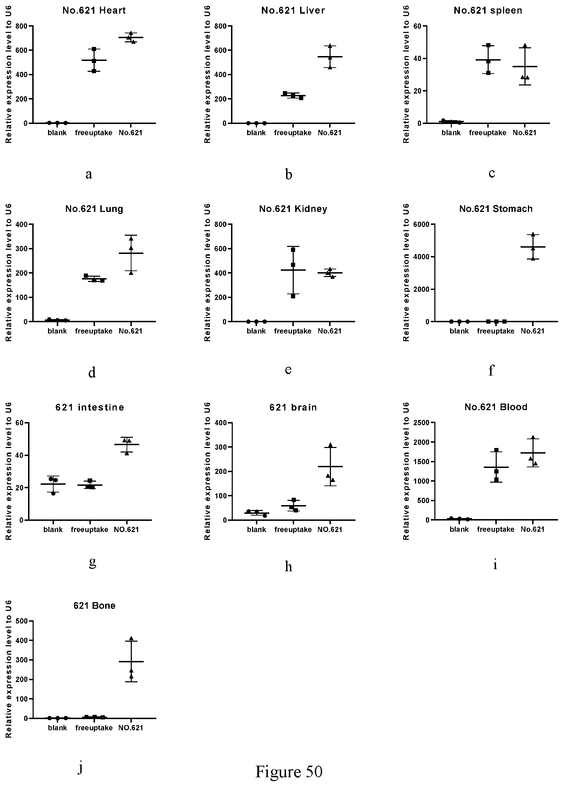

Figure 50 illustrates the results of the blank group, free uptake group, and lipid 621 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

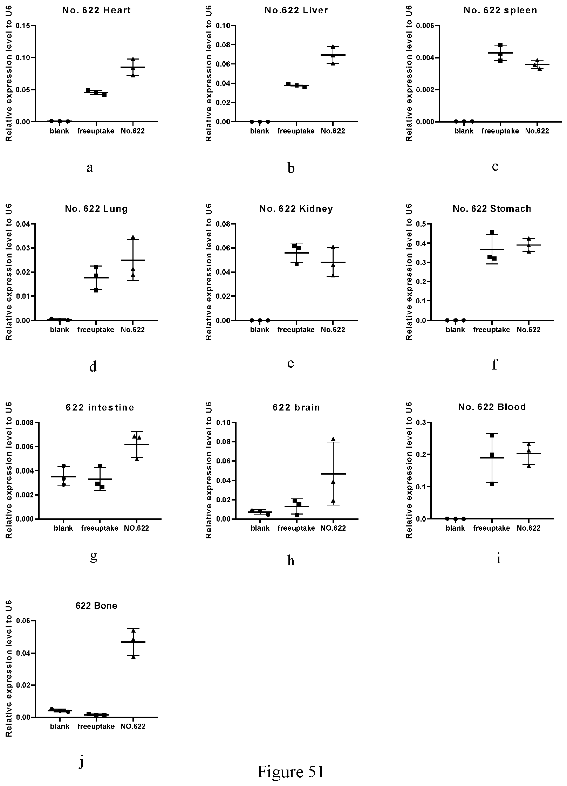

Figure 51 illustrates the results of the blank group, free uptake group, and lipid 622 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

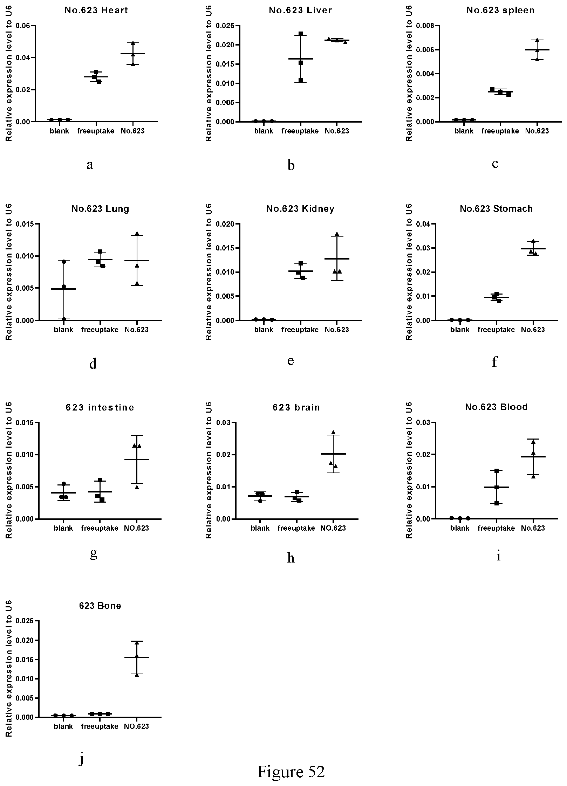

Figure 52 illustrates the results of the blank group, free uptake group, and lipid 623 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

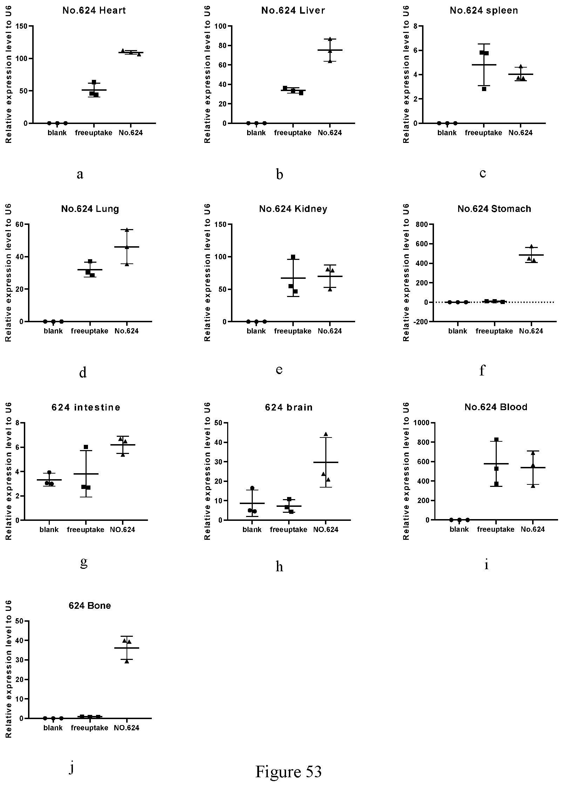

Figure 53 illustrates the results of the blank group, free uptake group, and lipid 624 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

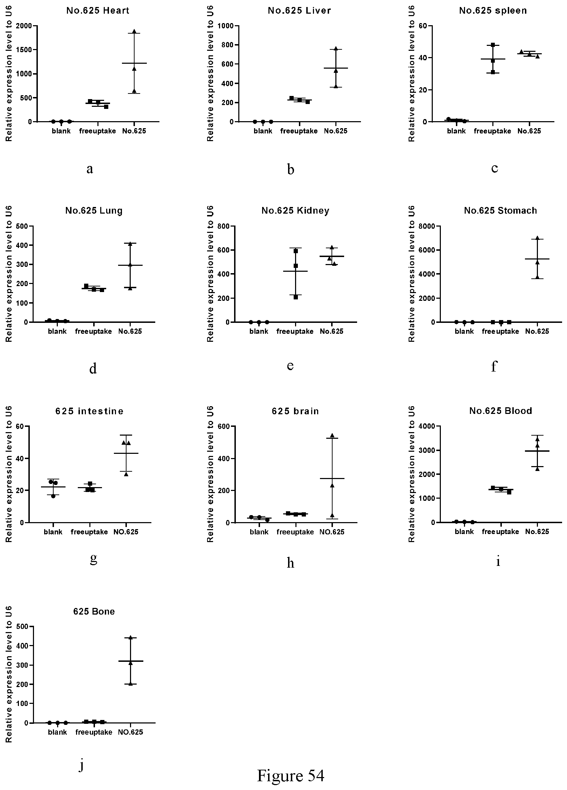

Figure 54 illustrates the results of the blank group, free uptake group, and lipid 625 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

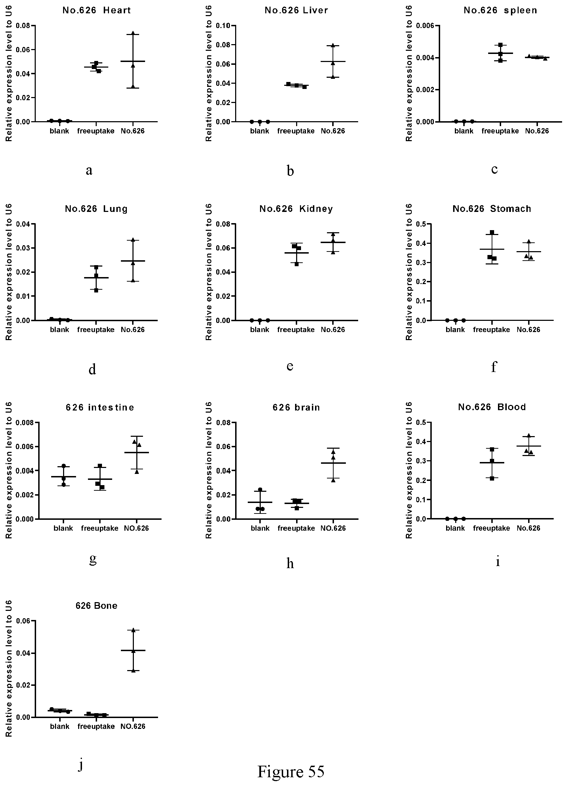

Figure 55 illustrates the results of the blank group, free uptake group, and lipid 626 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

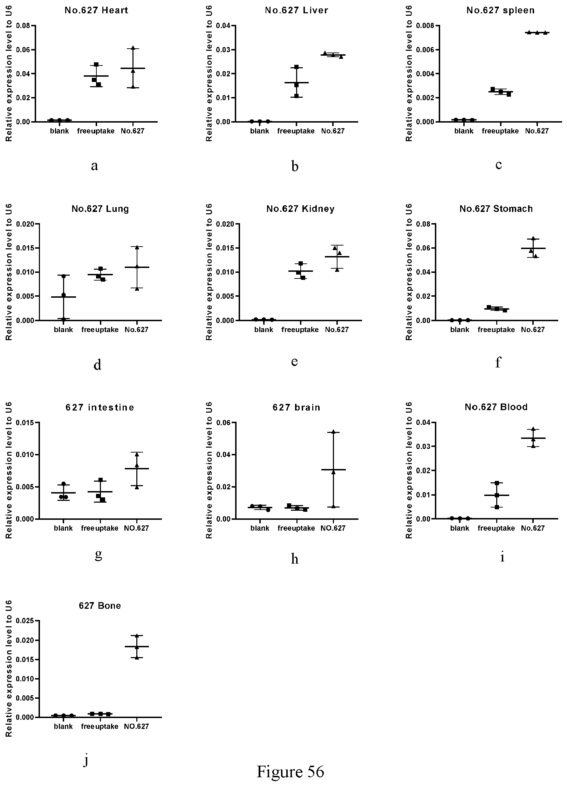

Figure 56 illustrates the results of the blank group, free uptake group, and lipid 627 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

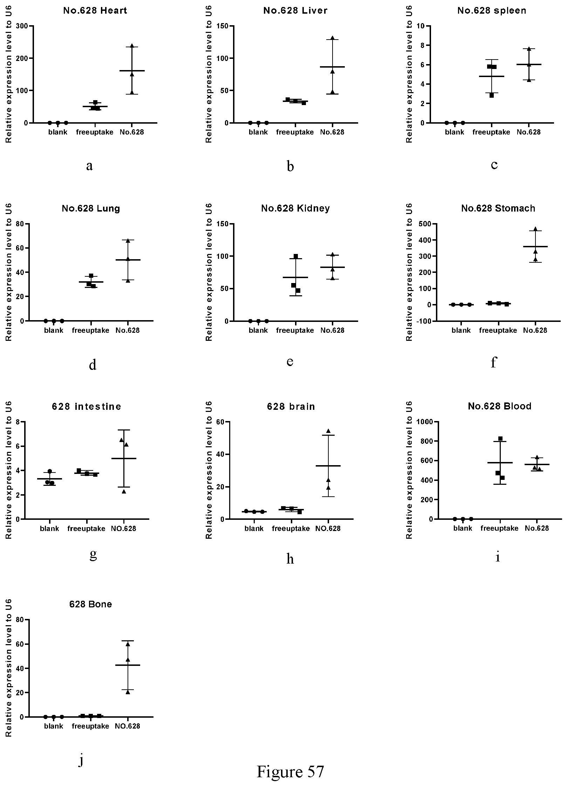

Figure 57 illustrates the results of the blank group, free uptake group, and lipid 628 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

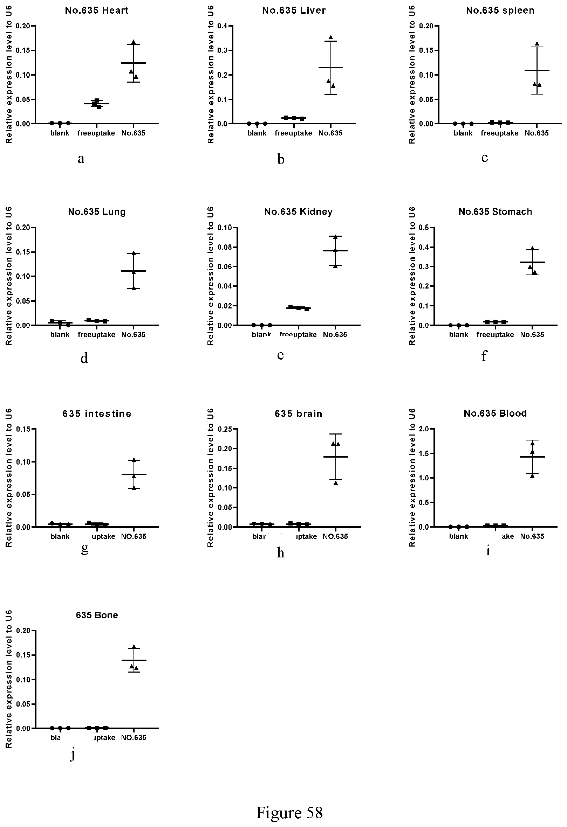

Figure 58 illustrates the results of the blank group, free uptake group, and lipid 635 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

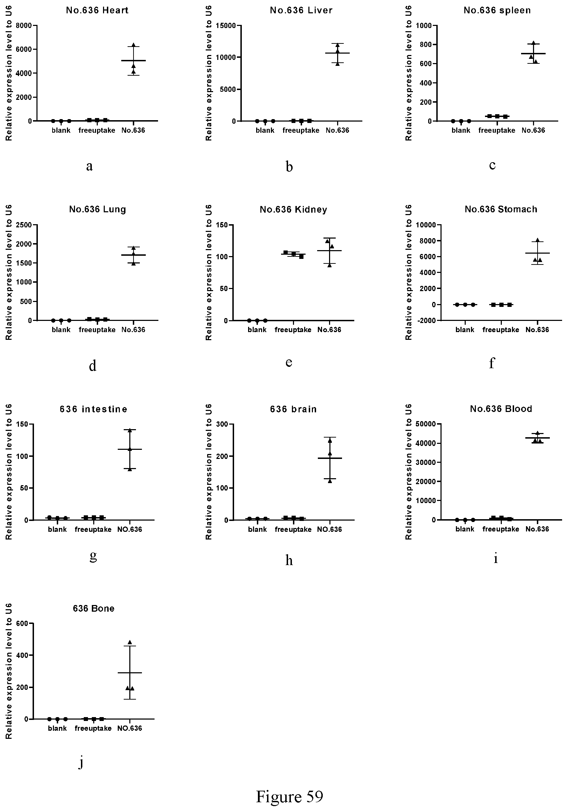

Figure 59 illustrates the results of the blank group, free uptake group, and lipid 636 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), and femur (j) of mouse.

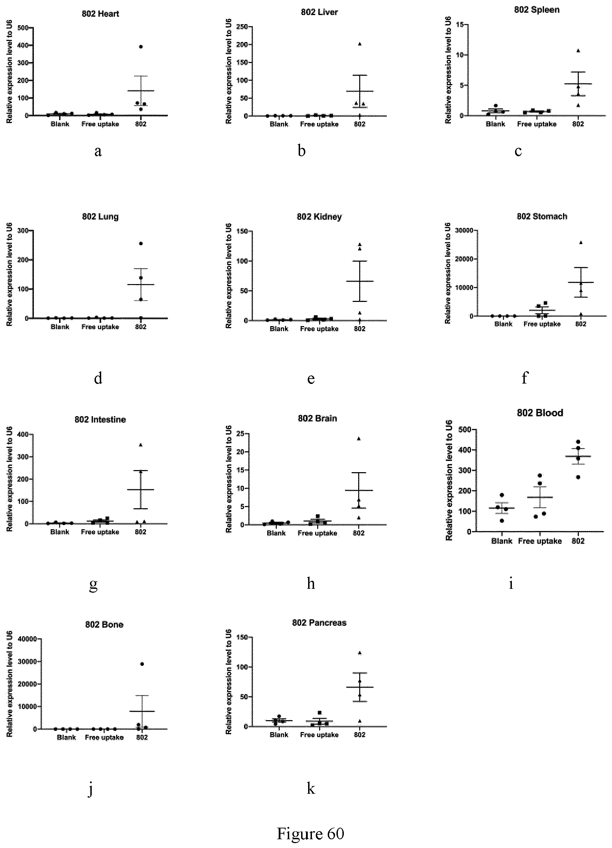

Figure 60 illustrates the results of the blank group, free uptake group, and lipid 802 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

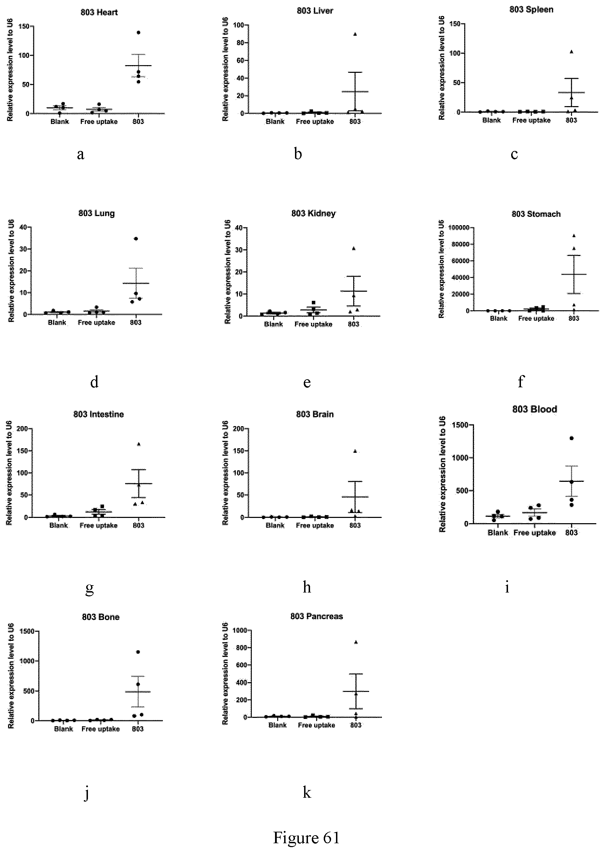

Figure 61 illustrates the results of the blank group, free uptake group, and lipid 803 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

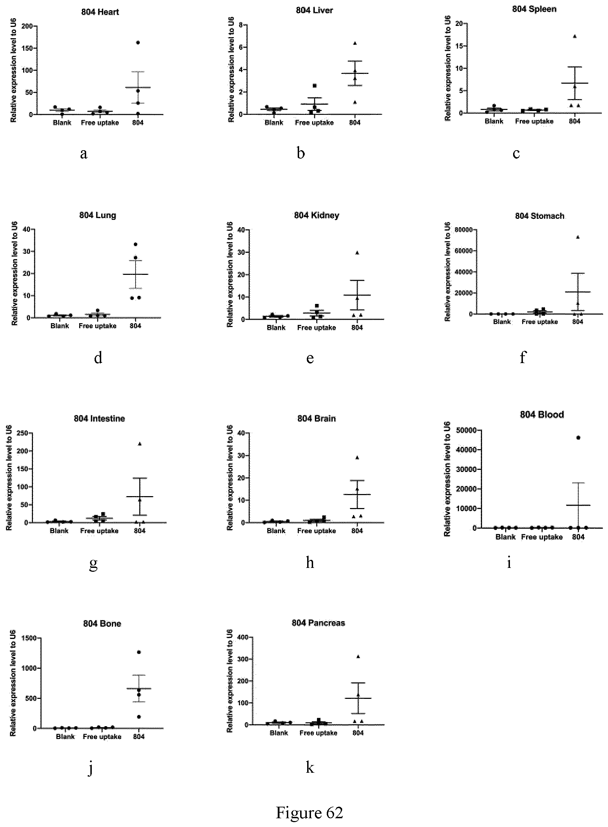

Figure 62 illustrates the results of the blank group, free uptake group, and lipid 804 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

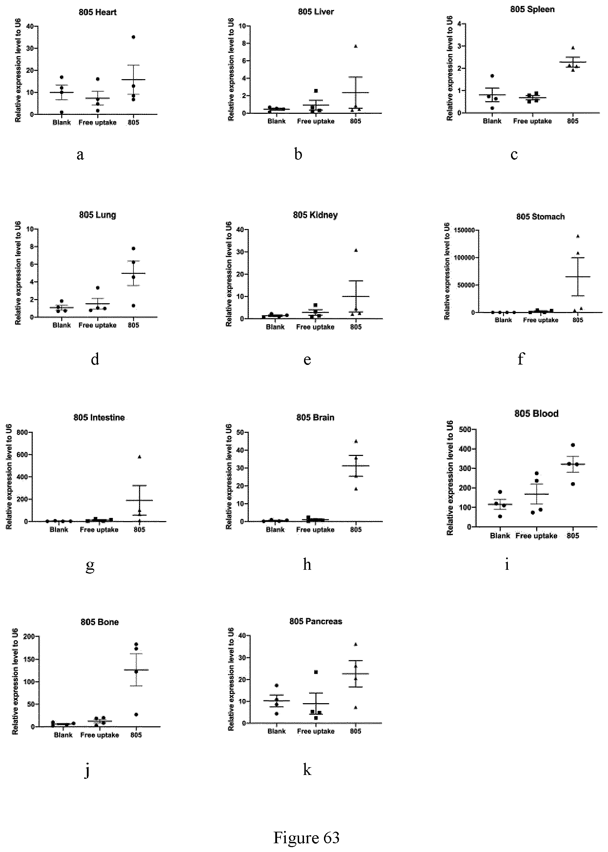

Figure 63 illustrates the results of the blank group, free uptake group, and lipid 805 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

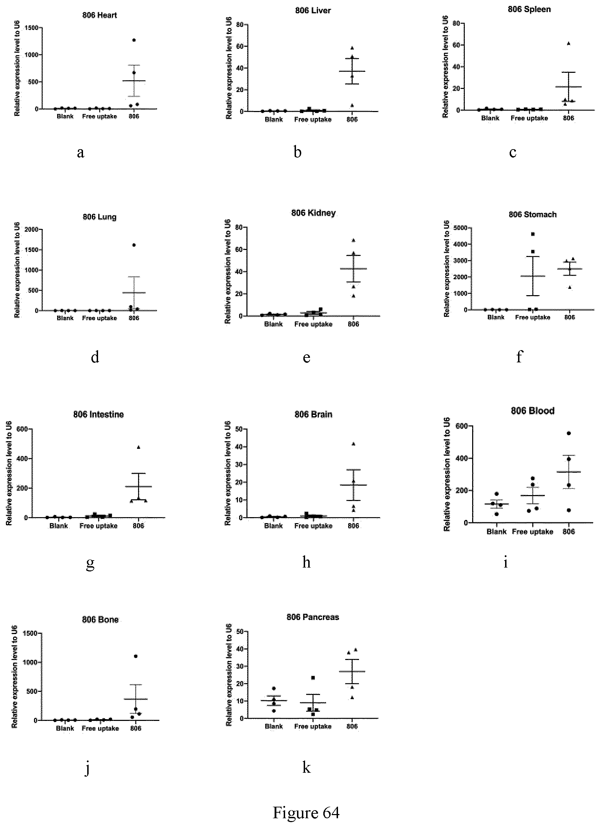

Figure 64 illustrates the results of the blank group, free uptake group, and lipid 806 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

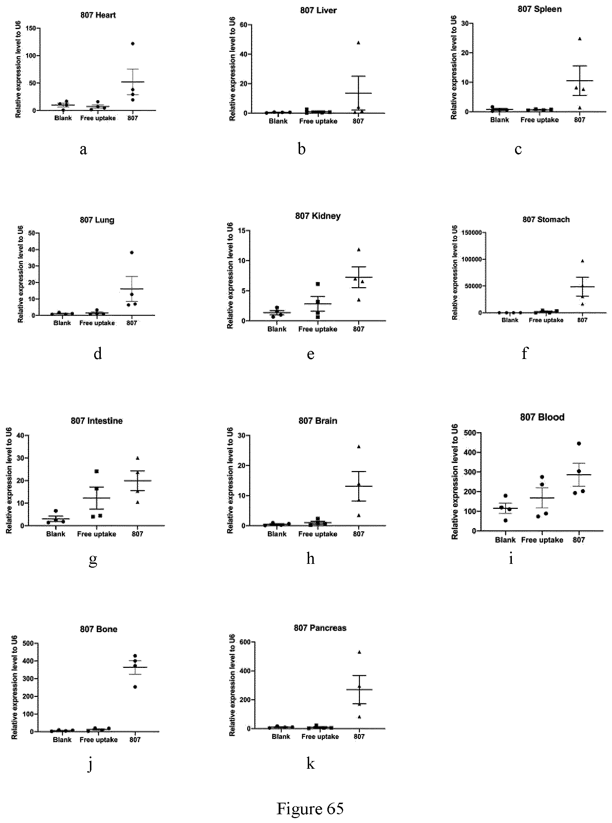

Figure 65 illustrates the results of the blank group, free uptake group, and lipid 807 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

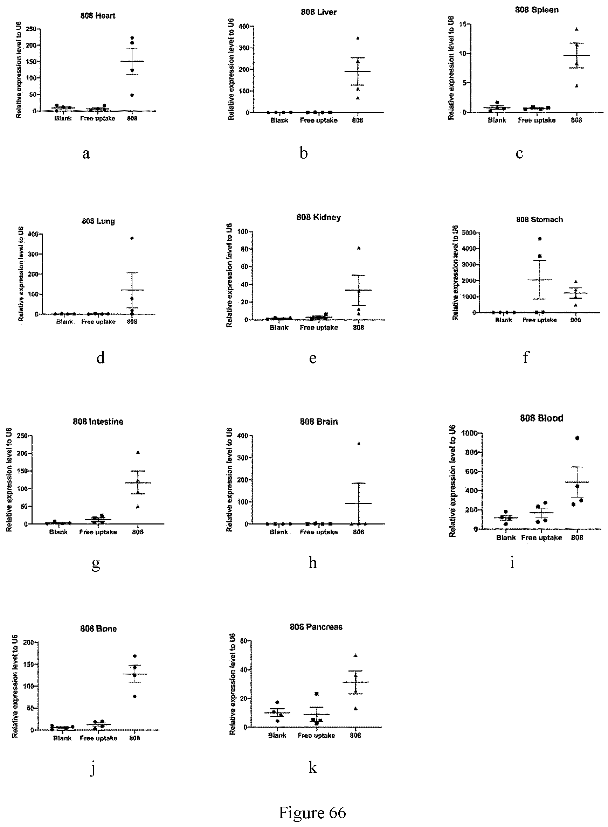

Figure 66 illustrates the results of the blank group, free uptake group, and lipid 808 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

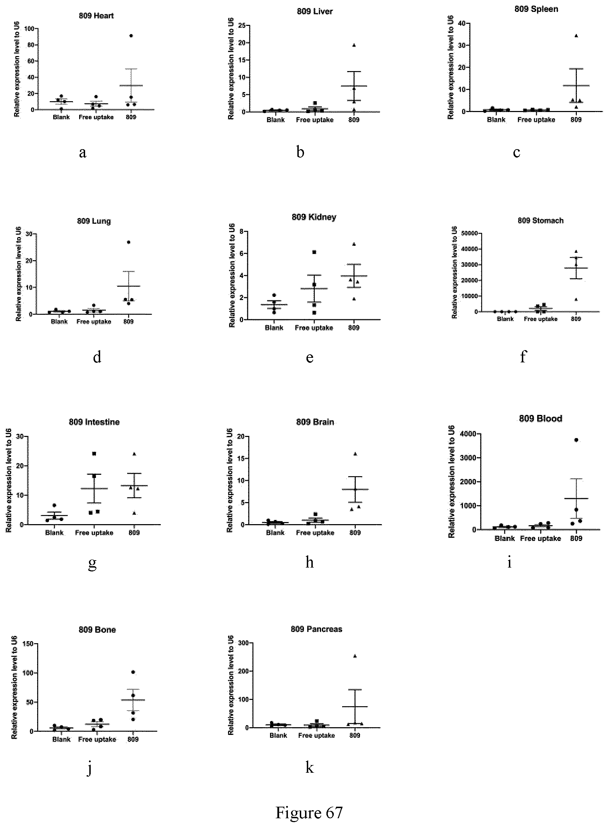

Figure 67 illustrates the results of the blank group, free uptake group, and lipid 809 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

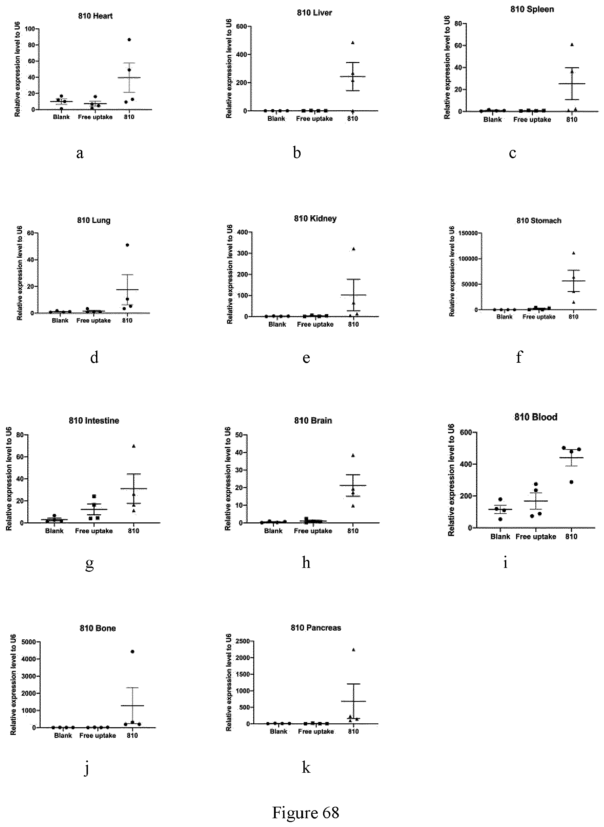

Figure 68 illustrates the results of the blank group, free uptake group, and lipid 810 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

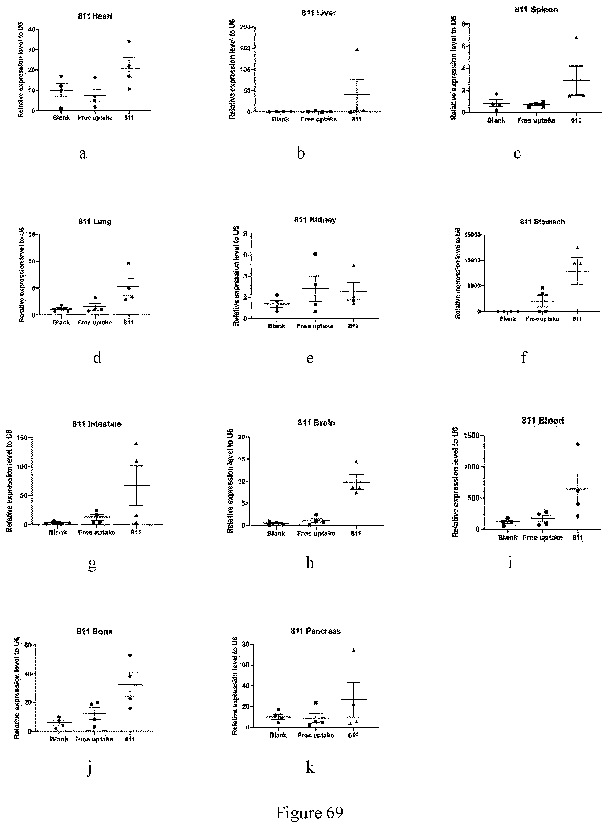

Figure 69 illustrates the results of the blank group, free uptake group, and lipid 811 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

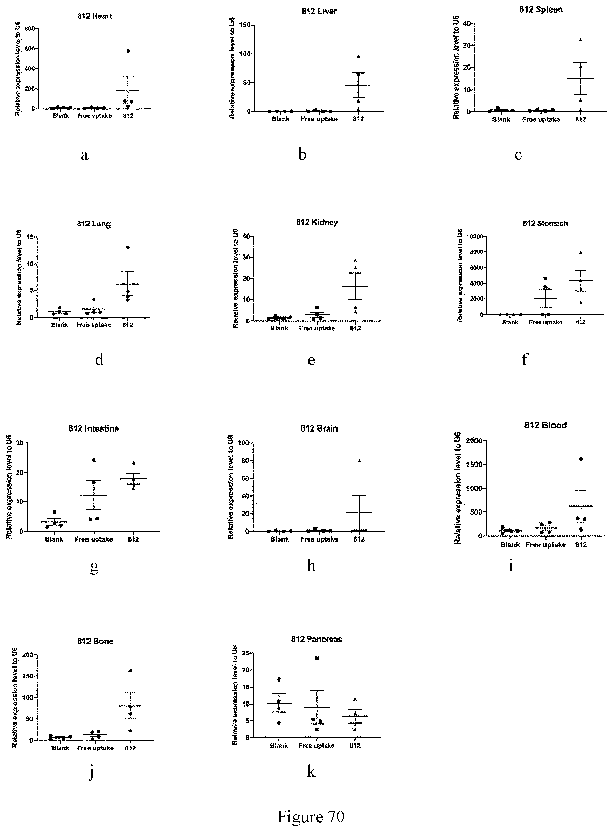

Figure 70 illustrates the results of the blank group, free uptake group, and lipid 812 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

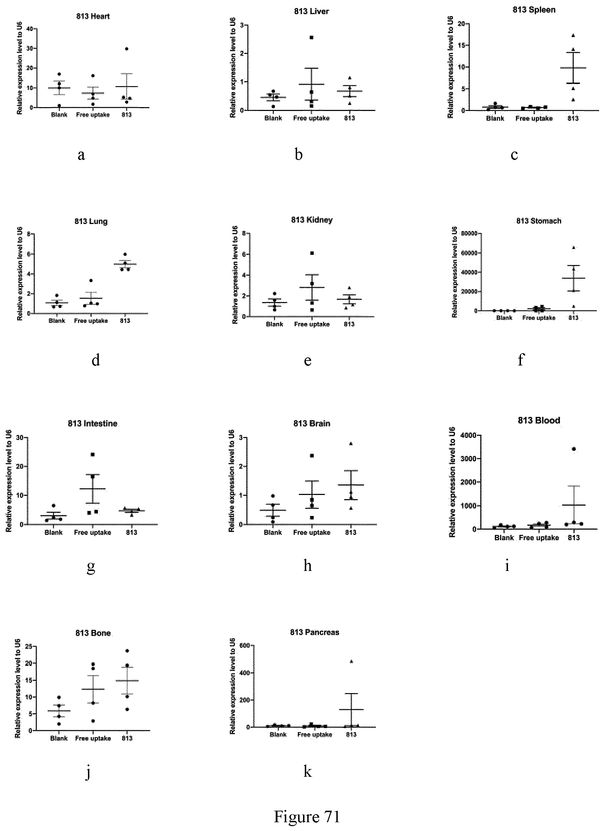

Figure 71 illustrates the results of the blank group, free uptake group, and lipid 813 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

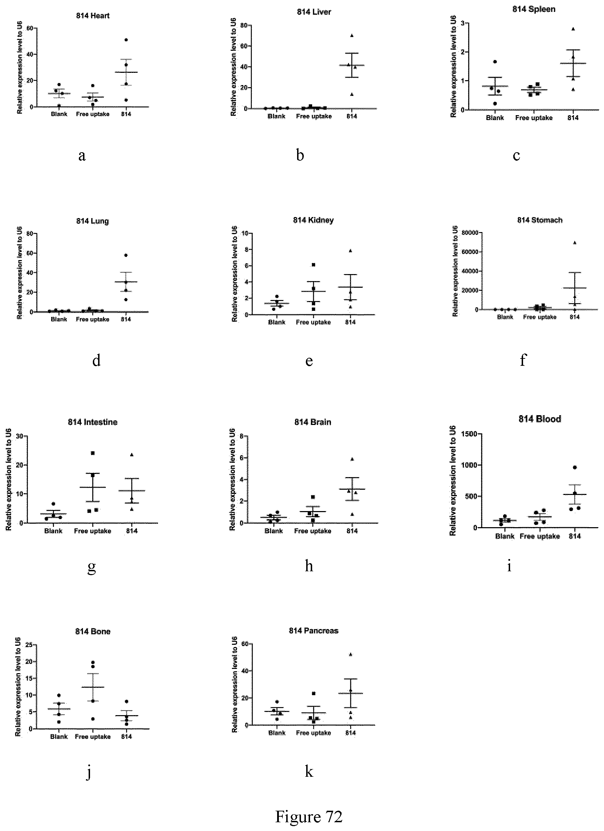

Figure 72 illustrates the results of the blank group, free uptake group, and lipid 814 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

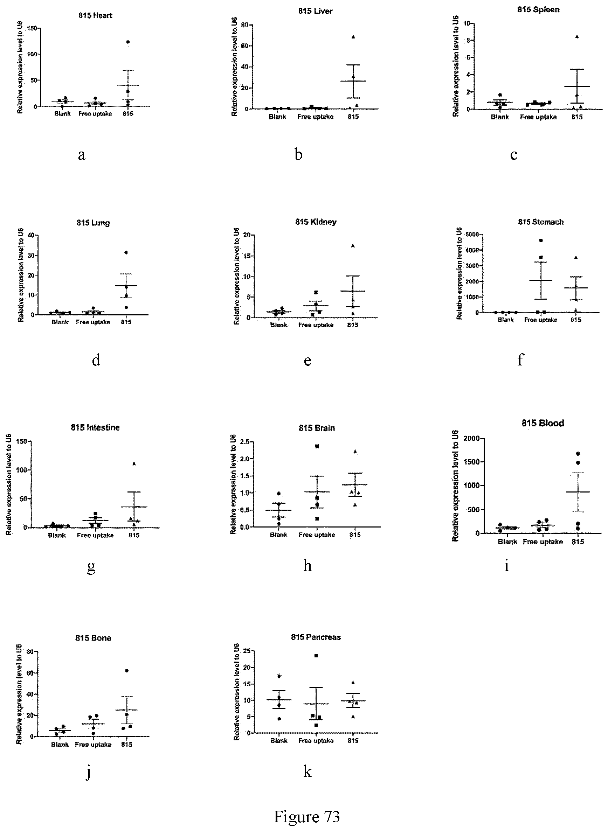

Figure 73 illustrates the results of the blank group, free uptake group, and lipid 815 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

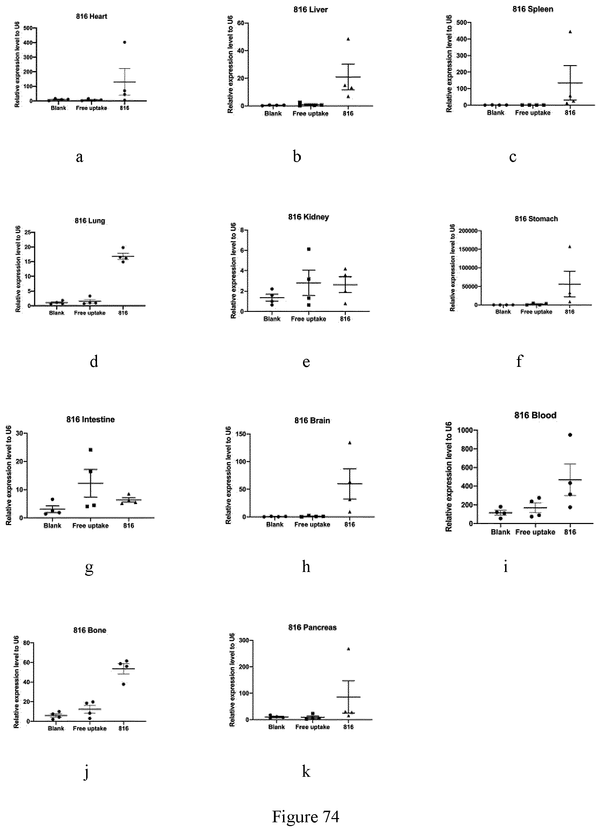

Figure 74 illustrates the results of the blank group, free uptake group, and lipid 816 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

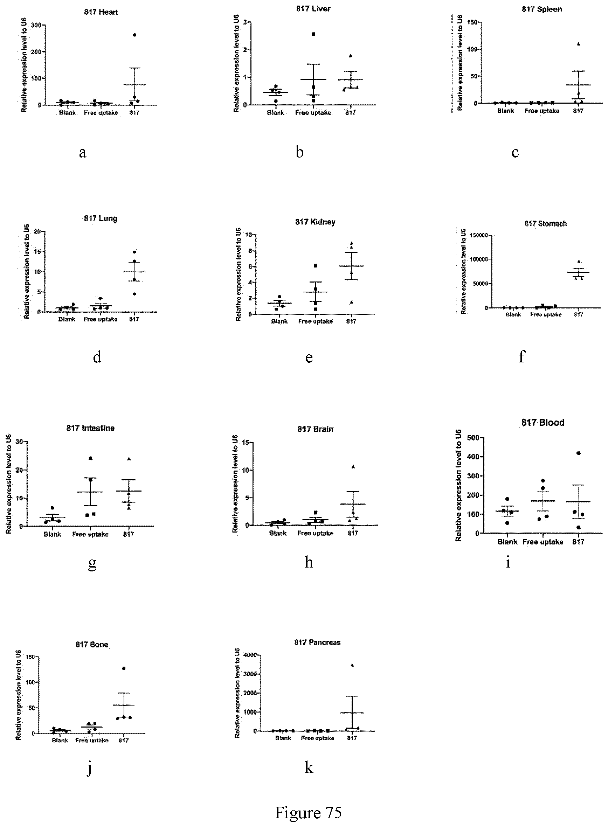

Figure 75 illustrates the results of the blank group, free uptake group, and lipid 817 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

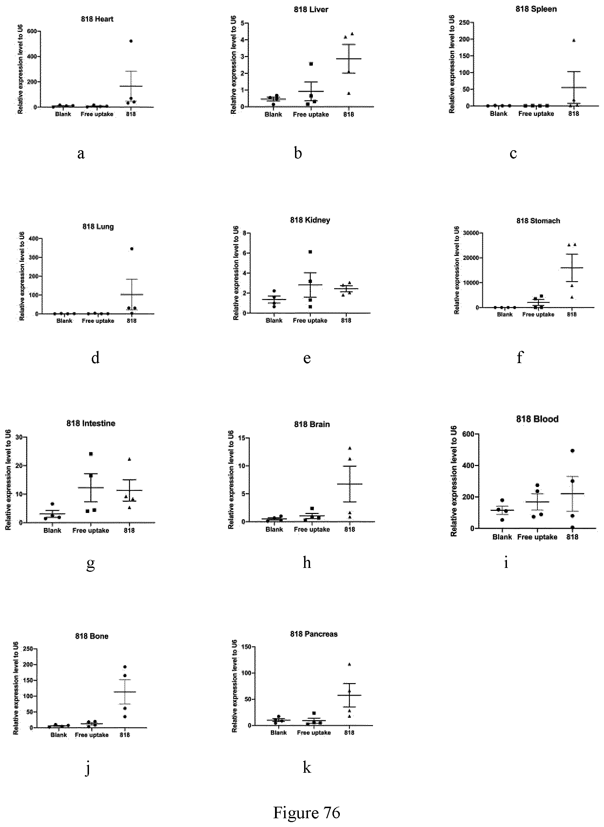

Figure 76 illustrates the results of the blank group, free uptake group, and lipid 818 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

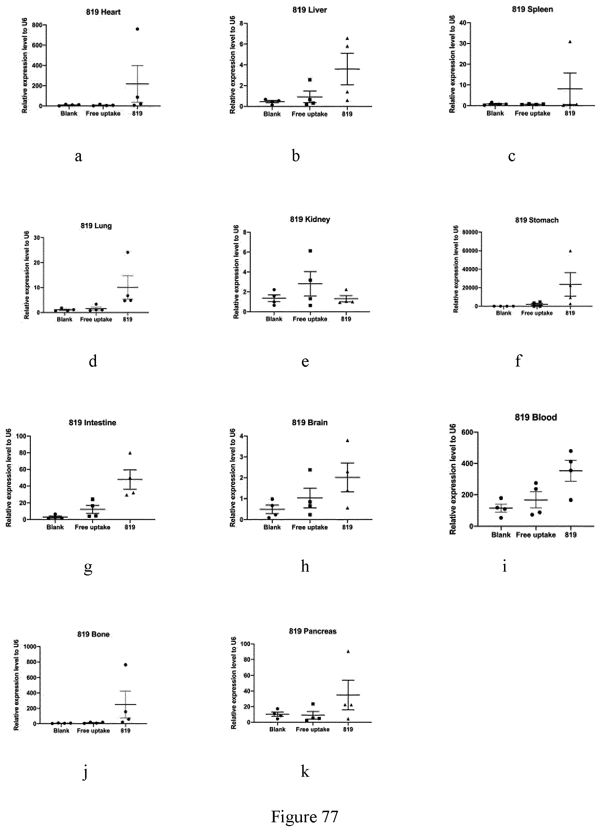

Figure 77 illustrates the results of the blank group, free uptake group, and lipid 819 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

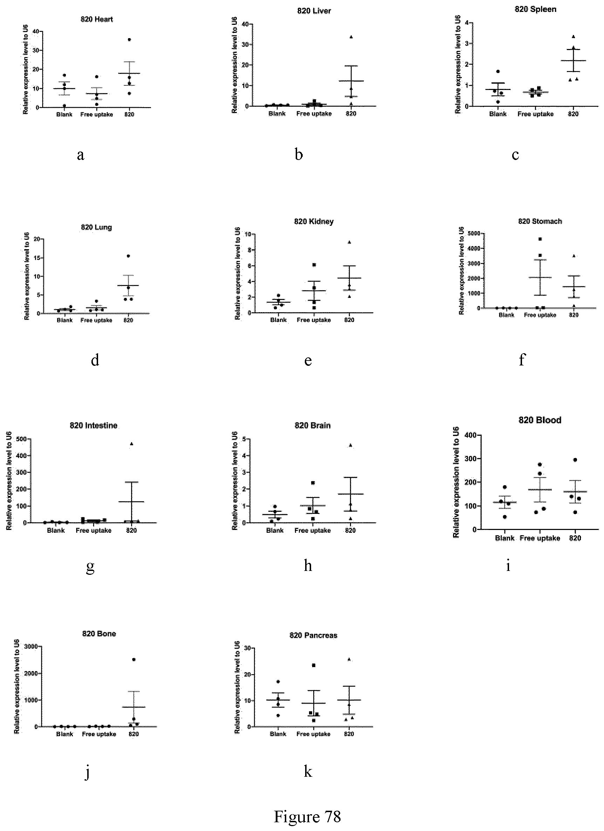

Figure 78 illustrates the results of the blank group, free uptake group, and lipid 820 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

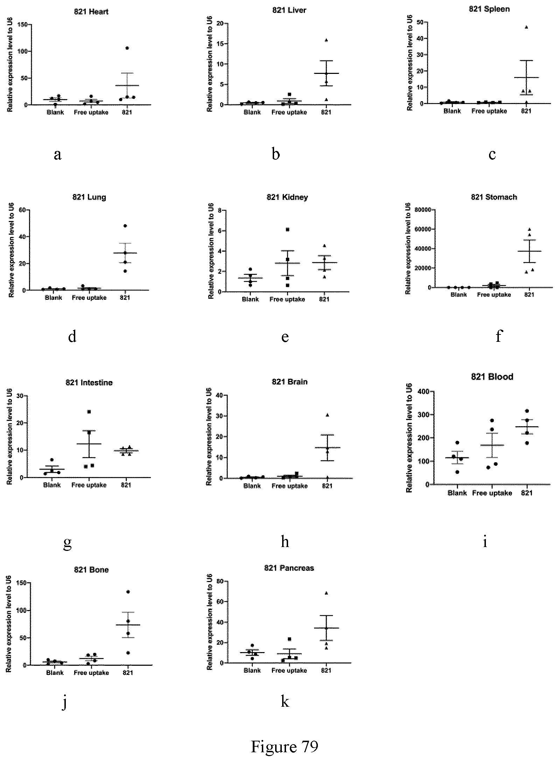

Figure 79 illustrates the results of the blank group, free uptake group, and lipid 821 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

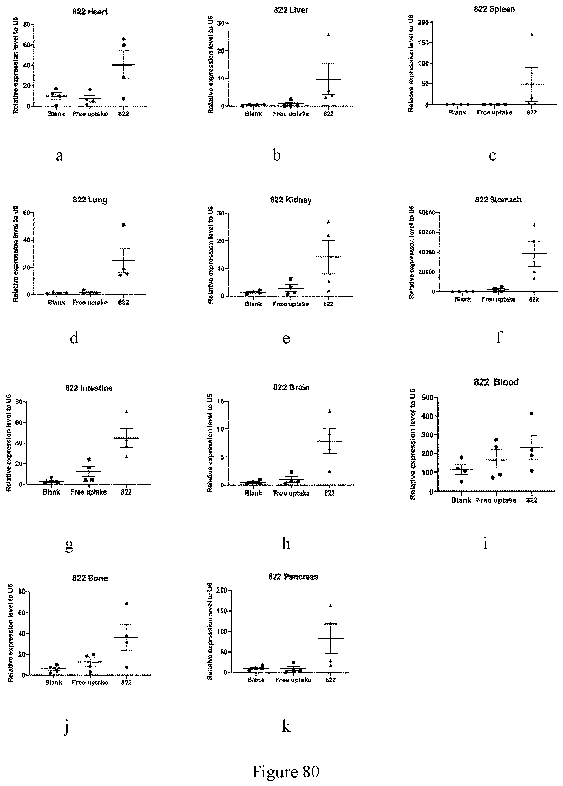

Figure 80 illustrates the results of the blank group, free uptake group, and lipid 822 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

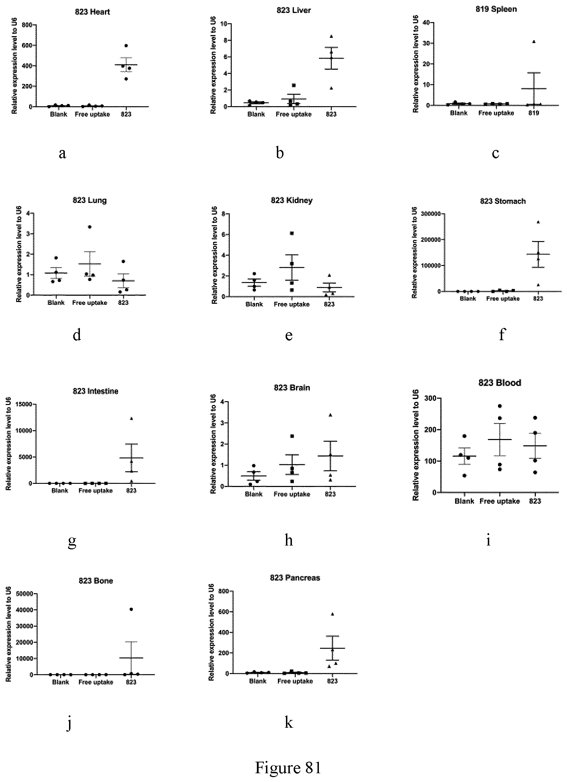

Figure 81 illustrates the results of the blank group, free uptake group, and lipid 823 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

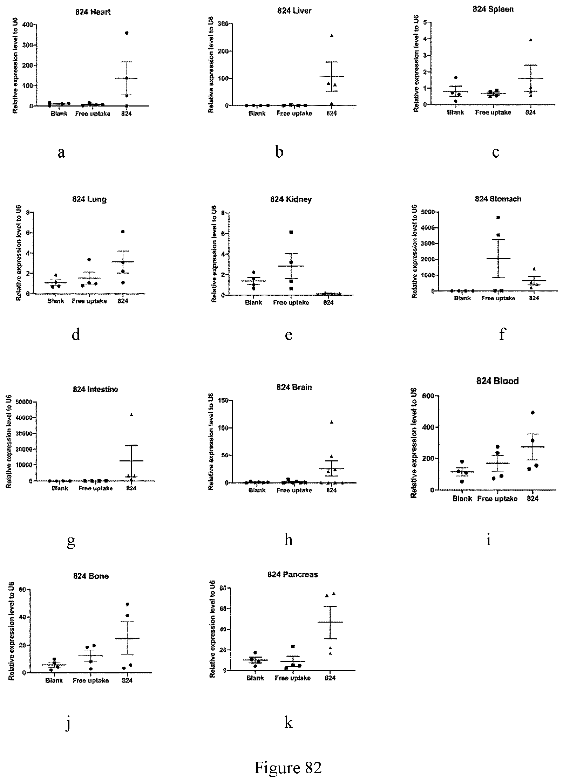

Figure 82 illustrates the results of the blank group, free uptake group, and lipid 824 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

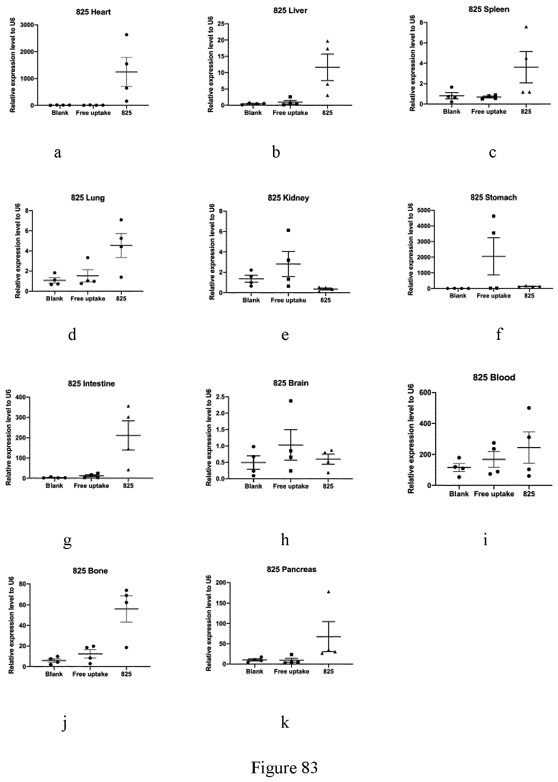

Figure 83 illustrates the results of the blank group, free uptake group, and lipid 825 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

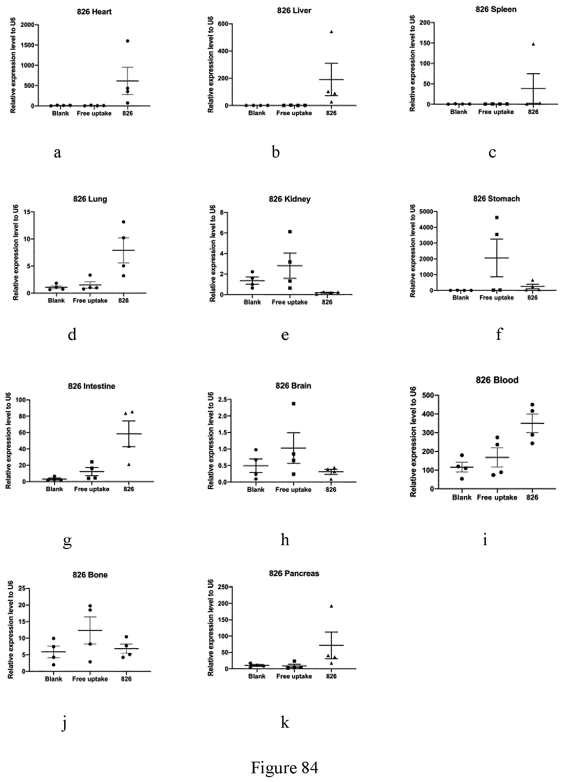

Figure 84 illustrates the results of the blank group, free uptake group, and lipid 826 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

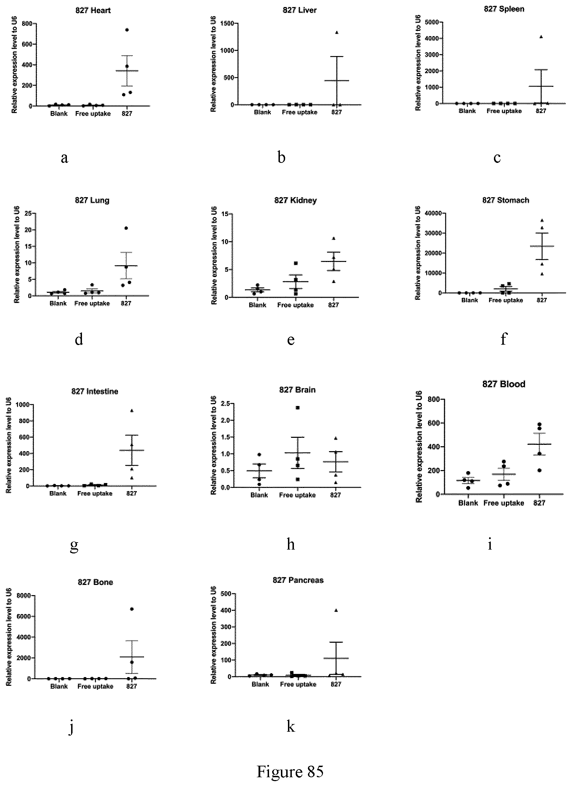

Figure 85 illustrates the results of the blank group, free uptake group, and lipid 827 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

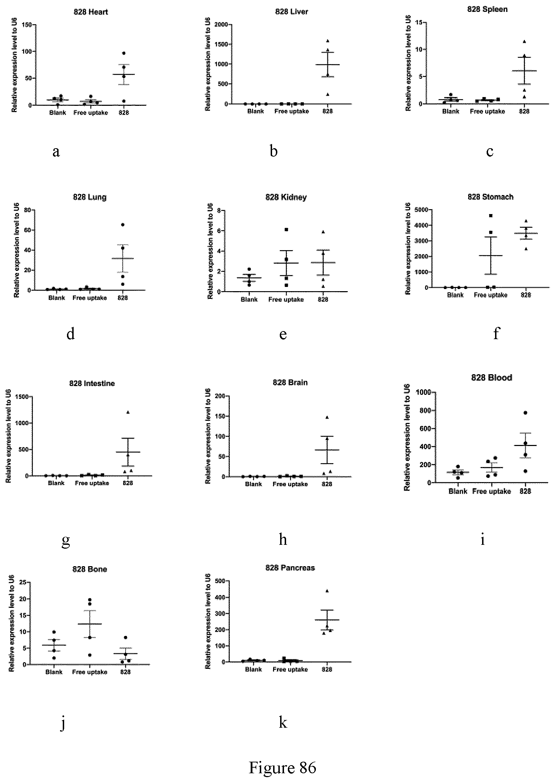

Figure 86 illustrates the results of the blank group, free uptake group, and lipid 828 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

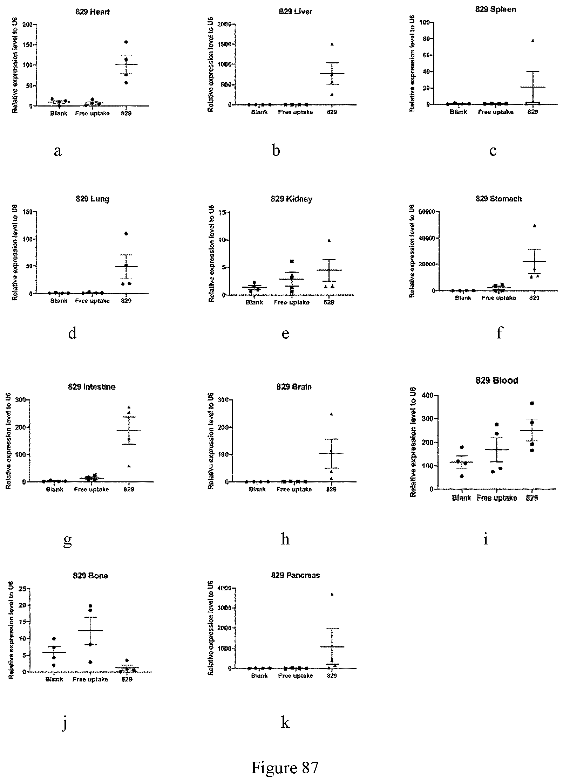

Figure 87 illustrates the results of the blank group, free uptake group, and lipid 829 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

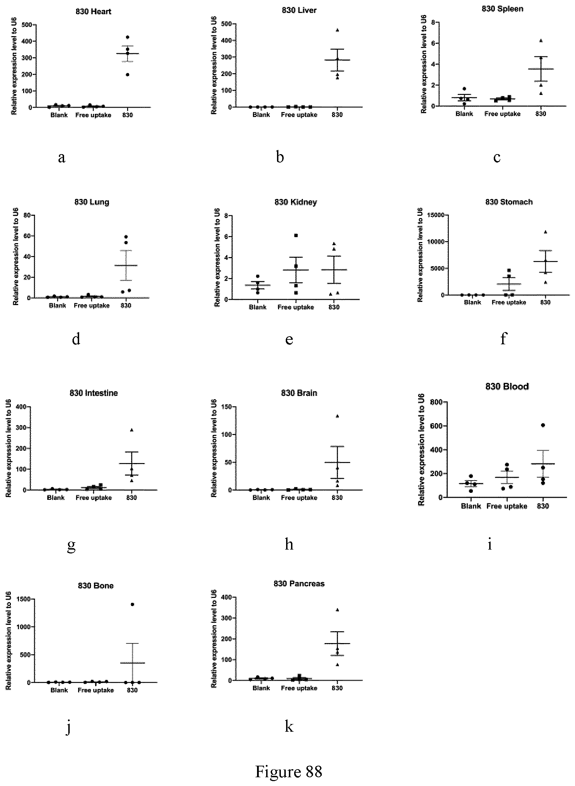

Figure 88 illustrates the results of the blank group, free uptake group, and lipid 830 for delivery of PGY-sRNA-26 into heart tissue (a), liver tissue (b), spleen tissue (c), lung tissue (d), kidney tissue (e), stomach tissue (f), intestine tissue (g), brain tissue (h), blood (i), femur (j), and pancreas tissue (k) of mouse.

### DETAILED DESCRIPTION OF THE INVENTION

The present invention is based, at least in part, on the suprising discovery by the inventor of the present application after extensive experimentation that there are some lipid components in traditional Chinese medicine, and the lipids derived from extracts of traditional Chinese medicine can facilitate the absorption/entry of nucleic acids such as small RNA into cells and/or in vivo target sites of a subject in need thereof. For the purposes of the present invention, these lipid components may also be synthetic.

The present application is described in detail below with reference to some embodiments of the present application which show the structure and formula in an exemplary way. Although the present application is described in conjunction with listed embodiments, it should be understood that it is not intended to limit the present application to those embodiments described. Instead, the present application is intended to cover all alternatives, modifications and equivalents falling within the scope limited by the Claims. Those skilled in the art will recognize other methods and materials similar to or equivalent to those described herein that may be used to implement the present application, and the present application is not limited in any way to the methods and materials described herein. Where the referenced literature and similar materials differ from or contradict the description of the present application (including limited terms, use of terms, techniques, etc.), the description of the present Application shall prevail.

It should also be understood that certain features described in different embodiments may also be provided in combination in a single embodiment. Conversely, multiple features described in a single embodiment may also be provided separately or in any appropriate subcombination.

It should also be understood that the numerical points recorded herein are intended to include the numerical points themselves, as well as the numerical ranges between any two of the recorded points (as these numerical ranges have been listed separately).

### Definition

As used herein, the following definitions shall apply unless otherwise stated. For the purposes of the present application, chemical elements are identified according to the Periodic Table of Elements (CAS Edition) of the Handbook of Chemistry and Physics (75th Edition). In addition, the general principles of organic chemistry and specific functional parts and reactivity are described in "Organic Chemistry", Thomas Sorrell, University Science Books, Sausalito: 1999 and "March's Advanced Organic Chemistry," 5th Ed., Smith, M.B. and March, J., John Wiley & Sons, New York: 2001, the entirety of which is incorporated by reference into the present application.

Linking substituents are described herein. When the structure clearly requires a linking group, it should be understood that the Markush variable listed for that group is a linking group. For example, if the structure requires a linking group and the definition of a Markush group for that variable lists "alkyl", it should be understood that "alkyl" means an alkylene linking group.

The term "substituted" as used herein, whether or not preceded by the term "optionally", represents that one or more of the hydrogens in a given group are replaced by suitable substituents. Unless otherwise stated, the "substituted" group may have a suitable substituent at each position of the group suitable for substitution, and the substituent may be the same or different at each position when more than one position in any given structure can be substituted by more than one substituent selected from a particular group. The combinations of substituents contemplated by the present invention are preferably those resulting in the formation of stable or chemically viable compounds. The term "stable" as used herein refers to a compound that remains substantially unchanged when subjected to conditions that permit its production, detection, and (in some embodiments) its recovery, purification, and use for one or more of the purposes disclosed herein. Unless specifically referred to as "unsubstituted", the chemical moieties described herein are to be understood to include substituents. For example, when referring to "aryl", it includes substituted aryl and unsubstituted aryl.

When a bond to a substituent is shown to corss a bond connecting two atoms in a ring, such substituent may be bonded to any atom in the ring. When a substituent is listed without indicating the atom via which such substituent is bonded to the rest of the compound of a given formula, such substituent may be bonded via any atomic bond in such formula. Combinations of substituents and/or variables are permissible, but only if such combination results in a stable compound.

The term "Ci-j" as used herein indicates a range of the number of carbon atoms, where i and j are integers and j is greater than i, and the range of the number of carbon atoms includes the endpoints (i.e., i and j) and every integer point between the endpoints. For example, C₁₋₆ indicates a range of 1 to 6 carbon atoms, including 1 carbon atom, 2 carbon atoms, 3 carbon atoms, 4 carbon atoms, 5 carbon atoms, and 6 carbon atoms. In some embodiments, the term "C₁₋₁₂" indicates 1 to 12, particularly 1 to 10, particularly 1 to 8, particularly 1 to 6, particularly 1 to 5, particularly 1 to 4, particularly 1 to 3, or particularly 1 to 2 carbon atoms.

The term "alkyl" as used herein, whether as part of another term or used independently, refers to saturated straight or branched alkyl groups. The term "Cᵢ₋ⱼ alkyl" refers to alkyl having i to j carbon atoms. In some embodiments, alkyl contains 1 to 20 carbon atoms. In some embodiments, alkyl contains 5 to 20 carbon atoms. In some embodiments, alkyl contains 1 to 20 carbon atoms, 1 to 19 carbon atoms, 1 to 18 carbon atoms, 1 to 17 carbon atoms, 1 to 16 carbon atoms, 1 to 15 carbon atoms, 1 to 14 carbon atoms, 1 to 13 carbon atoms, 1 to 12 carbon atoms, 1 to 11 carbon atoms, 1 to 10 carbon atoms, 1 to 9 carbon atoms, 1 to 8 carbon atoms, 1 to 7 carbon atoms, 1 to 6 carbon atoms, 1 to 5 carbon atoms, 1 to 4 carbon atoms, 1 to 3 carbon atoms, or 1 to 2 carbon atoms. Examples of alkyl include, but are not limited to, methyl, ethyl, 1-propyl (n-propyl), 2-propyl (isopropyl), 1-butyl (n-butyl), 2-methyl-1-propyl (isobutyl), 2-butyl (neobutyl), 2-methyl-2-propyl (tert-butyl), 1-pentyl (n-pentyl), 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 4-methyl-2-pentyl, 3-methyl-3-pentyl, 2-methyl-3-pentyl, 2-methyl-3-pentyl, 2,3-dimethyl-2-butyl, 3,3-dimethyl-2-butyl, 1-heptyl, 1-octyl, 1-nonyl, 1-decyl, etc. Examples of "C₁₋₂₄ alkyl" include, but are not limited to, methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, and eicosyl.

The term "alkenyl" as used herein, whether as part of another term or used independently, refers to a straight or branched hydrocarbon radical having at least one carbon-carbon double bond, which may be optionally substituted independently by one or more substituents described herein, and includes radicals having "cis" and "trans" orientations, or alternatively "E" and "Z" orientations. In some embodiments, alkenyl contains 2 to 20 carbon atoms. In some embodiments, alkenyl contains 5 to 20 carbon atoms. In some embodiments, alkenyl contains 2 to 20 carbon atoms, 2 to 19 carbon atoms, 2 to 18 carbon atoms, 2 to 17 carbon atoms, 2 to 16 carbon atoms, 2 to 15 carbon atoms, 2 to 14 carbon atoms, 2 to 13 carbon atoms, 2 to 12 carbon atoms, 2 to 11 carbon atoms, 2 to 10 carbon atoms, 2 to 9 carbon atoms, 2 to 8 carbon atoms, 2 to 7 carbon atoms, 2 to 6 carbon atoms, 2 to 5 carbon atoms, 2 to 4 carbon atoms, or 2 to 3 carbon atoms. In some embodiments, alkenyl contains more than one carbon-carbon double bond. It should be understood that where alkenyl contains more than one carbon-carbon double bond, the double bonds may be separated from each other or conjugated. In some embodiments, alkenyl contains 5 carbon-carbon double bonds, 4 carbon-carbon double bonds, 3 carbon-carbon double bonds, 2 carbon-carbon double bonds, or 1 carbon-carbon double bond.

The term "cycloalkyl" as used herein, whether as part of another term or used independently, refers to a saturated monocyclic and polycyclic ring system wherein all ring atoms are carbon, and which contains at least three ring-forming carbon atoms. In some embodiments, cycloalkyl may contain 3 to 20 ring-forming carbon atoms, 3 to 19 ring-forming carbon atoms, 3 to 18 ri ring-forming ng carbon atoms, 3 to 17 ring-forming carbon atoms, 3 to 16 ring-forming carbon atoms, 3 to 15 ring-forming carbon atoms, 3 to 14 ring-forming carbon atoms, 3 to 13 ring-forming carbon atoms, 3 to 12 ring-forming carbon atoms, 3 to 11 ring-forming carbon atoms, 3 to 10 ring-forming carbon atoms, 3 to 9 ring-forming carbon atoms, 3 to 8 ring-forming carbon atoms, 3 to 7 ring-forming carbon atoms, 3 to 6 ring-forming carbon atoms, 3 to 5 ring-forming carbon atoms, 4 to 20 ring-forming carbon atoms, 4 to 19 ring-forming carbon atoms, 4 to 18 ring-forming carbon atoms, 4 to 17 ring-forming carbon atoms, 4 to 16 ring-forming carbon atoms, 4 to 15 ring-forming carbon atoms, 4 to 14 ring-forming carbon atoms, 4 to 13 ring-forming carbon atoms, 4 to 12 ring-forming carbon atoms, 4 to 11 ring-forming carbon atoms, 4 to 10 ring-forming carbon atoms, 4 to 9 ring-forming carbon atoms, 4 to 8 ring-forming carbon atoms, 4 to 7 ring-forming carbon atoms, 4 to 6 ring-forming carbon atoms, or 4 to 5 ring-forming carbon atoms. Cycloalkyl may optionally be substituted at one or more ring positions by one or more substituents described herein. Cycloalkyl may be saturated, partially unsaturated, or fully unsaturated. In some embodiments, cycloalkyl may be a saturated cyclic alkyl. In some embodiments, cycloalkyl may be an unsaturated cyclic alkyl group containing at least one double or triple bond in the ring system.

The term "cycloalkenyl" as used herein, whether as part of another term or used independently, refers to a monocyclic and polycyclic ring system containing 3-20 ring-forming carbon atoms and at least one carbon-carbon double bond, wherein all ring atoms are carbon, provided that the size of the cycloalkenyl ring allows. In some embodiments, cycloalkenyl contains more than 1 carbon-carbon double bond. It should be understood that where the alkenyl group contains more than one carbon-carbon double bond, the double bonds may be separated from each other or conjugated. In some embodiments, cycloalkenyl contains 5 carbon-carbon double bonds, 4 carbon-carbon double bonds, 3 carbon-carbon double bonds, 2 carbon-carbon double bonds, or 1 carbon-carbon double bond.

As used herein, the term "heteroatom" refers to nitrogen, oxygen, sulfur or phosphorus and comprises any oxidized form of nitrogen or sulfur and any quaternized form of basic nitrogen.

The term "heteroalkyl" as used herein, whether as part of another term or used independently, refers to alkyl in which at least one carbon atom is substituted by a heteroatom selected from nitrogen, oxygen, sulfur or phosphorus, wherein the heteroatom may be positioned either at the end or in the middle of the alkyl. Heteroalkyl includes alkoxy (i.e., alkyl-O-), wherein the alkyl may contain 1 to 6 carbon atoms, 1 to 5 carbon atoms, 1 to 4 carbon atoms, 1 to 3 carbon atoms, or 1 to 2 carbon atoms. Examples of alkoxy include, but are not limited to, methoxy, ethoxy, propoxy (such as n-propoxy and isopropoxy), and tert-butoxy.

The term "heteroalkenyl" as used herein, whether as part of another term or used independently, refers to alkenyl in which at least one carbon atom is substituted by a heteroatom selected from nitrogen, oxygen, sulfur or phosphorus, wherein the heteroatom may be positioned either at the end or in the middle of the alkenyl.

The term "aryl" as used herein, whether as part of another term or used independently, refers to a monocyclic and polycyclic system having a total of 5 to 20 ring members, which may be optionally independently substituted by one or more substituents described herein, wherein at least one ring in the system is aromatic, and wherein each ring in the system contains 3 to 12 ring members. Examples of "aryl" include but are not limited to phenyl, biphenyl, naphthyl, anthracenyl, etc., which may have one or more substituents. As used herein, groups wherein an aromatic ring is fused with one or more additional rings are also included within the scope of the term "aryl". In the case of a polycyclic system, only one ring needs to be aromatic (e.g., 2,3-dihydroindole), but all rings can be aromatic (e.g., quinoline). The second ring may also be fused or bridged. Examples of polycyclic aryl include, but are not limited to, benzofuranyl, indenyl, phthalimidyl, naphthalimidyl, endyl or tetrahydronaphthyl. An aryl may optionally be substituted at one or more ring positions by one or more substituents as described herein.

The term "heterocyclyl" as used herein refers to a cycloalkyl wherein one or more (e.g., 1, 2, or 3) ring atoms are substituted by a heteroatom including, but not limited to, oxygen, sulfur, nitrogen, phosphorus, etc. Examples of heterocyclyl containing one heteroatom include pyrrolidine, tetrahydropyran, tetrahydrofuran and piperidine, and examples of a heterocyclyl containing two heteroatoms include morpholine and piperazine.

The term "aralkyl" as used herein, whether as part of another term or used independently, refers to aryl-alkyl-. In some embodiments, the alkyl may contain 1 to 6 carbon atoms, 1 to 5 carbon atoms, 1 to 4 carbon atoms, 1 to 3 carbon atoms, or 1 to 2 carbon atoms. In some embodiments, the aryl may contain 6 to 12 carbon atoms, 6 to 11 carbon atoms, 6 to 10 carbon atoms, 6 to 9 carbon atoms, 6 to 8 carbon atoms, or 6 to 7 carbon atoms. Examples of "aralkyl" include, but are not limited to, various phenylalkyl and various naphthylalkyl.

The term "oxo" as used herein refers to =O.

The term "hydroxyl" as used herein refers to -OH.

The term "amine" as used herein refers to -NH₂.

The term "isomer" as used herein, whether as part of another term or used independently, refers to compound molecules having the same molecular formula in which atoms or groups of atoms are connected to each other in the same order but are arranged differently in space, including any and all geometric isomers and stereoisomers. In some embodiments, the isomers are cis-isomers and trans-isomers. In some embodiments, the isomers are E-isomers and Z-isomers. In some embodiments, the isomers are R-enantiomers and S-enantiomers. In some embodiments, the isomers are D-isomers and L-isomers. In some embodiments, the isomers are diastereoisomers.

The term "enantiomer" as used herein refers to two stereoisomers of a compound that are non-superimposable mirror images of each other. The term "diastereoisomer" as used herein refers to a pair of optical isomers that are not mirror images of each other. Diastereoisomers have different physical properties, such as melting point, boiling point, spectral characteristics, and reactivity.

The term "isomer mixture" as used herein refers to a mixture formed by mixing two or more isomers in equal proportions. In some embodiments, the isomer mixture is a 1:1 mixture of E-isomer and Z-isomer. In some embodiments, the isomer mixture is a 1:1 mixture of R-isomer and S-isomer. In some embodiments, the isomer mixture is a 1:1:1:1 mixture of four diastereoisomers.

As used herein, the term "pharmaceutically acceptable salt" refers to a salt or zwitterionic form of a compound described herein that is, within a reasonable medical judgment, suitable for use in contact with human or animal tissues without excessive toxicity, irritation, allergic reaction, or other problems, and that is commensurate with a reasonable benefit/risk ratio.

As used herein, the term "pharmaceutical composition" refers to a composition comprising the lipid composition and nucleic acid as described in the present application, which may optionally further comprise a pharmaceutically acceptable carrier.

As used herein, the term "pharmaceutically acceptable" refers to a compound, material, composition and/or formulation that is, within a reasonable medical judgment, is suitable for use in contact with human and animal tissues without excessive toxicity, irritation, allergic reaction or other problems or complications, and that is commensurate with a reasonable benifit/risk ratio. In some embodiments, a pharmaceutically acceptable compound, material, composition and/or formulation refers to those approved by a regulatory administration (e.g., the U.S. Food and Drug Administration, the Chinese Food and Drug Administration, or the European Medicines Agency) or generally listed in a publically recognized pharmacopoeia (e.g., the U.S. Pharmacopoeia, the Chinese Pharmacopoeia, or the European Pharmacopoeia).

As used herein, the term "pharmaceutically acceptable carrier" means a pharmaceutically acceptable material, composition or vehicle, such as a liquid or solid filler, diluent, excipient, solvent or encapsulation material, which is involved in carrying or delivering the compound provided herein from one location, body fluid, tissue, organ (internal or external) or part of the body to another location, body fluid, tissue, organ or part of the body. Pharmaceutically acceptable carriers may be vehicle, diluent, excipient, or other materials that can be used to contact animal tissues without excessive toxicity or side effects. Examples of pharmaceutically acceptable carriers include sugar, starch, cellulose, malt, tragacanth, gelatin, Ringer's solution, alginic acid, isotonic saline, buffer, etc. Pharmaceutically acceptable carriers available for use in the present application include those commonly known in the art, such as those disclosed in "Remington Pharmaceutical Sciences", Mack Pub. Co., New Jersey (1991), that is incorporated by reference herein.

The term "deliver", "delivery", or "delivering" as used herein encompasses both topical and systematic delivery. "Topical delivery" refers to the direct delivery of a therapeutic agent to be delivered (such as nucleic acid) to a target site in the organism. For example, the agent can be locally delivered by direct injection into a target site (such as a disease site, such as a tumor or inflammation site) or a target organ (such as the heart, spleen, lungs, kidneys, etc.). "Systematic delivery" is the delivery causing a therapeutic agent (such as nucleic acid) extensively biologically distributed within an organism, thereby exposing an effective amount of the therapeutic agent to most parts of the body. In order to achieve the extensive biological distribution, a lifetime in blood is usually required such that the therapeutic agents are not degraded or cleared rapidly before reaching a target site far from the administration site. Systematic delivery of a lipid composition may be performed in any suitable way, including, for example, oral administration, inhalation administration, intralimentary administration, intravenous administration, subcutaneous administration, and intraperitoneal administration.

As used herein, the term "lipid" refers to a class of organic compounds that include, but are not limited to, esters of fatty acids and are characterized as insoluble in water (e.g. solubility in water of less than about 0.01 % by weight) but soluble in many organic solvents. A lipid can be, for example, simple lipid (such as fat, oil, wax), compound lipid (such as phospholipid, glycolipid), and derived lipid (such as steroid).

The term "nucleic acid" as used herein refers to polymers containing at least two deoxyribonucleotides or ribonucleotides, which may be in single-stranded or double-stranded form. Nucleic acids may comprise natural ribonucleotides and deoxyribonucleotides, as well as non-natural ribonucleotides and deoxyribonucleotides. Natural ribonucleotides include, for example, adenosine, guanosine, cytidine, uridine, pseudouridine, inosine, and xanthosine. Natural deoxyribonucleotides include, for example, deoxyadenosine, deoxyguanosine, deoxycytidine, and deoxythymidine. Non-natural ribonucleotides and deoxyribonucleotides typically have modified nucleic bases, modified ribose or deoxyribose, and/or modified phosphate ester linkages.

"Nucleotide" comprises sugar (e.g., deoxyribose or ribose), a base, and a phosphate group. Nucleotides are linked together by phosphate groups to form polymers. "Base" includes purines and pyrimidines, and further includes natural compounds such as adenine, thymine, guanine, cytosine, uracil, inosine, and natural analogs, as well as synthetic derivatives of purines and pyrimidines with modifications such as amine, alcohol, thiol, carboxyl, and alkyl halide. Nucleic acids in the present application may also include nucleotide analogs or modified nucleotides, which can be synthetic, naturally occurring, or non-naturally occurring, with binding characteristics similar to natural nucleic acids. Examples of nucleotide analogs or modified nucleotides include, but are not limited to, nucleotides with thio-phosphate esters, amino-phosphate esters, methyl-phosphate esters, or chiral-methyl-phosphate esters, 2'-O-methyl ribonucleotides, and peptide-nucleic acids (PNAs).

Examples of nucleic acids include DNA or RNA. DNA includes coding DNA and non-coding DNA, examples of which include antisense DNA, plasmid DNA, preconcentrated DNA, PCR products, DNA vectors (P 1, PAC, BAC, YAC, artificial chromosomes), expression cassettes, hybrid sequences, chromosomal DNA, or derivatives and combinations thereof. Examples of RNA include antisense RNA, siRNA, asymmetric interfering RNA (aiRNA), microRNA (miRNA), mRNA, tRNA, rRNA, viral RNA (vRNA), long non-coding RNA (lncRNA), Piwi-interacting RNA (piRNA), small nucleolar RNA (snoRNA), tRNA-derived small RNA (tsRNA), or their combinations.

Unless otherwise indicated, specific nucleic acid sequences inherently include their conservatively modified variants (e.g., degenerate codon substitutions), alleles, orthologs, SNPs, complementary sequences, and explicitly indicated sequences. Specifically, degenerate codon substitutions can be achieved by generating sequences in which one or more selected (or all) codons at the third position are replaced with mixed basic and/or deoxyadenosine residues (Batzer et al., Nucleic Acid Res., 19:5081(1991); Ohtsuka et al., J. Biol. Chem., 260:2605-2608(1985); Rossolini et al., Mol. Cell. Probes, 8:91-98(1994)).

The term "complementary" in the context of nucleotide pairing used herein includes classical Watson-Crick pairing, i.e., G-C, A-T, or A-U pairing. Classical Watson-Crick pairing also includes situations in which one or two nucleotides have been modified (e.g., by ribose modification or phosphate ester backbone modification). The term "complementary" sequences as used herein may also include non-Watson-Crick base pairs and/or base pairs formed by non-natural and modified nucleotides.

As used herein, the term "treat", "treating", or "treatment" refers to eliminating, reducing or improving a disease or condition, and/or related symptoms. Although not excluded, the treatment of a disease or condition does not require the complete elimination of the disease or condition, and/or related symptoms. The term "treat", "treating", or "treatment" as used herein may include "prophylaxis", which refers to reducing the possibility of recurrence of a disease or condition, or reducing the possibility of recurrence of a previously controlled disease or condition, in a subject who is not subject to a disease or condition but is at risk or prone to the disease or condition or whose disease or condition is at risk or prone to recurrence. Within the meaning of the present invention, "treat", "treating", or "treatment" also includes relapse prevention or stage prevention, as well as treatment of acute or chronic signs, symptoms and/or dysfunction. Treatment can be symptom-specific, for example, to suppress symptoms. It can work for a short period of time, for a medium period, or it can be a long-term treatment, such as in the case of maintenance therapy.

As used herein, the term "subject" means any organism to which the lipid composition described herein may be administered for therapeutic purposes. In some embodiments, subject refers to primates (e.g., human), dogs, rabbits, guinea pigs, pigs, rats, and mice. In some embodiments, the subject is primates. In other embodiments, the subject is human.

### Method and Use of Lipid Compounds for Delivering Nucleic Acid

The present application provides method and use of lipid compounds for delivering nucleic acid. The present invention is at least partly based on the discovery of the remarkable effects of certain lipid compounds in nucleic acid delivery. The present application provides various lipid compounds, which have been found to form stable nucleic acid lipid mixture and effectively deliver nucleic acids into cells, particularly the in vivo cells. The lipid compounds provided herein can deliver nucleic acid molecules into the body and specific target organs within the body via oral administration, thereby achieving desirable therapeutic effects. Surprisingly, different lipid compounds exhibit varying effects in delivering nucleic acids to target organs, demonstrating different preferences for different target organs. Some lipid compounds show significant nucleic acid delivery effects for more than one target organ, and efficiently deliver nucleic acids to multiple target organs, thus having broad use in nucleic acid delivery. Certain lipid compounds exhibit particularly notable delivery effects for a specific target organ, making them suitable for delivering nucleic acids that act on such specific organ.

In one aspect, the present application provides use of a lipid composition in the manufacture of an agent for delivering nucleic acid, or a method of delivering nucleic acid, wherein the lipid composition comprises one or more compounds of formula (I),

A-L₁-L₃-L₂-B (I)

or salts, hydrates, or solvates thereof:
wherein:
L₁ is absent, straight or branched C₁₋₂₄ alkyl, straight or branched C₂₋₂₄ alkenyl, aryl optionally substituted by alkoxy or hydroxy, or straight or branched C₁₋₂₄ alkyl optionally substituted by aralkyl;
L₂ is absent, straight or branched C₁₋₂₄ alkyl optionally substituted by amine, or straight or branched C₂₋₂₄ alkenyl;
L₃ is 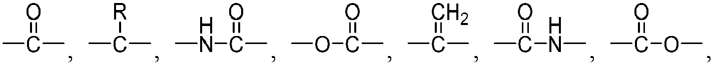 cycloalkyl optionally substituted by oxo, or heterocyclyl optionally substituted by oxo;
A is hydrogen, hydroxy, -COOH, -N(R')₂, straight or branched C₁₋₂₄ alkyl optionally substituted by hydroxy, straight or branched C₂₋₂₄ alkenyl, straight or branched C₂₋₂₄ heteroalkenyl optionally substituted by one or more substituents selected from hydroxy or amine, aryl optionally substituted by alkoxy, aminosulfonyl, or hydroxy, or heterocyclyl optionally substituted by oxo;
B is hydroxy, -CHO, -COOH, -C(O)R', -OC(O)R', straight or branched C₁₋₂₄ alkyl, straight or branched C₂₋₂₄ alkenyl;
R is hydrogen, straight or branched C₁₋₂₄ alkyl;
each R' is independently straight or branched C₁₋₂₄ alkyl.

In some embodiments, L₁ is absent. In some embodiments, L₁ is straight or branched C₁₋₂₄ alkyl. In some embodiments, L₁ is straight or branched C₂₋₂₄ alkenyl. In some embodiments, L₁ is aryl optionally substituted by alkoxy or hydroxy. In some embodiments, L₁ is straight or branched C₁₋₂₄ alkyl optionally substituted by aralkyl.

In some embodiments, L₂ is absent. In some embodiments, L₂ is straight or branched C₁₋₂₄ alkyl optionally substituted by amine. In some embodiments, L₂ is straight or branched C₂₋₂₄ alkenyl.

In some embodiments, L₃ is 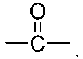 In some embodiments, L₃ is 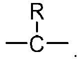 In some embodiments, L₃ is 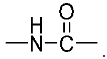 In some embodiments, L₃ is 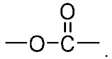 In some embodiments, L₃ is 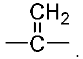 In some embodiments, L₃ is 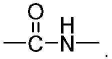 In some embodiments, L₃ is 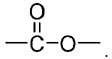 In some embodiments, L₃ is cycloalkyl optionally substituted by oxo. In some embodiments, L₃ is heterocyclyl optionally substituted by oxo.

In some embodiments, A is hydrogen. In some embodiments, A is hydroxy. In some embodiments, A is -COOH. In some embodiments, A is -N(R')₂. In some embodiments, A is straight or branched C₁₋₂₄ alkyl optionally substituted by hydroxy. In some embodiments, A is straight or branched C₂₋₂₄ alkenyl. In some embodiments, A is straight or branched C₂₋₂₄ heteroalkenyl optionally substituted by one or more substituents selected from hydroxy or amine. In some embodiments, A is heterocyclyl optionally substituted by oxo. In some embodiments, A is aryl optionally substituted by alkoxy, aminosulfonyl, or hydroxy.

In some embodiments, B is hydroxy. In some embodiments, B is -CHO. In some embodiments, B is -COOH. In some embodiments, B is -C(O)R'. In some embodiments, B is -OC(O)R'. In some embodiments, B is straight or branched C₁₋₂₄ alkyl. In some embodiments, B is straight or branched C₂₋₂₄ alkenyl.

In some embodiments, R is hydrogen. In some embodiments, R is straight or branched C₁₋₂₄ alkyl. In some embodiments, each R' is independently straight or branched C₁₋₂₄ alkyl.

In some embodiments, the lipid composition comprises one or more compounds of formula (I) selected from the group consisting of lipids 501, 502, 503, 505, 519, 520, 521, 522, 525, 526, 527, 528, 529, 530, 537, 542, 543, 572, 573, 574, 578, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830, or salts, hydrates, or solvates thereof, wherein the information of the lipid such as chemical name and chemical structure is presented in Table 1.

In another aspect, the present application also provides use of a lipid composition in the manufacture of an agent for delivering nucleic acid, or a method of delivering nucleic acid, wherein the lipid composition comprises one or more compounds selected from the group consisting of lipids 501, 502, 503, 505, 519, 520, 521, 522, 525, 526, 527, 528, 529, 530, 537, 542, 543, 572, 573, 574, 578, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830, or salts, hydrates, or solvates thereof, wherein the information of the lipid such as chemical name and chemical structure is presented in Table 1.

**Table 1. Cas number, structure, and product name of Lipids 501-636**

| **lipid compound number** | **Cas number** | **structure and product name** |
|---|---|---|
| 501 | 39924-52-2 | 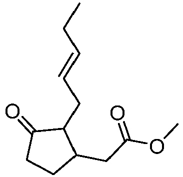 |
| | | methyl jasmonate (methyl 2-[3-oxo-2-[(E)-pent-2-enyl]cyclopentyl]acetate) |
| 502 | 689-67-8 | 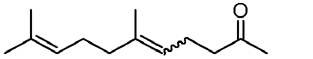 |
| | | 6,10-dimethylundeca-5,9-dien-2-one (isomer mixture) |
| 503 | 1462-03-9 | 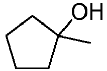 |
| | | 1-methylcyclopentan-1-ol |
| 505 | 1653-31-2 | 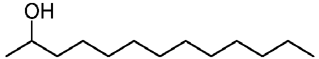 |
| | | tridecan-2-ol |
| 519 | 171022-15-4 | 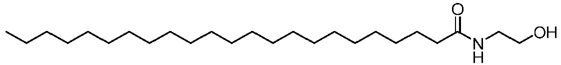 |
| | | N-(2-hydroxyethyl)tricosanamide |
| 520 | 28874-58-0 | 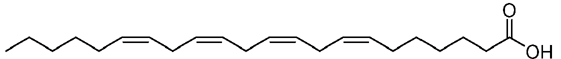 |
| | | (7Z,10Z,13Z,16Z)-docosa-7,10,13,16-tetraenoic acid |
| 521 | 10015-68-6 | 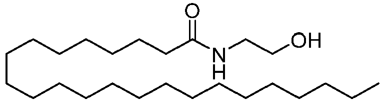 |
| | | N-(2-hydroxyethyl)tetracosanamide |
| 522 | 2014-58-6 | 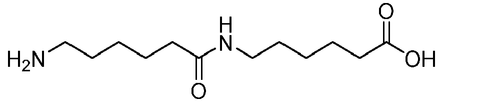 |
| | | 6-(6-aminohexanoylamino)hexanoic acid |
| 525 | 103560-62-9 | 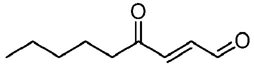 |
| | | (E)-4-oxonon-2-enal |
| 526 | 928-97-2 | 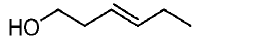 |
| | | (E)-hex-3-en-1-ol |
| 527 | 221682-41-3 | 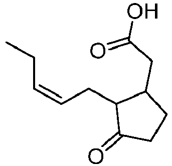 |
| | | jasmonic acid (isomer mixture) (2-[3-oxo-2-[(Z)-pent-2-enyl]cyclopentyl]acetic acid) |
| 528 | 871-83-0 | 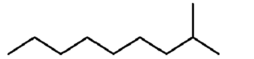 |
| | | 2-methylnonane |
| 529 | 505-54-4 | 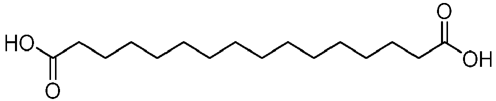 |
| | | hexadecanedioic acid |
| 530 | 542-32-5 | 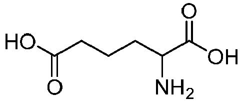 |
| | | 2-aminohexanedioic acid |
| 537 | 404-86-4 | 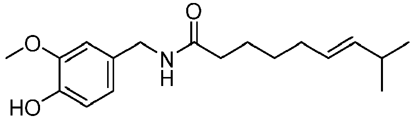 |
| | | capsaicin ((E)-N-[(4-hydroxy-3-methoxyphenyl)methyl]-8-methylnon-6-enamide) |
| 542 | 25182-74-5 | 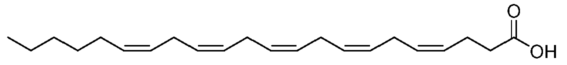 |
| | | (4Z,7Z,10Z,13Z,16Z)-docosa-4,7,10,13,16-pentaenoic acid |
| 543 | 140631-27-2 | 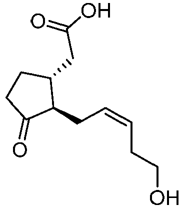 |
| | | 12-Hydroxyjasmonic acid (2-[(1R,2R)-2-[(Z)-5-hydroxypent-2-enyl]-3-oxocyclopentyl]acetic acid) |
| 572 | 1460-18-0 | 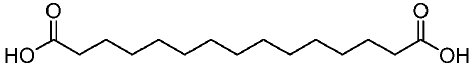 |
| | | pentadecanedioic acid |
| 573 | 2319-29-1 | 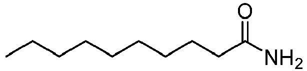 |
| | | Decanamide |
| 574 | 2765-11-9 | 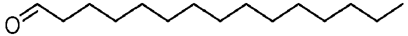 |
| | | pentadecanal |
| 578 | 544-31-0 | 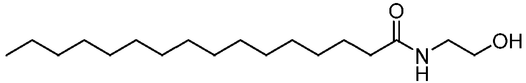 |
| | | N-(2-hydroxyethyl)hexadecanamide |
| 593 | 2345-28-0 | 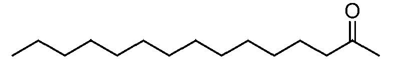 |
| | | pentadecan-2-one |
| 594 | 124-25-4 | 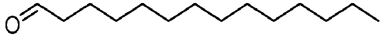 |
| | | tetradecanal |
| 595 | 1118-90-7 | 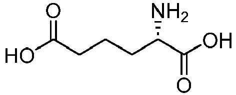 |
| | | (2S)-2-aminohexanedioic acid |
| 596 | 516-05-2 | 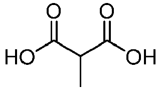 |
| | | 2-methylpropanedioic acid |
| 597 | 26098-55-5 | 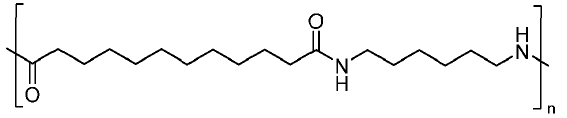 |
| | | nylon 6/12 (dodecanedioic acid-1,6-hexanediamine polymer) |
| 598 | 4839-46-7 | 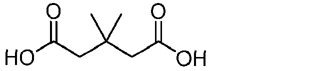 |
| | | 3,3-dimethylpentanedioic acid |
| 599 | 629-54-9 | 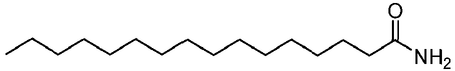 |
| | | hexadecanamide |
| 600 | 506-42-3 | 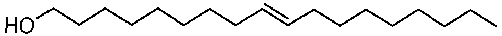 |
| | | (E)-octadec-9-en-1-ol |
| 601 | 124-26-5 | 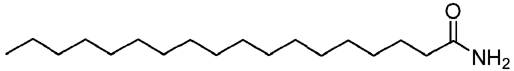 |
| | | octadecanamide |
| 602 | 629-80-1 | 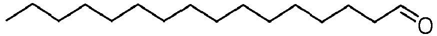 |
| | | hexadecanal |
| 603 | 674-26-0 | 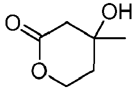 |
| | | 4-hydroxy-4-methyloxan-2-one |
| 604 | 693-54-9 | 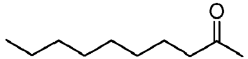 |
| | | decan-2-one |
| 605 | 1002-84-2 | 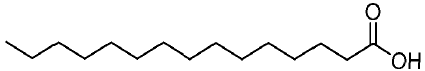 |
| | | pentadecanoic acid |
| 606 | 498-24-8 | 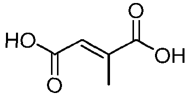 |
| | | (E)-2-methylbut-2-enedioic acid |
| 607 | 4436-74-2 | 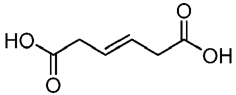 |
| | | (E)-hex-3-enedioic acid |
| 608 | 498-21-5 | 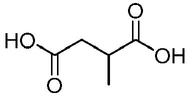 |
| | | 2-methylbutanedioic acid |
| 609 | 629-76-5 | 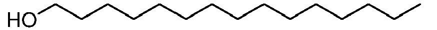 |
| | | pentadecan-1-ol |
| 610 | 626-97-1 | 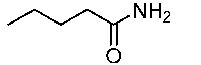 |
| | | pentanamide |
| 611 | 1120-16-7 | 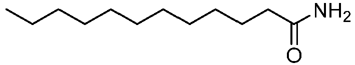 |
| | | dodecanamide |
| 612 | 517-23-7 | 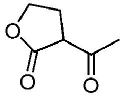 |
| | | 3-acetyloxolan-2-one |
| 613 | 626-82-4 | 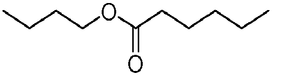 |
| | | butyl hexanoate |
| 614 | 2639-63-6 | 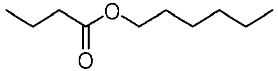 |
| | | hexyl butanoate |
| 615 | 112-31-2 | 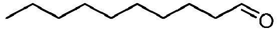 |
| | | decanal |
| 616 | 3572-64-3 | 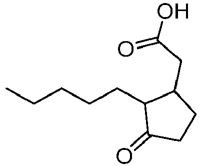 |
| | | 2-(3-oxo-2-pentylcyclopentyl)acetic acid |
| 617 | 143-13-5 | 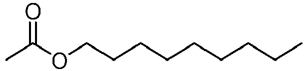 |
| | | nonyl acetate |
| 618 | 4313-03-5 | 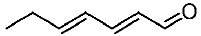 |
| | | (2E,4E)-hepta-2,4-dienal |
| 619 | 4312-99-6 | 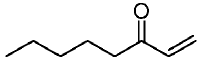 |
| | | oct-1-en-3-one |
| 620 | 506-32-1 | 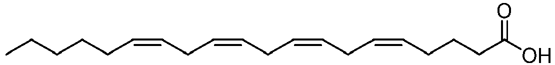 |
| | | (5Z,8Z,11Z,14Z)-icosa-5,8,11,14-tetraenoic acid |
| 621 | 625-33-2 | 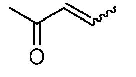 |
| | | pent-3-en-2-one (isomer mixture) |
| 622 | 143537-62-6 | 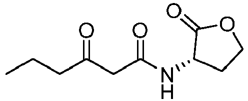 |
| | | 3-oxo-N-[(3S)-2-oxooxolan-3-yl]hexanamide |
| 623 | 328-42-7 | 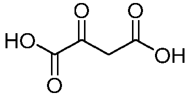 |
| | | 2-oxobutanedioic acid |
| 624 | 506-30-9 | 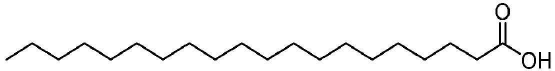 |
| | | icosanoic acid |
| 625 | 7620-28-2 | 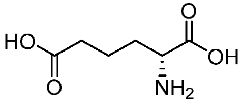 |
| | | (2R)-2-aminohexanedioic acid |
| 626 | 97-65-4 | 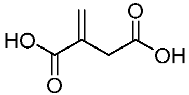 |
| | | 2-methylidenebutanedioic acid |
| 627 | 141-82-2 | 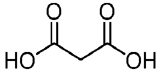 |
| | | propanedioic acid |
| 628 | 110-15-6 | 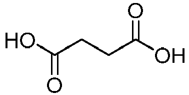 |
| | | butanedioic acid |
| 635 | 112-72-1 | 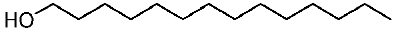 |
| | | tetradecan-1-ol |
| 636 | 112-92-5 | 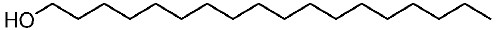 |
| | | octadecan-1-ol |
| 802 | 827029-48-1 | 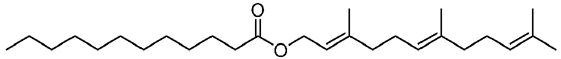 |
| | | [(2E,6E)-3,7,11-trimethyldodeca-2,6,10-trienyl] dodecanoate |
| 803 | 78368-57-7 | 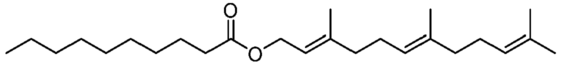 |
| | | [(2E,6E)-3,7,11-trimethyldodeca-2,6,10-trienyl] decanoate |
| 804 | N/A | 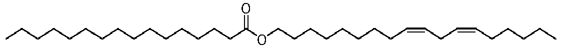 |
| | | [(9Z,12Z)-octadeca-9,12-dienyl] hexadecanoate |
| 805 | 1286724-00-2 | 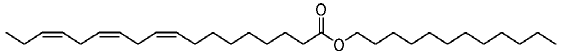 |
| | | dodecyl (9Z, 12Z, 15Z)-octadeca-9, 12, 15-trienoate |
| 806 | 17673-63-1 | 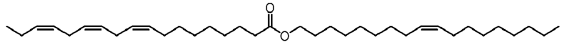 |
| | | [(Z)-octadec-9-enyl] (9Z,12Z,15Z)-octadeca-9,12,15-tri enoate |
| 807 | 158840-60-9 | 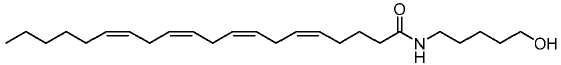 |
| | | (5Z,8Z,11Z,14Z)-N-(5-hydroxypentyl)icosa-5,8,11,14-tetraenamide |
| 808 | 150314-38-8 | 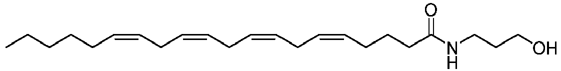 |
| | | (5Z,8Z,11Z,14Z)-N-(3-hydroxypropyl)icosa-5,8,11,14-tetraenamide |
| 809 | 514829-93-7 | 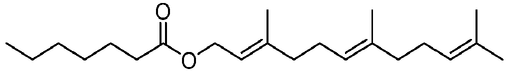 |
| | | [(2E,6E)-3,7,11-trimethyldodeca-2,6,10-trienyl] heptanoate |
| 810 | 187223-87-6 | 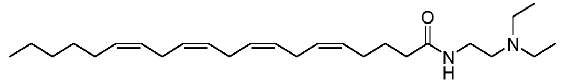 |
| | | (5Z,8Z,11Z,14Z)-N-[2-(diethylamino)ethyl]icosa-5,8,11,14-tetraenamide |
| 811 | 130416-65-8 | 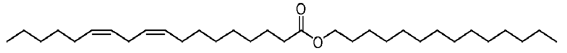 |
| | | tetradecyl (9Z,12Z)-octadeca-9,12-dienoate |
| 812 | N/A | 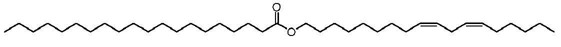 |
| | | [(9Z,12Z)-octadeca-9,12-dienyl] icosanoate |
| 813 | N/A | 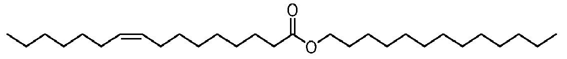 |
| | | tridecyl (Z)-hexadec-9-enoate |
| 814 | 78405-75-1 | 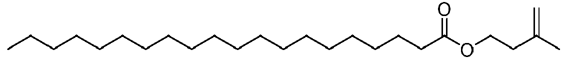 |
| | | 3-methylbut-3-enyl icosanoate |
| 815 | 78405-77-3 | 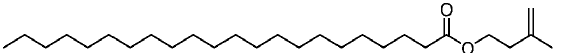 |
| | | 3-methylbut-3-enyl docosanoate |
| 816 | N/A | 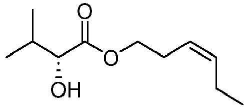 |
| | | [(Z)-hex-3-enyl] (2R)-2-hydroxy-3-methylbutanoate |
| 817 | 2692623-62-2 | 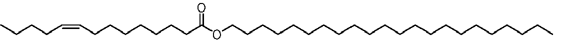 |
| | | docosyl (Z)-tetradec-9-enoate |
| 818 | N/A | 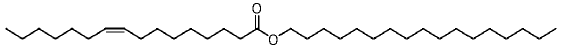 |
| | | heptadecyl (Z)-hexadec-9-enoate |
| 819 | N/A | 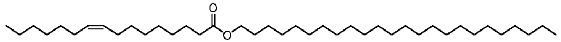 |
| | | tetracosyl (Z)-hexadec-9-enoate |
| 820 | N/A | 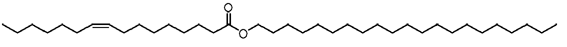 |
| | | henicosyl (Z)-hexadec-9-enoate |
| 821 | N/A | 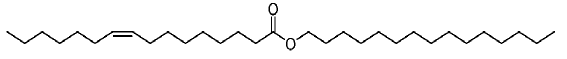 |
| | | pentadecyl (Z)-hexadec-9-enoate |
| 822 | N/A | 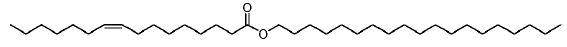 |
| | | nonadecyl (Z)-hexadec-9-enoate |
| 823 | 1609384-99-7 | 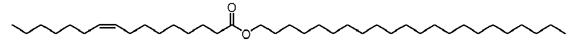 |
| | | docosyl (Z)-hexadec-9-enoate |
| 824 | 210757-15-6 | 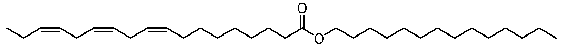 |
| | | tetradecyl (9Z,12Z,15Z)-octadeca-9,12,15-trienoate |
| 825 | N/A | 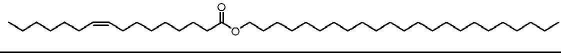 |
| | | tricosyl (Z)-hexadec-9-enoate |
| 826 | 166100-32-9 | 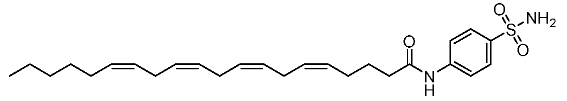 |
| | | (5Z,8Z,11Z,14Z)-N-(4-sulfamoylphenyl)icosa-5,8,11,14-tetraenamide |
| 827 | 326493-93-0 | 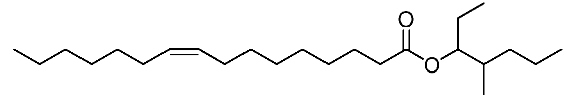 |
| | | 4-methylheptan-3-yl (Z)-hexadec-9-enoate |
| 828 | 326493-96-3 | 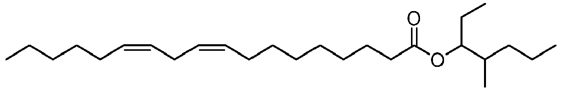 |
| | | 4-methylheptan-3-yl (9Z,12Z)-octadeca-9,12-dienoate |
| 829 | N/A | 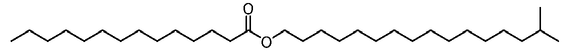 |
| | | 15-methylhexadecyl tetradecanoate |
| 830 | 37571-95-2 | 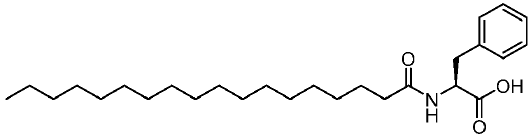 |
| | | (2S)-2-(octadecanoylamino)-3-phenylpropanoic acid |

In some embodiments, the compound is synthetic. The above compounds can be synthesized by means of chemical synthesis according to the chemical structure of the compounds. Alternatively, some of the compounds in the present application are commercially available.

In some embodiments, the compound derives from extracts of traditional Chinese medicine. "Extracts of traditional Chinese medicine" as used herein refers to extracts obtained from traditional Chinese medicinal materials or medicinal plants by suitable methods. The extracts of traditional Chinese medicine may be obtained using any suitable extraction method. As an example, the herbal decoction pieces (Yin Pian) of traditional Chinese medicine can be soaked in water, followed by strong fire decoction and weak fire decoction in sequence. The decocted Chinese medicine liquid is concentrated, and then added chloroform-methanol, chloroform and water in sequence and mixed thoroughly, and the chloroform layer is taken to obtain the extracts of traditional Chinese medicine. In some embodiments, the compound monomer may be further isolated or purified from the extracts of traditional Chinese medicine.

In some embodiments, the extracts of traditional Chinese medicine are obtained by Bligh&Dyer process (Bligh E.G. and Dyer, W. J., A rapid method for total lipid extraction and purification, Can. J. Biochem. Physiol., 1959, 37: 911-917) to extract liposoluble components, or obtained by decoction and extraction of traditional Chinese medicine.

In some embodiments, the extracts of traditional Chinese medicine are obtained by decoction and extraction of traditional Chinese medicine.

In some embodiments, the extracts of traditional Chinese medicine are obtained by soaking the traditional Chinese medicine in water, followed by strong fire decoction and weak fire decoction in sequence. The decocted Chinese medicine liquid is concentrated, and then added chloroform-methanol, chloroform and water in sequence and mixed, and the chloroform layer is taken and extracted.

### Lipid composition

In some embodiments, the lipid composition provided herein comprises one or more compounds of the present application or salts, hydrates, or solvates thereof. The lipid composition may be one of the compounds provided herein or salts, hydrates, or solvates thereof, or may be a mixture of two or more of the compounds provided herein.

In some embodiments, the lipid composition may also comprise one or more lipid compounds other than the compounds provided herein. These lipid compounds may be, for example, neutral lipids, charged lipids, steroid compounds, and polymer-conjugated lipids. "Neutral lipid" refers to a lipid compound that exists in an uncharged or a neutral zwitterionic form at a selected pH value (e.g. physiological pH). "Charged lipid" refers to a lipid compound that exists in a positively or negatively charged form that is not limited by pH values in a useful physiological range (e.g., pH of about 3 to about 9).

In some embodiments, the lipid composition may further comprise one or more solvents capable of mixing with the compound provided herein or salts, hydrates, or solvates thereof to form a homogeneous mixture.

The solvent comprised in the lipid composition may comprise organic solvent or solvent mixture, such as chloroform, dichloromethane, diethyl ether, cyclohexane, cyclopentane, benzene, toluene, methanol or other aliphatic alcohols, such as ethanol, propanol, isopropanol, butanol, tert-butanol, isobutanol, pentanol, and hexanol. These solvents can be used alone, in combination and/or optionally together with suitable buffers as solvents in the lipid composition. The solvent can be selected depending on the polarity of the solvent, the ease for removing the solvent at a later stage in the formation of the lipid nucleic acid mixture, and/or pharmaceutically acceptable properties. In some embodiments, the solvent is non-toxic, or pharmaceutically acceptable. Exemplary pharmaceutically acceptable solvents include lower alcohols (1-6 carbon atoms), such as methanol, ethanol, n-butanol, isobutanol, and n-butanol. In some embodiments, an appropriate amount of solvent may be used such that the nucleic acid and lipid form a clear single-phase mixture.

The lipid compositions provided herein can be used for delivering nucleic acids or preparing agents for delivering nucleic acid. In certain embodiments, the nucleic acids include DNA or RNA. In some embodiments, the nucleic acids may include coding DNA, non-coding DNA, antisense nucleic acids, messenger RNA (mRNA), long non-coding RNA (lncRNA), and small RNA (such as microRNA (miRNA), small interfering RNA (siRNA), Piwi-interacting RNA (piRNA), small nucleolar RNA (snoRNA), tRNA-derived small RNA (tsRNA), and the like.

In some embodiments, the nucleic acids are single-stranded or double-stranded. Examples of single-stranded nucleic acids include miRNA, mRNA, antisense DNA, antisense RNA, etc. Examples of double-stranded nucleic acids include siRNA, double-stranded DNA, double-stranded RNA, etc.

In some embodiments, the nucleic acids have a stem-loop structure. The term "stem-loop structure" refers to the presence of reverse complementary sequences in two parts of a single-stranded nucleic acid. Upon base pairing, these reverse complementary sequences form a double-stranded structure, and the non-complementary portion between the two reverse complementary regions protrudes to form a loop structure. The stem-loop structure is also known as a hairpin structure.

In some embodiments, the nucleic acids include small nucleic acids. Small nucleic acids refer to nucleic acids with shorter lengths (e.g., less than 200 nucleotides). Small nucleic acids can be non-coding, such as small RNA (e.g., miRNA, siRNA, piRNA, snoRNA, tsRNA), and can be either single-stranded or double-stranded. In some embodiments, the nucleic acids are small nucleic acids having a length of 14-32bp, 16-28bp, or 18-24bp.

In some embodiments, the nucleic acids are nucleic acid drugs. Nucleic acid drugs can belong to various categories, such as antisense nucleic acids, siRNA, CpG oligodeoxynucleotides, nucleic acid aptamers, mRNA or DNA encoding target proteins, miRNA, etc. Exemplary nucleic acid drugs include AEG35156, aganirsen, AP 12009, Apatorsen, ATL1103, AVT-02 UE, Bevasiranib Sodium, BMN 044, BMN 053, CpG 7909, Custirsen, Drisapersen, Eteplirsen, Fomivirsen, Pegaptanib, Mipomersen, Defibrotide, Nusinersen, Patisiran, Tegsedi, and Fovista.

In some embodiments, the nucleic acid is used for treating diseases.

In some embodiments, the nucleic acid is used for treating cancer, inflammation, fibrotic disease, autoimmune disease or autoinflammatory disease, bacterial infection, behavioral and psychiatric disorder, blood disease, chromosomal disease, congenital and hereditary disease, connective tissue disease, digestive disease, ear nose throat disease, endocrine disease, environmental disease, eye disease, female reproductive disease, fungal infection, heart disease, hereditary cancer syndrome, immune system disorder, kidney and urinary disease, lung disease, male reproductive disease, metabolic disorder, mouth disease, musculoskeletal disease, myelodysplastic syndrome, neonatal screening, nutritional disease, parasitic disease, rare cancer, rare disease, skin disease, or viral infection.

In some embodiments, the nucleic acid is used for treating hepatocellular carcinoma, corneal neovascularization, recurrent or refractory anaplastic astrocytoma (WHO Grade III), or secondary glioblastoma (WHO Grade IV), advanced squamous cell lung cancer, acromegaly, psoriasis, Duchenne muscular dystrophy, advanced non-small cell lung cancer, metastatic castration-resistant prostate cancer, cytomegalovirus retinitis, HIV infection, hepatitis B, hepatitis C, hyperlipoprotein disease, total knee replacement, type 2 diabetes, familial amyloid polyneuropathy (FAP), wet macular degeneration (e.g., neovascular age-related macular degeneration, subfoveal neovascular age-related macular degeneration, exudative age-related macular degeneration), hypercholesterolemia, Crohn's disease, extensive liver fibrosis, infantile spinal muscular atrophy, melanoma, neonatal coronary artery disease, mild allergic asthma, chronic lymphocytic leukemia, and hypertriglyceridemia, small cell obliterans of the liver with renal or lung dysfunction after hematopoietic stem cell transplantation, or hereditary transthyretin amyloidosis.

In some embodiments, the lipid composition can form a lipid nucleic acid mixture with nucleic acid. The nucleic acid can be delivered by the lipid nucleic acid mixture. "A lipid nucleic acid mixture" in the present application refers to a lipid-based mixture for delivering nucleic acid, such as liposomes. Examples of liposomes include, for example, lipid particles or lipid vesicles that encapsulate nucleic acids. The lipid nucleic acid mixture may be prepared by suitable methods, including, for exampoe, mixing, heating, reverse-phase evaporation.

The mixing method includes the steps of mixing the compound provided herein or salts, hydrates, or solvates thereof with the nucleic acid to be delivered. In some embodiments, the mixing may be a direct mixing of the lipid composition provided herein with the nucleic acid to be delivered (for example, a mixture of dry powder of the lipid composition and the nucleic acid, followed by adding an appropriate solvent to form a lipid nucleic acid mixture with lipid encapsulating the nucleic acid). In some embodiments, the mixing may conducted by mixing the lipid composition provided herein that is dissolved in an appropriate solvent with the nucleic acid, or mixing the nucleic acid dissolved in an appropriate solvent with the lipid composition provided herein, or mixing the lipid composition provided herein dissolved in an appropriate solvent with the nucleic acid dissolved in an appropriate solvent. Examples of suitable solvents that may be used for the lipid composition of the present application are described previously in the specification of the present application. Suitable solvents that may be used for the nucleic acid include water (DEPC treated water, double steamed water), buffers, saline, or glucose solutions, etc. The mixing step may be conducted with any suitable step, for example, nucleic acid (or its solution) may be added to a lipid composition (or its solution), or a lipid composition (or its solution) may be added to nucleic acid (or its solution). In some embodiments, the mixing may also include steps such as vortex, ultrasound, etc., to facilitate uniform mixing.

In some embodiments, the mixing method comprises adding an ethanol solution of the lipid composition of the present application to a suitable volume of the nucleic acid to be delivered in aqueous buffer, then mixing uniformly by vortex or ultrasonic, followed by incubating to obtain a lipid nucleic acid mixture. In some embodiments, the mixing method comprises combining an ethanol solution of the lipid compound of the present application with a suitable volume of an ethanol solution of the nucleic acid to be delivered, then mixing uniformly by vortex or ultrasonic, followed by incubating to obtain a lipid nucleic acid mixture, removing the ethanol, and resuspending the resulting lipid nucleic acid mixture in an aqueous buffer.

In some embodiments, the nucleic acid and the lipid compound can be mixed at a certain ratio. The ratio depends on the nucleic acid to be delivered and the amount thereof, as well as the lipid compound to be used to achieve the effect of nucleic acid encapsulation. The ratio can be any ratio that meets the therapeutic needs, as long as a stable lipid nucleic acid mixture is formed and the required amount of nucleic acid is provided. In some embodiments, the ratio of nucleic acid to lipid compound can be, for example, 0.1nmol : 100µg to 10nmol : 100µg, 0.2nmol : 100µg to 10nmol : 100µg, 0.3nmol : 100µg to 10nmol : 100µg, 0.4 nmol : 100µg to 10nmol : 100µg, 0.5 nmol : 100µg to 10nmol : 100µg, 1nmol : 100µg to 10nmol : 100µg, 2nmol : 100µg to 10nmol : 100µg, 3nmol : 100µg to 10nmol : 100µg, 4nmol : 100µg to 10nmol : 100µg, 5nmol : 100µg to 10nmol : 100µg, 6nmol : 100µg to 10nmol, 7nmol : 100µg to 10nmol, 8nmol : 100µg to 10nmol, or 9nmol : 100µg to 10nmol : 100µg. In some embodiments, the ratio of nucleic acid to lipid compound can be 1nmol : 100µg, 2nmol : 100µg, 3nmol : 100µg, 4nmol : 100µg, 5nmol : 100µg, 6nmol : 100µg, 7nmol : 100µg, 8nmol : 100µg, 9nmol : 100µg, or 10nmol : 100µg. In some embodiments, the ratio of nucleic acid to lipid compound can be 10nmol : 100µg.

The mixing method can be conducted at any suitable temperature, as long as a lipid nucleic acid mixture capable of delivering nucleic acid can be formed. In some embodiments, the nucleic acid and the lipid compound can be mixed at a suitable temperature, for example, but not limited, of 0°C to 100°C, 4°C to 100°C, 10°C to 100°C, 15°C to 100°C, 20°C to 100°C, 25°C to 100°C, 30°C to 100°C, 35°C to 100°C, 40°C to 100°C, 45°C to 100°C, 50°C to 100°C, 55°C to 100°C, 60°C to 100°C, 65°C to 100°C, 70°C to 100°C, 75°C to 100°C, 80°C to 100°C, 85°C to 100°C, 90°C to 100°C, or 95°C to 100°C. In some embodiments, the temperature is 0°C, 4°C, 10°C, 20°C, 30°C, 40°C, 50°C, 55°C, 60°C, 65°C, 70°C, 75°C, 80°C, 85°C, or 90°C. In some embodiments, the temperature is 90°C.

In some embodiments, the lipid nucleic acid mixture is prepared by heating method. The heating method comprises mixing a solution of the compound provided herein or salts, hydrates, or solvates thereof dissolved in an appropriate solvent with an aqueous solution of the nucleic acid to be delivered to obtain a mixture solution, and heating the mixture solution at an appropriate temperature. In some embodiments, the step of heating the mixture solution is conducted at a temperature selected from 25°C to 100°C, 30°C to 100°C, 40°C to 100°C, 50°C to 100°C, 60°C to 100°C, 70°C to 100°C, 80°C to 100°C, 90°C to 100°C or 95°C to 100°C. In some embodiments, the step of heating the mixture solution is conducted at a temperature selected from about 30°C, about 35°C, about 37°C, about 40°C, about 45°C, about 50°C, about 55°C, about 60°C, about 65°C, about 70°C, about 75°C, about 80°C, about 85°C, about 90°C, about 95°C, or about 100°C. In some embodiments, the step of heating the mixture solution is conducted at 90°C.

In some embodiments, the heating method comprises heating the mixture solution for an appropriate amount of time. Those skilled of art can select an appropriate heating time according to the properties of the lipid compound and nucleic acid used and the desired lipid nucleic acid mixture. The heating time can be, for example, about 5 minutes to about 24 hours, about 5 minutes to about 20 hours, about 5 minutes to about 16 hours, about 10 minutes to about 20 hours, about 10 minutes to about 16 hours, about 15 minutes to about 24 hours, about 15 minutes to about 20 hours, about 30 minutes to about 24 hours, about 30 minutes to about 20 hours, about 40 minutes to about 16 hours, about 50 minutes to about 12 hours, about 1 hour to about 8 hours, or about 2 hours to about 4 hours. In some embodiments, the heating time can be, for example, about 5 minutes, about 10 minutes, about 15 minutes, about 20 minutes, about 30 minutes, about 40 minutes, about 50 minutes, about 1 hour, about 2 hours, about 3 hours, about 4 hours, about 5 hours, about 6 hours, about 7 hours, about 8 hours, about 9 hours, about 10 hours, about 12 hours, about 16 hours, about 20 hours, or about 24 hours. In some embodiments, the heating time is about 15 minutes.

In some embodiments, the heating method further comprises a cooling step. The cooling can be conducted after the step of heating the mixture solution at an appropriate temperature, as long as the formed lipid nucleic acid mixture is not disrupted. Exemplary cooling temperature includes, not limited to, 25°C to -80°C, 20°C to -80°C, 15°C to -80°C, 10°C to -80°C, 4°C to -80°C, 0°C to -80°C, -10°C to - 80°C, -20°C to -80°C, -30°C to -80°C, or -40°C to -80°C. In some embodiments, the mixture solution is cooled at room temperature (such as 25°C or 20°C) after the step of heating the mixture solution.

In some embodiments, the lipid nucleic acid mixture is prepared by reverse-phase evaporation method. The reverse-phase evaporation method comprises adding an aqueous solution of nucleic acid to a solution of a lipid compound dissolved in an appropriate solvent, followed by removing the solvent via, e.g., ultrasonic or evaproration. In some embodiments, the reverse-phase evaporation method further comprises hydration after removal of the solvent to obtain a lipid nucleic acid mixture. In some embodiments, the hydration includes adding water or a suitable culture medium to the system. In some embodiments, the suitable culture medium is, for example, an OPTI-MEM culture medium.

In some embodiments, the solvent removal step is conducted at an appropriate temperature, such as about 25°C to about 70°C, 30°C to about 70°C, about 30°C to about 65°C, about 40°C to about 65°C, about 40°C to about 60°C, or about 50°C to about 60°C. In some embodiments, the solvent removal step is performed at about 25°C, 30°C, about 35°C, about 37°C, about 40°C, about 45°C, about 50°C, about 55°C, about 60°C, about 65°C, or about 70°C. In some embodiments, the solvent removal step is performed at about 55°C.

In some embodiments, the nucleic acid delivery is a delivery by oral administration, inhalation or injection. In some embodiments, the nucleic acid delivery is delivered by oral means. In some embodiments, the agent is used for delivering nucleic acid by oral administration. In some embodiments, the nucleic acid delivery is in vivo digestive tract delivery.

In some embodiments, the lipid nucleic acid mixture, after being orally administered to an individual, can deliver the nucleic acid to a target organ or target tissue of interest of the individual.

In some embodiments, the lipid nucleic acid mixture, after being orally administered to an individual, can deliver the nucleic acid to the heart tissue of the individual. In some embodiments, the lipid nucleic acid mixture comprises a lipid compound selected from the group consisting of lipids 501, 502, 519, 520, 522, 526, 527, 528, 529, 530, 537, 542, 543, 572, 573, 574, 578, 593, 594, 595, 596, 597, 598, 599, 600, 601, 602, 603, 604, 607, 608, 609, 613, 614, 616, 620, 621, 623, 624, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830.

In some embodiments, the lipid nucleic acid mixture, after being orally administered to an individual, can deliver the nucleic acid to the hepatic tissue of the individual. In some embodiments, the lipid nucleic acid mixture comprises a lipid compound selected from the group consisting of lipids 501, 503, 505, 520, 521, 526, 527, 528, 529, 530, 537, 542, 543, 572, 573, 574, 578, 593, 594, 595, 596, 597, 598, 599, 600, 601, 602, 603, 604, 606, 607, 609, 612, 613, 614, 615, 616, 617, 618, 619, 620, 621, 622, 623, 624, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830.

In some embodiments, the lipid nucleic acid mixture, after being orally administered to an individual, can deliver the nucleic acid to the splenic tissue of the individual. In some embodiments, the lipid nucleic acid mixture comprises a lipid compound selected from the group consisting of lipids 501, 502, 503, 520, 522, 525, 526, 527, 528, 529, 530, 537, 542, 543, 572, 573, 574, 578, 593, 594, 595, 596, 597, 598, 599, 600, 601, 602, 603, 604, 608, 610, 611, 613, 614, 615, 616, 617, 619, 620, 622, 623, 625, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830.

In some embodiments, the lipid nucleic acid mixture, after being orally administered to an individual, can deliver the nucleic acid to the lung tissue of the individual. In some embodiments, the lipid nucleic acid mixture comprises a lipid compound selected from the group consisting of lipids 501, 503, 519, 520, 522, 525, 526, 527, 528, 529, 530, 537, 542, 543, 578, 593, 594, 595, 596, 597, 598, 599, 600, 601, 602, 603, 604, 608, 609, 610, 613, 614, 615, 616, 617, 618, 620, 621, 622, 624, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830.

In some embodiments, the lipid nucleic acid mixture, after being orally administered to an individual, can deliver the nucleic acid to the renal tissue of the individual. In some embodiments, the lipid nucleic acid mixture comprises a lipid compound selected from the group consisting of lipids 501, 502, 503, 519, 521, 525, 526, 527, 528, 529, 530, 537, 542, 543, 572, 574, 578, 593, 594, 595, 596, 597, 598, 599, 600, 601, 602, 603, 604, 607, 609, 610, 613, 617, 619, 620, 623, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830.

In some embodiments, the lipid nucleic acid mixture, after being orally administered to an individual, can deliver the nucleic acid to the gastric tissue of the individual. In some embodiments, the lipid nucleic acid mixture comprises a lipid compound selected from the group consisting of lipids 501, 502, 503, 505, 519, 522, 525, 526, 527, 528, 529, 530, 537, 542, 543, 572, 573, 574, 578, 593, 594, 595, 596, 597, 598, 599, 600, 601, 602, 603, 604, 605, 607, 608, 609, 610, 611, 612, 613, 615, 617, 619, 620, 621, 622, 623, 624, 625, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830.

In some embodiments, the lipid nucleic acid mixture, after being orally administered to an individual, can deliver the nucleic acid to the intestinal tissue of the individual. In some embodiments, the lipid nucleic acid mixture comprises a lipid compound selected from the group consisting of lipids 501, 502, 503, 522, 525, 526, 527, 528, 529, 530, 537, 542, 543, 572, 573, 593, 594, 595, 596, 597, 598, 599, 600, 601, 602, 603, 604, 612, 613, 614, 615, 616, 617, 618, 619, 620, 621, 622, 623, 624, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830.

In some embodiments, the lipid nucleic acid mixture, after being orally administered to an individual, can deliver the nucleic acid to the brain tissue of the individual. In some embodiments, the lipid nucleic acid mixture comprises a lipid compound selected from the group consisting of lipids 501, 502, 503, 505, 520, 526, 527, 528, 529, 530, 537, 542, 543, 572, 573, 574, 578, 593, 594, 595, 596, 597, 598, 599, 600, 601, 602, 603, 604, 609, 613, 614, 615, 616, 617, 618, 619, 620, 621, 622, 623, 624, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830.

In some embodiments, the lipid nucleic acid mixture, after being orally administered to an individual, can deliver the nucleic acid to blood of the individual. In some embodiments, the lipid nucleic acid mixture comprises a lipid compound selected from the group consisting of lipids 501, 502, 503, 505, 521, 522, 526, 527, 528, 529, 530, 537, 542, 543, 572, 573, 574, 578, 593, 594, 595, 596, 597, 598, 599, 600, 601, 602, 603, 604, 605, 606, 607, 613, 614, 615, 616, 617, 619, 621, 622, 623, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830.

In some embodiments, the lipid nucleic acid mixture, after being orally administered to an individual, can deliver the nucleic acid to the thigh bone of the individual. In some embodiments, the lipid nucleic acid mixture comprises a lipid compound selected from the group consisting of lipids 526, 527, 528, 529, 530, 537, 542, 543, 572, 573, 574, 578, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830.

In some embodiments, the lipid nucleic acid mixture, after being orally administered to an individual, can deliver the nucleic acid to the pancreatic tissue of the individual. In some embodiments, the lipid nucleic acid mixture comprises a lipid compound selected from the group consisting of lipids 802, 803, 804, 805, 806, 807, 808, 809, 810, 811, 812, 813, 814, 815, 816, 817, 818, 819, 820, 821, 822, 823, 824, 825, 826, 827, 828, 829, and 830.

In some embodiments, after the lipid nucleic acid mixture is orally administered to an individual, the nucleic acid is delivered to two or more tissues.

In some embodiments, the nucleic acid delivery comprises in vitro cellular delivery. "In vitro" as used herein refers to outside a multicellular organism, such as a human or animal body. In vitro cells include such as in vitro cell cultures, in vitro tissues or cells. In vitro delivery comprises delivering nucleic acid into cells outside the body, for example, comprises contacting the lipid nucleic acid mixture with in vitro cells under the condition that allows the nucleic acid to enter the cell.

### Pharmaceutical composition

In another aspect, the present application also provides a pharmaceutical composition comprising a lipid composition and a nucleic acid, wherein the lipid composition comprises one or more compounds selected from the group consisting of lipids_501, 502, 503, 505, 519, 520, 521, 522, 525, 526, 527, 528, 529, 530, 537, 542, 543, 572, 573, 574, 578, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830, or salts, hydrates, or solvates thereof.

The pharmaceutical compositions provided in the present application may be in solid or liquid form, including semi-solid, semi-liquid, suspension and colloidal forms. Solid form can be, for example, tablet or powder. Liquid form can be, for example, an oral solution, an oral syrup, an injectable liquid, or an aerosol suitable for, for example, inhalation administration.

In some embodiments, the pharmaceutical composition is formulated to be administered orally, by inhalation, via the digestive tract, or via the respiratory tract. In some embodiments, the pharmaceutical composition is formulated to deliver the nucleic acid by in vivo digestive tract delivery. In some embodiments, the pharmaceutical composition is an oral pharmaceutical composition. When used for oral administration, the pharmaceutical composition is preferably in solid or liquid form.

As a solid pharmaceutical composition for oral administration, the pharmaceutical composition can be formulated in the form of powder, pellet, compressed tablet, pill, capsule, chewable gum, etc. Typically, such solid composition may also contain one or more inert diluents or edible carriers (such as starch, lactose, or dextrin) and one or more excipients selected from the group consisting of adhesive (e.g., carboxymethyl cellulose, ethyl cellulose, microcrystalline cellulose, tragacanth or gelatin), dissolver (e.g., alginate, sodium alginate, Primogel, cornstarch, etc.), lubricant (e.g., magnesium stearate or Sterotex), glidant (e.g., colloidal silica), sweetener (e.g., sucrose or saccharin), flavoring agent; and colorant.

As a liquid pharmaceutical composition for oral administration, the pharmaceutical composition may be formulated as, for example, elixir, syrup, solution, emulsion, or suspension. When used for oral administration, the liquid pharmaceutical composition contains one or more excipients selected from the group consisting of sweetener, preservatives, stain/colorant, and flavoring agent.

The pharmaceutical composition of the present disclosure can also be formulated to be administered by inhalation through the respiratory tract. An appropriate inhalation formulation may include formulation that can be administered as aerosol. The aerosol can be delivered in single-phase, biphase or three-phase systems. For example, the aerosol can be delivered by liquefied gas and compressed gas, or by a suitable system wherein the active ingredient is dispersed. The delivery of the aerosol includes the necessary container, activator, valve, sub-container, etc., which together can form a kit.

In some embodiments, the pharmaceutical compositions of the present application may also be formulated to be administered parenterally, such as by injection. The liquid composition for injection may be a solution, suspension or injection powder and may comprise one or more of surfactant, preservatives, wetting agent, dispersant, suspension, buffer, stabilizer and isotonic agent.

In some embodiments, the pharmaceutical composition may also be used for delivery through in vitro cells.

In some embodiments, the lipid composition and the nucleic acid are present at least partially or fully in the form of mixture in the pharmaceutical composition. In some embodiments, the mixture is prepared by heating method, reverse-phase evaporation method, or mixing method.

In another aspect, the present application also provides the use of the pharmaceutical compositions of the present application in the manufacture of a medicament for the prevention and/or treatment of diseases that can be prevented and/or treated with nucleic acid, or for in vivo delivering nucleic acid to a subject in need thereof.

The pharmaceutical composition provided in the present application can be used for the treatment of diseases that can be treated with nucleic acid. Common diseases for example, but not limited to, cancer, inflammation, fibrotic disease, autoimmune disease or autoinflammatory disease, bacterial infection, behavioral and psychiatric disorder, blood disease, chromosomal disease, congenital and hereditary disease, connective tissue disease, digestive disease, ear nose throat disease, endocrine disease, environmental disease, eye disease, female reproductive disease, fungal infection, heart disease, hereditary cancer syndrome, immune system disorder, kidney and urinary disease, lung disease, male reproductive disease, metabolic disorder, mouth disease, musculoskeletal disease, myelodysplastic syndrome, neonatal screening, nutritional disease, parasitic disease, rare cancer, rare disease, skin disease, or viral infection.

Examples of cancers include, but are not limited to, stomach cancer, lung cancer, colorectal cancer, liver cancer, pancreatic cancer, cervical cancer, breast cancer, leukemia, multiple myeloma.

Examples of inflammation include, but are not limited to, pneumonia, myocarditis, acute and chronic gastritis, acute and chronic enteritis, acute and chronic hepatitis, acute and chronic nephritis, dermatitis, encephalitis, lymphadenitis, conjunctivitis, keratitis, iridocycletis, otitis media, allergic rhinitis, asthma, pulmonary fibrosis, chronic obstructive pulmonary disease, atopic dermatitis, sickle cell disease, multiple sclerosis, systemic lupus erythematosus, lupus nephritis.

Known nucleic acid drugs may treat a variety of diseases. Indications for known nucleic acid drugs include, for example, hepatocellular carcinoma, corneal neovascularization, recurrent or refractory anaplastic astrocytoma (WHO Grade III), or secondary glioblastoma (WHO Grade IV), advanced squamous cell lung cancer, acromegaly, psoriasis, Duchenne muscular dystrophy, advanced non-small cell lung cancer, metastatic castration-resistant prostate cancer, cytomegalovirus retinitis, HIV infection, hepatitis B, hepatitis C, hyperlipoprotein disease, total knee replacement, type 2 diabetes, familial amyloid polyneuropathy (FAP), wet macular degeneration (e.g., neovascular age-related macular degeneration, subfoveal neovascular age-related macular degeneration, exudative age-related macular degeneration), hypercholesterolemia, Crohn's disease, extensive liver fibrosis, infantile spinal muscular atrophy, melanoma, neonatal coronary artery disease, mild allergic asthma, chronic lymphocytic leukemia, and hypertriglyceridemia, small cell obliterans of the liver with renal or lung dysfunction after hematopoietic stem cell transplantation, or hereditary transthyretin amyloidosis.

### Kit

In another aspect, the present application also provides a kit comprising one or more compounds or pharmaceutically acceptable salts, hydrates, or solvates thereof, positioned in a first container, and a nucleic acid positioned in a second container, wherein the one or more compounds are selected from the group consisting of lipids 501, 502, 503, 505, 519, 520, 521, 522, 525, 526, 527, 528, 529, 530, 537, 542, 543, 572, 573, 574, 578, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 635, and 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830.

In some embodiments, the lipid composition and the nucleic acid in the kit are formulated at least partially or fully as a lipid nucleic acid mixture before use. In some embodiments, the mixture is prepared by heating method, reverse-phase evaporation method, or mixing method.

In some embodiments, wherein the kit is formulated as an oral pharmaceutical composition.

In some embodiments, wherein the kit is formulated to be administered orally, by inhalation, through the digestive tract, or through the respiratory tract.

In some embodiments, wherein the kit is formulated to deliver the nucleic acid through in vitro cells delivery, or in vivo digestive tract delivery.

In another aspect, the present application provides the use of the kit in the manufacture of a medicament for the prevention and/or treatment of diseases that can be prevented and/or treated with nucleic acid, or for in vivo delivering nucleic acid to a subject in need thereof.

### Method of delivering nucleic acid

In another aspect, the present application provides a method of delivering nucleic acid to a target cell, comprising the administration to the target cell of the pharmaceutical composition of the present application or a lipid nucleic acid mixture formulated from the kit of the present application.

In another aspect, the present application provides a method of in vivo delivering nucleic acid to a subject in need thereof, comprising administrating to the subject the pharmaceutical composition of the present application or a lipid nucleic acid mixture formulated from the kit of the present application.

In some embodiments, the subject is human or an animal, such as a mammal.

In some embodiments, the nucleic acid is in vivo delivered to the blood circulation or to the target tissue/cell of the subject.

In some embodiments, the method comprises administering the medicament orally, by inhalation, or by injection.

In some embodiments, the method comprises administering the medicament through the digestive tract, or through the respiratory tract.

In some embodiments, the method comprises administering the medicament orally.

Item 1: use of a lipid composition in the manufacture of an agent for delivering nucleic acid, wherein the lipid composition comprises one or more compounds of formula (I),

A-L₁-L₃-L₂-B (I)

or salts, hydrates, or solvates thereof:
wherein:
L₁ is absent, straight or branched C₁₋₂₄ alkyl, straight or branched C₂₋₂₄ alkenyl, aryl optionally substituted by alkoxy or hydroxy, or straight or branched C₁₋₂₄ alkyl optionally substituted by aralkyl;
L₂ is absent, straight or branched C₁₋₂₄ alkyl optionally substituted by amine, or straight or branched C₂₋₂₄ alkenyl;
L₃ is 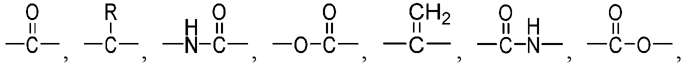 cycloalkyl optionally substituted by oxo, or heterocyclyl optionally substituted by oxo;
A is hydrogen, hydroxy, -COOH, -N(R')₂, straight or branched C₁₋₂₄ alkyl optionally substituted by hydroxy, straight or branched C₂₋₂₄ alkenyl, straight or branched C₂₋₂₄ heteroalkenyl optionally substituted by one or more substituents selected from hydroxy or amine, aryl optionally substituted by alkoxy, aminosulfonyl, or hydroxy, or heterocyclyl optionally substituted by oxo;
B is hydroxy, -CHO, -COOH, -C(O)R', -OC(O)R', straight or branched C₁₋₂₄ alkyl, straight or branched C₂₋₂₄ alkenyl;
R is hydrogen, straight or branched C₁₋₂₄ alkyl;
each R' is independently straight or branched C₁₋₂₄ alkyl.

Item 2: the use of Item 1, wherein the lipid composition is used for delivering the nucleic acid via oral administration, inhalation, or injection.

Item 3: the use of Item 2, wherein the lipid composition is used for delivering the nucleic acid via oral administration.

Item 4: the use of any one of the preceding items, wherein the delivery comprises in vitro cells delivery, or in vivo digestive tract delivery.

Item 5: the use of any one of the preceding items, wherein the lipid composition is mixed with the nucleic acid to prepare a lipid nucleic acid mixture, optionally, the lipid nucleic acid mixture is prepared by heating method, reverse-phase evaporation method, or mixing method.

Item 6: the use of Item 5, wherein the heating method comprises mixing the lipid composition with the nucleic acid to obtain a mixture, and heating the mixture at a temperature selected from 25°C to 100°C, 30°C to 100°C, 40°C to 100°C, 50°C to 100°C, 60°C to 100°C, 70°C to 100°C, 80°C to 100°C, 90°C to 100°C, or 95°C to 100°C.

Item 7: the use of Item 6, wherein heating the mixture at a temperature selected from 30°C, 35°C, 37°C, 40°C, 45°C, 50°C, 55°C, 60°C, 65°C, 70°C, 75°C, 80°C, 85°C, 90°C, 95°C, or 100°C.

Item 8: the use of any one of Item 6 or 7, wherein mixing the lipid composition with the nucleic acid comprises adding an organic solvent solution of the lipid composition to an aqueous solution of the nucleic acid.

Item 9: the use of Item 5, wherein the reverse-phase evaporation method comprises mixing an aqueous solution of the nucleic acid with an organic solvent solution of a lipid compound to obtain a mixture solution, and then removing the organic solvent from the mixture solution.

Item 10: the use of Item , wherein the organic solvent in the mixture solution is removed at a temperature selected from about 25°C to about 70°C, 30°C to about 70°C, about 30°C to about 65°C, about 40°C to about 65°C, about 40°C to about 60°C, or about 50°C to about 60°C.

Item 11: the use of Item 9, wherein mixing an aqueous solution of the nucleic acid with an organic solvent solution of a lipid compound comprises adding the aqueous solution of the nucleic acid into the organic solvent solution of the lipid compound.

Item 12: the use of any one of the preceding items, L₁ is absent.

Item 13: the use of any one of the preceding items, L₁ is straight or branched C₁₋₂₄ alkyl.

Item 14: the use of any one of the preceding items, L₁ is straight or branched C₂₋₂₄ alkenyl.

Item 15: the use of any one of the preceding items, L₁ is aryl optionally substituted by alkoxy or hydroxy.

Item 16: the use of any one of the preceding items, L₁ is straight or branched C₁₋₂₄ alkyl optionally substituted by aralkyl.

Item 17: the use of any one of the preceding items, L₂ is absent.

Item 18: the use of any one of the preceding items, L₂ is straight or branched C₁₋₂₄ alkyl optionally substituted by amine.

Item 19: the use of any one of the preceding items, L₂ is straight or branched C₂₋₂₄ alkenyl.

Item 20: the use of any one of the preceding items, L₃ is

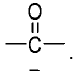

Item 21: the use of any one of the preceding items, L₃ is

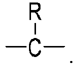

Item 22: the use of any one of the preceding items, L₃ is

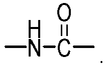

Item 23: the use of any one of the preceding items, L₃ is

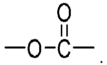

Item 24: the use of any one of the preceding items, L₃ is

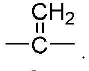

Item 25: the use of any one of the preceding items, L₃ is

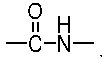

Item 26: the use of any one of the preceding items, L₃ is

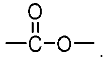

Item 27: the use of any one of the preceding items, L₃ is cycloalkyl optionally substituted by oxo.

Item 28: the use of any one of the preceding items, L₃ is heterocyclyl optionally substituted by oxo.

Item 29: the use of any one of the preceding items, A is hydrogen.

Item 30: the use of any one of the preceding items, A is hydroxy.

Item 31: the use of any one of the preceding items, A is -COOH.

Item 32: the use of any one of the preceding items, A is -N(R')₂.

Item 33: the use of any one of the preceding items, A is straight or branched C₁₋₂₄ alkyl optionally substituted by hydroxy.

Item 34: the use of any one of the preceding items, A is straight or branched C₂₋₂₄ alkenyl.

Item 35: the use of any one of the preceding items, A is straight or branched C₂₋₂₄ heteroalkenyl optionally substituted by one or more substituents selected from hydroxy or amine.

Item 36: the use of any one of the preceding items, A is aryl substituted by aminosulfonyl or hydroxy.

Item 37: the use of any one of the preceding items, A is heterocyclyl optionally substituted by oxo.

Item 38: the use of any one of the preceding items, B is hydroxy.

Item 39: the use of any one of the preceding items, B is -CHO.

Item 40: the use of any one of the preceding items, B is -COOH.

Item 41: the use of any one of the preceding items, B is -C(O)R'.

Item 42: the use of any one of the preceding items, B is -OC(O)R'.

Item 43: the use of any one of the preceding items, B is straight or branched C₁₋₂₄ alkyl.

Item 44: the use of any one of the preceding items, B is straight or branched C₂₋₂₄ alkenyl.

Item 45: the use of any one of the preceding items, R is hydrogen.

Item 46: the use of any one of the preceding items, R is straight or branched C₁₋₂₄ alkyl.

Item 47: the use of any one of the preceding items, each R' is independently straight or branched C₁₋₂₄ alkyl.

Item 48: the use of any one of the preceding items, wherein A is straight C₂₋₂₄ alkenyl having 1-5 double bonds.

Item 49: the use of any one of the preceding items, wherein B is straight C₂₋₂₄ alkenyl having 1-5 double bonds.

Item 50: the use of Item 48 or 49, wherein the double bond is positioned in the straight C₂₋₂₄ alkenyl at a position selected from C1-C2 position, C2-C3 position, C3-C4 position, C4-C5 position, C5-C6 position, C6-C7 position, C7-C8 position, C8-C9 position, C9-C10 position, C10-C11 position, C12-C13 position, C13-C14, C14-C15, C15-C16, C16-C17 position or combination thereof.

Item 51: use of a lipid composition in the manufacture of an agent for delivering nucleic acid, wherein the lipid composition comprises one or more compounds selected from the group consisting of

| **Lipid Compound No.** | **Product Name** |
|---|---|
| 501 | methyl jasmonate |
| 502 | 6,10-dimethylundeca-5,9-dien-2-one (isomer mixture) |
| 503 | 1-methylcyclopentan-1-ol |
| 505 | tridecan-2-ol |
| 519 | N-(2-hydroxyethyl)tricosanamide |
| 520 | (7Z,10Z,13Z,16Z)-docosa-7,10,13,16-tetraenoic acid |
| 521 | N-(2-hydroxyethyl)tetracosanamide |
| 522 | 6-(6-aminohexanoylamino)hexanoic acid |
| 525 | (E)-4-oxonon-2-enal |
| 526 | (E)-hex-3-en-1-ol |
| 527 | jasmonic acid (isomer mixture) |
| 528 | 2-methylnonane |
| 529 | hexadecanedioic acid |
| 530 | 2-aminohexanedioic acid |
| 537 | capsaicin |
| 542 | (4Z,7Z,10Z,13Z,16Z)-docosa-4,7,10,13,16-pentaenoic acid |
| 543 | 12-Hydroxyjasmonic acid |
| 572 | pentadecanedioic acid |
| 573 | decanamide |
| 574 | pentadecanal |
| 578 | N-(2-hydroxyethyl)hexadecanamide |
| 593 | pentadecan-2-one |
| 594 | tetradecanal |
| 595 | (2S)-2-aminohexanedioic acid |
| 596 | 2-methylpropanedioic acid |
| 597 | nylon 6/12 (dodecanedioic acid-1,6-hexanediamine polymer) |
| 598 | 3,3-dimethylpentanedioic acid |
| 599 | hexadecanamide |
| 600 | (E)-octadec-9-en-1-ol |
| 601 | octadecanamide |
| 602 | hexadecanal |
| 603 | 4-hydroxy-4-methyloxan-2-one |
| 604 | decan-2-one |
| 605 | pentadecanoic acid |
| 606 | (E)-2-methylbut-2-enedioic acid |
| 607 | (E)-hex-3-enedioic acid |
| 608 | 2-methylbutanedioic acid |
| 609 | pentadecan-1-ol |
| 610 | pentanamide |
| 611 | dodecanamide |
| 612 | 3-acetyloxolan-2-one |
| 613 | butyl hexanoate |
| 614 | hexyl butanoate |
| 615 | decanal |
| 616 | 2-(3-oxo-2-pentylcyclopentyl)acetic acid |
| 617 | nonyl acetate |
| 618 | (2E,4E)-hepta-2,4-dienal |
| 619 | oct-1-en-3-one |
| 620 | (5Z,8Z,11Z,14Z)-icosa-5,8,11,14-tetraenoic acid |
| 621 | pent-3-en-2-one (isomer mixture) |
| 622 | 3-oxo-N-[(3S)-2-oxooxolan-3-yl]hexanamide |
| 623 | 2-oxobutanedioic acid |
| 624 | icosanoic acid |
| 625 | (2R)-2-aminohexanedioic acid |
| 626 | 2-methylidenebutanedioic acid |
| 627 | propanedioic acid |
| 628 | butanedioic acid |
| 635 | tetradecan-1-ol |
| 636 | octadecan-1-ol |
| 802 | [(2E,6E)-3,7,11-trimethyldodeca-2,6,10-trienyl] dodecanoate |
| 803 | [(2E,6E)-3,7,11-trimethyldodeca-2,6,10-trienyl] decanoate |
| 804 | [(9Z,12Z)-octadeca-9,12-dienyl] hexadecanoate |
| 805 | dodecyl (9Z,12Z,15Z)-octadeca-9,12,15-trienoate |
| 806 | [(Z)-octadec-9-enyl] (9Z,12Z,15Z)-octadeca-9,12,15-trienoate |
| 807 | (5Z,8Z,11Z,14Z)-N-(5-hydroxypentyl)icosa-5,8,11,14-tetraenamide |
| 808 | (5Z,8Z,11Z,14Z)-N-(3-hydroxypropyl)icosa-5,8,11,14- |
| | tetraenamide |
| 809 | [(2E,6E)-3,7,11-trimethyldodeca-2,6,10-trienyl] heptanoate |
| 810 | (5Z,8Z,11Z,14Z)-N-[2-(diethylamino)ethyl]icosa-5,8,11,14-tetraenamide |
| 811 | tetradecyl (9Z,12Z)-octadeca-9,12-dienoate |
| 812 | [(9Z,12Z)-octadeca-9,12-dienyl] icosanoate |
| 813 | tridecyl (Z)-hexadec-9-enoate |
| 814 | 3-methylbut-3-enyl icosanoate |
| 815 | 3-methylbut-3-enyl docosanoate |
| 816 | [(Z)-hex-3-enyl] (2R)-2-hydroxy-3-methylbutanoate |
| 817 | docosyl (Z)-tetradec-9-enoate |
| 818 | heptadecyl (Z)-hexadec-9-enoate |
| 819 | tetracosyl (Z)-hexadec-9-enoate |
| 820 | henicosyl (Z)-hexadec-9-enoate |
| 821 | pentadecyl (Z)-hexadec-9-enoate |
| 822 | nonadecyl (Z)-hexadec-9-enoate |
| 823 | docosyl (Z)-hexadec-9-enoate |
| 824 | tetradecyl (9Z,12Z,15Z)-octadeca-9,12,15-trienoate |
| 825 | tricosyl (Z)-hexadec-9-enoate |
| 826 | (5Z,8Z,11Z,14Z)-N-(4-sulfamoylphenyl)icosa-5,8,11,14-tetraenamide |
| 827 | 4-methylheptan-3-yl (Z)-hexadec-9-enoate |
| 828 | 4-methylheptan-3-yl (9Z,12Z)-octadeca-9,12-dienoate |
| 829 | 15-methylhexadecyl tetradecanoate |
| 830 | (2S)-2-(octadecanoylamino)-3-phenylpropanoic acid |

or salts, hydrates, or solvates thereof.

Item 52: the use of any one of the preceding items, wherein the salt is a pharmaceutically acceptable salt.

Item 53: the use of any one of the preceding items, wherein the compound derives from extracts of traditional Chinese medicine.

Item 54: the use of Item 53, wherein the extracts of traditional Chinese medicine are obtained by decoction and extraction of traditional Chinese medicine.

Item 55: the use of Item 54, wherein the extracts of traditional Chinese medicine are obtained by soaking the traditional Chinese medicine in water, followed by strong fire decoction and weak fire decoction in sequence, concentrating the decocted Chinese medicine liquid, adding chloroform-methanol, chloroform and water in sequence and mixing, and extracting the chloroform layer.

Item 56: the use of any one of preceding items, wherein the nucleic acid comprises DNA or RNA, optionally, the DNA is selected from coding DNA or non-coding DNA, the RNA is selected from antisense nucleic acid, mRNA, lncRNA, or small RNA

Item 57: the use of Item 56, wherein the nucleic acid comprises small nucleic acid having a length of 14-32bp, 16-28bp or 18-24bp.

Item 58: the use of any one of preceding items, wherein the nucleic acid is single-stranded or double-stranded.

Item 59: the use of any one of preceding items, wherein the nucleic acid has a stem-loop structure.

Item 60: the use of any one of the preceding items, wherein the nucleic acid is used for treating diseases.

Item 61: the use of Item 60, wherein the nucleic acid is used for treating cancer, inflammation, fibrotic disease, autoimmune disease or autoinflammatory disease, bacterial infection, behavioral and psychiatric disorder, blood disease, chromosomal disease, congenital and hereditary disease, connective tissue disease, digestive disease, ear nose throat disease, endocrine disease, environmental disease, eye disease, female reproductive disease, fungal infection, heart disease, hereditary cancer syndrome, immune system disorder, kidney and urinary disease, lung disease, male reproductive disease, metabolic disorder, mouth disease, musculoskeletal disease, myelodysplastic syndrome, neonatal screening, nutritional disease, parasitic disease, rare cancer, rare disease, skin disease, or viral infection.

Item 62: the use of Item 60, wherein the nucleic acid is used for treating hepatocellular carcinoma, corneal neovascularization, recurrent or refractory anaplastic astrocytoma (WHO Grade III), or secondary glioblastoma (WHO Grade IV), advanced squamous cell lung cancer, acromegaly, psoriasis, Duchenne muscular dystrophy, advanced non-small cell lung cancer, metastatic castration-resistant prostate cancer, cytomegalovirus retinitis, HIV infection, hepatitis B, hepatitis C, hyperlipoprotein disease, total knee replacement, type 2 diabetes, familial amyloid polyneuropathy (FAP), wet macular degeneration (e.g., neovascular age-related macular degeneration, subfoveal neovascular age-related macular degeneration, exudative age-related macular degeneration), hypercholesterolemia, Crohn's disease, extensive liver fibrosis, infantile spinal muscular atrophy, melanoma, neonatal coronary artery disease, mild allergic asthma, chronic lymphocytic leukemia, and hypertriglyceridemia, small cell obliterans of the liver with renal or lung dysfunction after hematopoietic stem cell transplantation, or hereditary transthyretin amyloidosis.

Item 63: a pharmaceutical composition, comprising a lipid composition and a nucleic acid, wherein the lipid composition comprises one or more compounds of formula (I),

A-L₁-L₃-L₂-B (I)

or salts, hydrates, or solvates thereof:

wherein:
L₁ is absent, straight or branched C₁₋₂₄ alkyl, straight or branched C₂₋₂₄ alkenyl, aryl optionally substituted by alkoxy or hydroxy, or straight or branched C₁₋₂₄ alkyl optionally substituted by aralkyl;
L₂ is absent, straight or branched C₁₋₂₄ alkyl optionally substituted by amine, or straight or branched C₂₋₂₄ alkenyl;
L₃ is 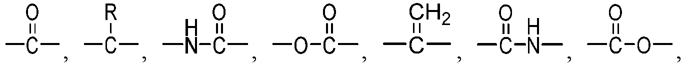 cycloalkyl optionally substituted by oxo, or heterocyclyl optionally substituted by oxo;
A is hydrogen, hydroxy, -COOH, -N(R')₂, straight or branched C₁₋₂₄ alkyl optionally substituted by hydroxy, straight or branched C₂₋₂₄ alkenyl, straight or branched C₂₋₂₄ heteroalkenyl optionally substituted by one or more substituents selected from hydroxy or amine, aryl optionally substituted by alkoxy, aminosulfonyl, or hydroxy, or heterocyclyl optionally substituted by oxo;
B is hydroxy, -CHO, -COOH, -C(O)R', -OC(O)R', straight or branched C₁₋₂₄ alkyl, straight or branched C₂₋₂₄ alkenyl;
R is hydrogen, straight or branched C₁₋₂₄ alkyl;
each R' is independently straight or branched C₁₋₂₄ alkyl.

Item 64: a pharmaceutical composition comprising a lipid composition and a nucleic acid, wherein the lipid composition comprises one or more compounds selected from the group consisting of lipids 501, 502, 503, 505, 519, 520, 521, 522, 525, 526, 527, 528, 529, 530, 537, 542, 543, 572, 573, 574, 578, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830, or salts, hydrates, or solvates thereof.

Item 65: the pharmaceutical composition of Item 63 or 64, whrerein the pharmaceutical composition is formulated to be administered orally, by inhalation, through the digestive tract, or through the respiratory tract.

Item 66: the pharmaceutical composition of Item 63 or 64, wherein the pharmaceutical composition is used for delivering the nucleic acid through in vitro cells delivery, or in vivo digestive tract delivery.

Item 67: the pharmaceutical composition of Item 63 or 64, wherein the pharmaceutical composition is an oral pharmaceutical composition.

Item 68: the pharmaceutical composition of any one of Items 63-67, wherein the lipid composition and the nucleic acid are present at least partially or wholly in the form of a lipid nucleic acid mixture.

Item 69: the pharmaceutical composition of Item 68, wherein the lipid nucleic acid mixture is prepared by heating method, reverse-phase evaporation method, or mixing method.

Item 70: the pharmaceutical composition of Item 69, wherein the heating method comprises mixing the lipid composition with the nucleic acid to obtain a mixture, and heating the mixture at a temperature selected from 25°C to 100°C, 50°C to 100°C, 80°C to 100°C , or 95°C to 100°C.

Item 71: the pharmaceutical composition of Item 70, wherein heating the mixture at a temperature selected from 37°C, 60°C, 80°C, or 100°C.

Item 72: the pharmaceutical composition of any one of Items 69-71, wherein mixing the lipid composition with the nucleic acid comprises adding an organic solvent solution of the lipid composition to an aqueous solution of the nucleic acid.

Item 73: the pharmaceutical composition of Item 69, wherein the reverse-phase evaporation method comprises mixing an aqueous solution of the nucleic acid with an organic solvent solution of a lipid compound to obtain a mixture solution, and then removing the organic solvent from the mixture solution at 25°C to 70°C, 30°C to 65°C, or 40°C to 60°C.

Item 74: the pharmaceutical composition of Item 73, wherein removing the organic solvent from the mixture solution at 55°C.

Item 75: the pharmaceutical composition of any one of Items 69 and 73-74, wherein the reverse-phase evaporation method comprises adding the aqueous solution of the nucleic acid into the organic solvent solution of the lipid composition.

Item 76: the use of the pharmaceutical composition in the manufacture of a medicament of any one of Items 63-75, the medicament is used for the prevention and/or treatment of diseases that can be prevented and/or treated with nucleic acid, or for in vivo delivering nucleic acid to a subject in need thereof.

Item 77: a kit, comprising:
one or more compounds of formula (I), positioned in a first container,

   A-L₁-L₃-L₂-B (I)
or salts, hydrates, or solvates thereof;
and a nucleic acid positioned in a second container;
wherein:
   L₁ is absent, straight or branched C₁₋₂₄ alkyl, straight or branched C₂₋₂₄ alkenyl, aryl optionally substituted by alkoxy or hydroxy, or straight or branched C₁₋₂₄ alkyl optionally substituted by aralkyl;
   L₂ is absent, straight or branched C₁₋₂₄ alkyl optionally substituted by amine, or straight or branched C₂₋₂₄ alkenyl;
   L₃ is 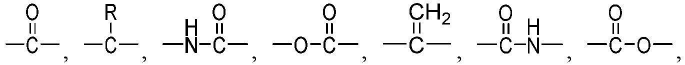 cycloalkyl optionally substituted by oxo, or heterocyclyl optionally substituted by oxo;
   A is hydrogen, hydroxy, -COOH, -N(R')₂, straight or branched C₁₋₂₄ alkyl optionally substituted by hydroxy, straight or branched C₂₋₂₄ alkenyl, straight or branched C₂₋₂₄ heteroalkenyl optionally substituted by one or more substituents selected from hydroxy or amine, aryl optionally substituted by alkoxy, aminosulfonyl, or hydroxy, or heterocyclyl optionally substituted by oxo;
   B is hydroxy, -CHO, -COOH, -C(O)R', -OC(O)R', straight or branched C₁₋₂₄ alkyl, straight or branched C₂₋₂₄ alkenyl;
   R is hydrogen, straight or branched C₁₋₂₄ alkyl;
   each R' is independently straight or branched C₁₋₂₄ alkyl.

Item 78: a kit, comprising:
one or more compounds positioned in a first container, or pharmaceutically acceptable salts, hydrates, or solvates thereof, and a nucleic acid positioned in a second container, wherein the one or more compounds are selected from the group consisting of lipids 501, 502, 503, 505, 519, 520, 521, 522, 525, 526, 527, 528, 529, 530, 537, 542, 543, 572, 573, 574, 578, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830.

Item 79: the kit of Item 77 or 78, wherein the lipid composition and the nucleic acid are formulated at least partially or wholly a lipid nucleic acid mixture before use.

Item 80: the kit of Item 79, wherein the lipid nucleic acid mixture is prepared by heating method, reverse-phase evaporation method, or mixing method.

Item 81: the kit of Item 80, wherein the heating method is performed at a temperature selected from 25°C to 100°C, 50°C to 100°C, 80°C to 100°C, or 95°C to 100°C.

Item 82: the kit of Item 81, wherein the heating method is performed at a temperature selected from 37°C, 60°C, 80°C, or 100°C.

Item 83: the kit any one of Items 80-82, wherein the heating method comprises adding an organic solvent solution of the lipid composition to an aqueous solution of the nucleic acid.

Item 84: the kit of Item 80, wherein the reverse-phase evaporation method is performed at a temperature selected from 25°C to 70°C, 30°C to 65°C, or 40°C to 60°C.

Item 85: the kit of Item 84, wherein the reverse-phase evaporation method is performed at a temperature of 55°C.

Item 86: the kit of any one of Items 80 and 84-85, wherein the reverse-phase evaporation method comprises adding an aqueous solution of the nucleic acid to an organic solvent solution of the lipid composition.

Item 87: the kit of any one of Items 77-86, wherein the kit is formulated to be an oral pharmaceutical composition.

Item 88: the kit of any one of Items 77-86, wherein the kit is formulated to be administered orally, by inhalation, through the digestive tract, or through the respiratory tract.

Item 89: the kit of any one of Items 77-86, wherein the kit is formulated to deliver the nucleic acid through in vitro cells delivery, or in vivo digestive tract delivery.

Item 90: the use of the kit in the manufacture of a medicament of Items 77-89, the medicament is used for the prevention and/or treatment of diseases that can be prevented and/or treated with nucleic acid, or for in vivo delivering nucleic acid to a subject in need thereof.

Item 91: a method of delivering nucleic acid to a target cell, comprising the administration to the target cell of the pharmaceutical composition of any one of Items 63-75, or a lipid nucleic acid mixture formulated from the kit of any one of Items 77-89.

Item 92: a method of in vivo delivering nucleic acid to a subject in need thereof, comprising administrating to the subject in need thereof the pharmaceutical composition of any one of Items 63-75, or a lipid nucleic acid mixture formulated from the kit of any one of Items 77-89.

Item 93: the method of Item 92, wherein the subject is human or an animal, such as a mammal.

Item 94: the method of Item 92 or 82, wherein the nucleic acid is in vivo delivered to the blood circulation or to the target tissue/cell of the subject.

Item 95: the method of any one of Items 92-94, wherein comprises administering the medicament orally, by inhalation, or by injection.

Item 96: the method of any one of Items 92-95, wherein comprises administering the medicament through the digestive tract, or through the respiratory tract.

Item 97: the method of Item 96, wherein comprises administering the medicament orally.

The above technical solution provided in the present application can significantly improve the efficient targeted delivery of nucleic acid, and overcome the defects of prior art nucleic acid liposomes that need to be improved, such as low encapsulation rate, poor safety, poor stability, complex preparation process, inhomogeneous product, poor reproducability, and poor targeting.

The above features mentioned in the present disclosure, or the features mentioned in examples, may be arbitrarily combined. All features disclosed in the specification of the present application may be used with any composition form, and each feature disclosed in the specification may be replaced by any alternative feature providing the same, equal or similar purpose. Thus, unless otherwise specified, the features disclosed are only general examples of equal or similar features.

The present disclosure is further described in combination with specific examples. It is understood that these examples are used only to illustrate the present disclosure and not to limit the scope of the present disclosure .

### Example 1: Extraction, Identification, and Synthesis of Lipids Derived from Traditional Chinese Medicine Sources

### 1.1 Preparation of Traditional Chinese Medicine Decoction

1) 100g of traditional Chinese medicine decoction pieces (purchased from Beijing Tong Ren Tang Pharmacy) were added 1000mL ddH₂O and soaked for 30 minutes.
2) The traditional Chinese medicine was decocted in a decoction pot over high heat for 15 minutes followed by decoction over low heat for 20 minutes.
3) 400mL of the decocted medicinal liquid was added to a rotary evaporator, and concentrate at 60°C, 60rpm for 30 minutes to 100mL.

### 1.2 Extraction of Lipids from Traditional Chinese Medicine

1) 600mL of chloroform-methanol mixture (chloroform: methanol=1: 2, v/v) was added to 160mL of the decoction obtained in above 1.1 (rotary evaporator concentrated) such that chloroform: methanol: water=1:2:0.8, and stirred for 10-15 minutes.
2) 200mL of chloroform was added to a conical flask, and stirred for 10 minutes.
3) 200mL of ddH2O was added to the conical flask such that chloroform: methanol:water=2: 2:1.8, and stirred for 10 minutes.
4) The upper layer liquid and insoluble substances in the middle layer were removed, the bottom chloroform layer was collected, and stored at -40°C.

### 1.3 Identification of Lipids from Traditional Chinese Medicine

HPLC-MS/MS was used to identify lipid components from traditional Chinese medicine.

### Instrument Conditions

### 1) Chromatography Conditions:

Instrument: Ultimate 3000; Column: Kinetex C18 (100×2.1mm, 1.9µm); Column Temperature: 45°C; Mobile Phase: A: acetonitrile:water (V/V, 60:40) with 10 mmol/L ammonium formate, B: acetonitrile:isopropanol (10:90, V/V) with 10 mmol/L ammonium formate and 0.1% formic acid. Flow Rate: 0.4 mL/min; Injection Volume: 4 µL.

### 2) Mass Spectrum Parameters:

a) Positive Mode: Heater Temperature 300°C, Sheath Gas Flow Rate 45 arb, Aux Gas Flow Rate 15 arb, Sweep Gas Flow Rate 1 arb, Nozzle Voltage 3.0 KV, Capillary Temperature 350°C, S-Lens RF Level 30%. Scan Range: 200-1500.
b) Negative Mode: Heater Temperature 300°C, Sheath Gas Flow Rate 45 arb, Aux Gas Flow Rate 15 arb, Sweep Gas Flow Rate 1 arb, Nozzle Voltage 2.5KV, Capillary Temperature 350°C, S-Lens RF Level 60%. Scan Range: 200-1500.

### 1.4 Acquisition of Lipids

The 59 fatty acid (FA) lipid compounds used in the following examples were purchased from Aladdin, Santa Cruz, MedChemExpress, Cayman, Macklin, and Sigma-Aldrich.

### Example 2: Preparation of Lipid Nucleic Acid Mixture (Heating Method):

The concentration of the lipid chloroform solution is 10 mg/ml (stock solution). The solutions were grouped according to the lipid compound number.

10 nmol of small RNA (as shown in Table 2) was dissolved in 400 µl of DEPC-treated water in a glass tube, 10 µl of the corresponding lipid stock solution was added such that the lipid mass in the system was 100 µg. The mixture was mixed thoroughly, heated in a 90°C water bath for 15 minutes, and then cooled naturally to obtain the lipid nucleic acid mixture of lipid and small RNA.

**Table 2. Small RNA Sequence Information (purchased from General Biol Co., Ltd. )**

| **Name** | **Sequence** | **Modification** |
|---|---|---|
| PGY-sRNA-26 | TCCGGAATGATTGGGCGTAAAGC GT (SEQ ID No. 1) | 3'-2O methylated |
| PGY-sRNA-26 Reverse Transcription Primer | GTCGTATCCAGTGCACGCTCCGA GGTATTCGCACTGGATACGACAC GCTT (SEQ ID No. 2) | |
| PGY- sRNA-26 Forward Primer | TCGCGCTCCGGAATGATTGGG (SEQ ID No. 3) | |
| PGY-sRNA-26 Reverse Primer | GTGCACGCTCCGAGGT (SEQ ID No. 4) | |

### Example 3: In Vivo Delivery Study of Lipid Nucleic Acid Mixture

### 1. Experimental Animals:

C57BL/6 male mice, approximately 6 weeks old, weighing 20-24g, were obtained from Beijing Vital River Laboratory Animal Technology Co., Ltd. The mice were housed in the SPF-grade animal facility at the Animal Center of the Institute of Basic Medical Sciences, Chinese Academy of Medical Sciences. The mice were fasted for 12 hours before gavage administration.

### 2. Preparation of Lipid-Small RNA Mixture:

The lipid nucleic acid mixture was prepared following the method described in Example 2. The resultant lipid nucleic acid mixture was orally administered to each mouse at a dose of 100 µg lipid:10 nmol single-stranded 3'-methylated small RNAPGY-sRNA-26.

### 3. Detection of Relative Entry Amount:

### 3.1 Experimental Groups:

1) Blank group (n=4): mice were administered with 500 µl saline by gavage, this group served as a blank control;
2) Free uptake group (n=4): mice were directly administered with small RNA PGY-sRNA-26 solution (10 nmol/mouse, 500 µl) by gavage, this group served as a negative control;
3) Lipid-treated group (n=4): mice were administered with 500 µl lipid nucleic acid mixture prepared in step 2 by gavage.

### 3.2 Tissue Sampling and RNA Extraction:

After 12 hours of gavage administration, 500 µl blood from the eyeball of each mouse was sampled, added to 1.5 ml Trizol Reagent LS (purchased from Invitrogen), thoroughly mixed for lysis. 3 ml of Trizol Reagent LSwas added to tissue samples and homogenized for complete lysis, wherein the sampled tissues include heart, liver, spleen, lung, kidney, stomach, intestine, brain, blood, femur, pancreas. RNA was extracted from 1ml of the blood or the tissue lysate following the steps below:
- Centrifuge at 4°C, 12,000rpm for 5 min, discard the precipitate, transfer Trizol to a new centrifuge tube;
- Add chloroform at 200µL chloroform /mL Trizol, shake well, stand at room temperature for 5 min;
- Centrifuge at 4°C, 12,000rpm for 15 min;
- Take the upper aqueous phase to another centrifuge tube, add isopropanol at 0.4 mL isopropanol /mL Trizol, mix well, and stand at low temperature for 10-20 min;
- Centrifuge at 4°C, 12,000 rpm for 15 min, discard the supernatant, RNA precipitated at the bottom of the tube;
- Add 1 mL of 75% ethanol, gently shake the centrifuge tube, suspend the precipitate;
- Centrifuge at 4°C, 12,000 rpm for 15 min, discard the supernatant, add 1 mL of 75% ethanol, gently shake the centrifuge tube, suspend the precipitate;
- Centrifuge at 4°C, 12,000rpm for 15min, discard the supernatant, air-dry at room temperature, dissolve RNA samples in 50 µL RNase-free H2O, measure OD value to quantify RNA concentration.

### 3.3 Reverse Transcription of sRNA to cDNA:

sRNA was reverse transcribed to cDNA using the High-Capacity cDNA Reverse Transcription Kit (Applied Biosystems, cat. no. 4368813) by stem-loop method (see, e.g., "Real-time quantification of microRNAs by stem-loop RT-PCR, Nucleic Acids Res. 2005 Nov 27;33(20):e179", incorporated herein by reference). The reverse transcription system is as follows: Template RNA (200 ng/µL) 10 µL, 10X RT Buffer 2.0 µL, 25X dNTP Mix (100 mM) 0.8 µL, U6 RT stem-loop primer (10 µM) 2.0µL, PGY-sRNA-26 RT stem-loop primer (10 µM) 2.0µL, MultiScribeTM Reverse Transcriptase (50 U/µL) 1.0µL, RNAse Inhibitor (40 U/µL) 1.0µL, nuclease-free H2O 1.2µL. After transient centrifugation, the reverse transcription system was placed in a PCR machine with the following reaction conditions: (1) 25°C, 10 min; (2) 37°C, 120 min; (3) 85°C, 5 min; (4) 4°C, stop reaction. After the reaction, add 20 µL RNAse-free ddH2O, adjust the final volume to 40 µL. The stem-loop primers used in this reverse transcription process were synthesized by Beijing Tianyi Huiyuan Biotechnology Co., Ltd. (U6 was used as a standard reference gene to calculate its relative expression, because U6 RT primer was used as a reference gene in relative quantification of RT-qPCR reactions for small RNA):
U6 RT stem-loop primer: 
GTCGTATCCAGTGCAGGGTCCGAGGTATTCGCACTGGATACGACAAAAATA
TG (SEQ ID No. 5);
PGY-sRNA-26 RT stem-loop primer:
   GTCGTATCCAGTGCACGCTCCGAGGTATTCGCACTGGATACGACACGCTT (SEQ ID No. 2).

### 3.4 Quantitative PCR Amplification Reaction:

The qPCR reaction system has a total volume of 10 µl, including: 5 µL 2×SYBR Green Master Mix, 0.5 µl forward primer (10 µM), 0.5 µl reverse primer (10 µM), 1 µl cDNA obtained from reverse transcription, and 3 µl RNAse-free ddH2O. LightCycler 480 Fluorescent Quantitative PCR Instrument was used with the PCR reaction conditions as follows: 95°C, 5 min for initial denaturation, followed by entering the PCR amplification cycle: (1) 95°C, 10 s; (2) 55°C, 10 s; (3) 72°C, 20 s; a total of 40 cycles; finally, a cooling step at 40°C for 10 s. The forward and reverse primers for the amplification reaction were designed and synthesized by Beijing Tianyi Huiyuan Biotechnology Co., Ltd. (U6 F primer: GCGCGTCGTGAAGCGTTC (SEQ ID No. 6), U6 R primer: GTGCAGGGTCCGAGGT (SEQ ID No. 7); PGY-sRNA-26 forward primer: TCGCGCTCCGGAATGATTGGG (SEQ ID No. 3), reverse primer miR all rev: GTGCACGCTCCGAGGT (SEQ ID No. 4)).

### 3.5 Calculation of Relative Expression Using the 2-ΔCt Method.

### 4. The experimental results are illustrated in figures.

Figures 1-59 respectively illustrate the results of the blank group, free uptake group, and different lipid for delivering the single-stranded small RNA PGY-sRNA-26 into different target tissues.

### Conclusion:

Lipids 501, 502, 519, 520, 522, 526, 527, 528, 529, 530, 537, 542, 543, 572, 573, 574, 578, 593, 594, 595, 596, 597, 598, 599, 600, 601, 602, 603, 604, 607, 608, 609, 613, 614, 616, 620, 621, 623, 624, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830 can effectively orally deliver single-stranded sRNA nucleic acids into mouse heart tissue.

Lipids 501, 503, 505, 520, 521, 526, 527, 528, 529, 530, 537, 542, 543, 572, 573, 574, 578, 593, 594, 595, 596, 597, 598, 599, 600, 601, 602, 603, 604, 606, 607, 609, 612, 613, 614, 615, 616, 617, 618, 619, 620, 621, 622, 623, 624, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830 can effectively orally deliver single-stranded sRNA nucleic acids into mouse liver tissue.

Lipids 501, 502, 503, 520, 522, 525, 526, 527, 528, 529, 530, 537, 542, 543, 572, 573, 574, 578, 593, 594, 595, 596, 597, 598, 599, 600, 601, 602, 603, 604, 608, 610, 611, 613, 614, 615, 616, 617, 619, 620, 622, 623, 625, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830 can effectively orally deliver single-stranded sRNA nucleic acids into mouse spleen tissue.

Lipids 501, 503, 519, 520, 522, 525, 526, 527, 528, 529, 530, 537, 542, 543, 578, 593, 594, 595, 596, 597, 598, 599, 600, 601, 602, 603, 604, 608, 609, 610, 613, 614, 615, 616, 617, 618, 620, 621, 622, 624, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830 can effectively orally deliver single-stranded sRNA nucleic acids into mouse lung tissue.

Lipids 501, 502, 503, 519, 521, 525, 526, 527, 528, 529, 530, 537, 542, 543, 572, 574, 578, 593, 594, 595, 596, 597, 598, 599, 600, 601, 602, 603, 604, 607, 609, 610, 613, 617, 619, 620, 623, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830 can effectively orally deliver single-stranded sRNA nucleic acids into mouse kidney tissue.

Lipids 501, 502, 503, 505, 519, 522, 525, 526, 527, 528, 529, 530, 537, 542, 543, 572, 573, 574, 578, 593, 594, 595, 596, 597, 598, 599, 600, 601, 602, 603, 604, 605, 607, 608, 609, 610, 611, 612, 613, 615, 617, 619, 620, 621, 622, 623, 624, 625, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830 can effectively orally deliver single-stranded sRNAnucleic acids into mouse stomach tissue.

Lipids 501, 502, 503, 522, 525, 526, 527, 528, 529, 530, 537, 542, 543, 572, 573, 593, 594, 595, 596, 597, 598, 599, 600, 601, 602, 603, 604, 612, 613, 614, 615, 616, 617, 618, 619, 620, 621, 622, 623, 624, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830 can effectively orally deliver single-stranded sRNA nucleic acids into mouse intestinal tissue.

Lipids 501, 502, 503, 505, 520, 526, 527, 528, 529, 530, 537, 542, 543, 572, 573, 574, 578, 593, 594, 595, 596, 597, 598, 599, 600, 601, 602, 603, 604, 609, 613, 614, 615, 616, 617, 618, 619, 620, 621, 622, 623, 624, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830 can effectively orally deliver single-stranded sRNA nucleic acids into mouse brain tissue.

Lipids 501, 502, 503, 505, 521, 522, 526, 527, 528, 529, 530, 537, 542, 543, 572, 573, 574, 578, 593, 594, 595, 596, 597, 598, 599, 600, 601, 602, 603, 604, 605, 606, 607, 613, 614, 615, 616, 617, 619, 621, 622, 623, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830 can effectively orally deliver single-stranded sRNA nucleic acids into mouse blood.

Lipids 526, 527, 528, 529, 530, 537, 542, 543, 572, 573, 574, 578, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830 can effectively orally deliver single-stranded sRNA nucleic acids into mouse femur.

Lipids 802, 803, 804, 805, 806, 807, 808, 809, 810, 811, 812, 813, 814, 815, 816, 817, 818, 819, 820, 821, 822, 823, 824, 825, 826, 827, 828, 829, and 830 can effectively orally deliver single-stranded sRNA nucleic acids into mouse pancreatic tissue.

In the case of the 10 tissues and blood examined in the experiment, the most effective delivery of nucleic acids was observed in liver and stomach tissues.

Lipids 501, 526, 527, 528, 529, 530, 537, 542, 543, 593, 594, 595, 596, 597, 598, 599, 600, 601, 602, 603, 604, 613, 625, 627, 628, 635, 636, 802, 803, 806, 810, and 822 exhibited the best delivery efficacy, effectively delivering nucleic acids into each tissue and blood tested, suggesting their potential as a broad-spectrum nucleic acid delivery carrier.

All references mentioned in this invention are cited as references in this application, just as each reference is cited individually. Additionally, it should be understood that, after reading the teachings of this invention, those skilled in the art can make various changes or modifications, and such equivalent forms are also within the scope defined by the claims attached to this application.

## Claims

1. Use of a lipid composition in the manufacture of an agent for delivering nucleic acid, wherein the lipid composition comprises one or more compounds of formula (I),
A-L₁-L₃-L₂-B (I)
or salts, hydrates, or solvates thereof:
wherein:
L₁ is absent, straight or branched C₁₋₂₄ alkyl, straight or branched C₂₋₂₄ alkenyl, aryl optionally substituted by alkoxy or hydroxy, or straight or branched C₁₋₂₄ alkyl optionally substituted by aralkyl;
L₂ is absent, straight or branched C₁₋₂₄ alkyl optionally substituted by amine, or straight or branched C₂₋₂₄ alkenyl;
L₃ is 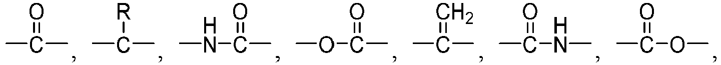 cycloalkyl optionally substituted by oxo, or heterocyclyl optionally substituted by oxo;
A is hydrogen, hydroxy, -COOH, -N(R')₂, straight or branched C₁₋₂₄ alkyl optionally substituted by hydroxy, straight or branched C₂₋₂₄ alkenyl, straight or branched C₂₋₂₄ heteroalkenyl optionally substituted by one or more substituents selected from hydroxy or amine, aryl optionally substituted by alkoxy, aminosulfonyl, or hydroxy, or heterocyclyl optionally substituted by oxo;
B is hydroxy, -CHO, -COOH, -C(O)R', -OC(O)R', straight or branched C₁₋₂₄ alkyl, straight or branched C₂₋₂₄ alkenyl;
R is hydrogen, straight or branched C₁₋₂₄ alkyl;
each R' is independently straight or branched C₁₋₂₄ alkyl.

2. Use of a lipid composition in the manufacture of an agent for delivering nucleic acid, wherein the lipid composition comprises one or more compounds selected from the group consisting of:
| **Lipid Compound No.** | **Product Name** |
|---|---|
| 501 | methyl jasmonate |
| 502 | 6,10-dimethylundeca-5,9-dien-2-one (isomer mixture) |
| 503 | 1-methylcyclopentan-1-ol |
| 505 | tridecan-2-ol |
| 519 | N-(2-hydroxyethyl)tricosanamide |
| 520 | (7Z,10Z,13Z,16Z)-docosa-7,10,13,16-tetraenoic acid |
| 521 | N-(2-hydroxyethyl)tetracosanamide |
| 522 | 6-(6-aminohexanoylamino)hexanoic acid |
| 525 | (E)-4-oxonon-2-enal |
| 526 | (E)-hex-3-en-1-ol |
| 527 | jasmonic acid (isomer mixture) |
| 528 | 2-methylnonane |
| 529 | hexadecanedioic acid |
| 530 | 2-aminohexanedioic acid |
| 537 | capsaicin |
| 542 | (4Z,7Z,10Z,13Z,16Z)-docosa-4,7,10,13,16-pentaenoic acid |
| 543 | 12-Hydroxyjasmonic acid |
| 572 | pentadecanedioic acid |
| 573 | decanamide |
| 574 | pentadecanal |
| 578 | N-(2-hydroxyethyl)hexadecanamide |
| 593 | pentadecan-2-one |
| 594 | tetradecanal |
| 595 | (2S)-2-aminohexanedioic acid |
| 596 | 2-methylpropanedioic acid |
| 597 | nylon 6/12 (dodecanedioic acid-1,6-hexanediamine polymer) |
| 598 | 3,3-dimethylpentanedioic acid |
| 599 | hexadecanamide |
| 600 | (E)-octadec-9-en-1-ol |
| 601 | octadecanamide |
| 602 | hexadecanal |
| 603 | 4-hydroxy-4-methyloxan-2-one |
| 604 | decan-2-one |
| 605 | pentadecanoic acid |
| 606 | (E)-2-methylbut-2-enedioic acid |
| 607 | (E)-hex-3-enedioic acid |
| 608 | 2-methylbutanedioic acid |
| 609 | pentadecan-1-ol |
| 610 | pentanamide |
| 611 | dodecanamide |
| 612 | 3-acetyloxolan-2-one |
| 613 | butyl hexanoate |
| 614 | hexyl butanoate |
| 615 | decanal |
| 616 | 2-(3-oxo-2-pentylcyclopentyl)acetic acid |
| 617 | nonyl acetate |
| 618 | (2E,4E)-hepta-2,4-dienal |
| 619 | oct-1-en-3-one |
| 620 | (5Z,8Z,11Z,14Z)-icosa-5,8,11,14-tetraenoic acid |
| 621 | pent-3-en-2-one (isomer mixture) |
| 622 | 3-oxo-N-[(3S)-2-oxooxolan-3-yl]hexanamide |
| 623 | 2-oxobutanedioic acid |
| 624 | icosanoic acid |
| 625 | (2R)-2-aminohexanedioic acid |
| 626 | 2-methylidenebutanedioic acid |
| 627 | propanedioic acid |
| 628 | butanedioic acid |
| 635 | tetradecan-1-ol |
| 636 | octadecan-1-ol |
| 802 | [(2E,6E)-3,7,11-trimethyldodeca-2,6,10-trienyl] dodecanoate |
| 803 | [(2E,6E)-3,7,11-trimethyldodeca-2,6,10-trienyl] decanoate |
| 804 | [(9Z,12Z)-octadeca-9,12-dienyl] hexadecanoate |
| 805 | dodecyl (9Z,12Z,15Z)-octadeca-9,12,15-trienoate |
| 806 | [(Z)-octadec-9-enyl] (9Z,12Z,15Z)-octadeca-9,12,15-trienoate |
| 807 | (5Z,8Z,11Z,14Z)-N-(5-hydroxypentyl)icosa-5,8,11,14-tetraenamide |
| 808 | (5Z,8Z,11Z,14Z)-N-(3-hydroxypropyl)icosa-5,8,11,14-tetraenamide |
| 809 | [(2E,6E)-3,7,11-trimethyldodeca-2,6,10-trienyl] heptanoate |
| 810 | (5Z,8Z,11Z,14Z)-N-[2-(diethylamino)ethyl]icosa-5,8,11,14-tetraenamide |
| 811 | tetradecyl (9Z,12Z)-octadeca-9,12-dienoate |
| 812 | [(9Z,12Z)-octadeca-9,12-dienyl] icosanoate |
| 813 | tridecyl (Z)-hexadec-9-enoate |
| 814 | 3-methylbut-3-enyl icosanoate |
| 815 | 3-methylbut-3-enyl docosanoate |
| 816 | [(Z)-hex-3-enyl] (2R)-2-hydroxy-3-methylbutanoate |
| 817 | docosyl (Z)-tetradec-9-enoate |
| 818 | heptadecyl (Z)-hexadec-9-enoate |
| 819 | tetracosyl (Z)-hexadec-9-enoate |
| 820 | henicosyl (Z)-hexadec-9-enoate |
| 821 | pentadecyl (Z)-hexadec-9-enoate |
| 822 | nonadecyl (Z)-hexadec-9-enoate |
| 823 | docosyl (Z)-hexadec-9-enoate |
| 824 | tetradecyl (9Z,12Z,15Z)-octadeca-9,12,15-trienoate |
| 825 | tricosyl (Z)-hexadec-9-enoate |
| 826 | (5Z,8Z,11Z,14Z)-N-(4-sulfamoylphenyl)icosa-5,8,11,14- |
| | tetraenamide |
| 827 | 4-methylheptan-3-yl (Z)-hexadec-9-enoate |
| 828 | 4-methylheptan-3-yl (9Z,12Z)-octadeca-9,12-dienoate |
| 829 | 15-methylhexadecyl tetradecanoate |
| 830 | (2S)-2-(octadecanoylamino)-3-phenylpropanoic acid |
or salts, hydrates, or solvates thereof.

3. The use of any one of the preceding claims, wherein the compound derives from extracts of traditional Chinese medicine.

4. The use of any one of the preceding claims, wherein the nucleic acid comprises DNA or RNA, optionally, the DNA is selected from coding DNA or non-coding DNA, the RNA is selected from antisense nucleic acid, mRNA, lncRNA, or small RNA.

5. The use of any one of the preceding claims, wherein the nucleic acid is used for treating diseases.

6. The use of claim 5, wherein the nucleic acid is used for treating cancer, inflammation, fibrotic disease, autoimmune disease or autoinflammatory disease, bacterial infection, behavioral and psychiatric disorder, blood disease, chromosomal disease, congenital and hereditary disease, connective tissue disease, digestive disease, ear nose throat disease, endocrine disease, environmental disease, eye disease, female reproductive disease, fungal infection, heart disease, hereditary cancer syndrome, immune system disorder, kidney and urinary disease, lung disease, male reproductive disease, metabolic disorder, mouth disease, musculoskeletal disease, myelodysplastic syndrome, neonatal screening, nutritional disease, parasitic disease, rare cancer, rare disease, skin disease, or viral infection.

7. A pharmaceutical composition, comprising a lipid composition and a nucleic acid, wherein the lipid composition comprises one or more compounds of formula (I),
A-L₁-L₃-L₂-B (I)
or salts, hydrates, or solvates thereof:
wherein:
L₁ is absent, straight or branched C₁₋₂₄ alkyl, straight or branched C₂₋₂₄ alkenyl, aryl optionally substituted by alkoxy or hydroxy, or straight or branched C₁₋₂₄ alkyl optionally substituted by aralkyl;
L₂ is absent, straight or branched C₁₋₂₄ alkyl optionally substituted by amine, or straight or branched C₂₋₂₄ alkenyl;
L₃ is 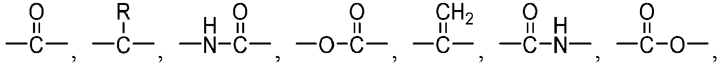 cycloalkyl optionally substituted by oxo, or heterocyclyl optionally substituted by oxo;
A is hydrogen, hydroxy, -COOH, -N(R')₂, straight or branched C₁₋₂₄ alkyl optionally substituted by hydroxy, straight or branched C₂₋₂₄ alkenyl, straight or branched C₂₋₂₄ heteroalkenyl optionally substituted by one or more substituents selected from hydroxy or amine, aryl optionally substituted by alkoxy, aminosulfonyl, or hydroxy, or heterocyclyl optionally substituted by oxo;
B is hydroxy, -CHO, -COOH, -C(O)R', -OC(O)R', straight or branched C₁₋₂₄ alkyl, straight or branched C₂₋₂₄ alkenyl;
R is hydrogen, straight or branched C₁₋₂₄ alkyl;
each R' is independently straight or branched C₁₋₂₄ alkyl.

8. A pharmaceutical composition, comprising a lipid composition and a nucleic acid, wherein the lipid composition comprises one or more compounds selected from the group consisting of lipids 501, 502, 503, 505, 519, 520, 521, 522, 525, 526, 527, 528, 529, 530, 537, 542, 543, 572, 573, 574, 578, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830, or salts, hydrates, or solvates thereof.

9. A kit, comprising:
one or more compounds of formula (I):
A-L₁-L₃-L₂-B (I)
or pharmaceutically acceptable salts, hydrates, or solvates thereof, positioned in a first container;
and a nucleic acid positioned in a second container;
wherein:
L₁ is absent, straight or branched C₁₋₂₄ alkyl, straight or branched C₂₋₂₄ alkenyl, aryl optionally substituted by alkoxy or hydroxy, or straight or branched C₁₋₂₄ alkyl optionally substituted by aralkyl;
L₂ is absent, straight or branched C₁₋₂₄ alkyl optionally substituted by amine, or straight or branched C₂₋₂₄ alkenyl;
L₃ is 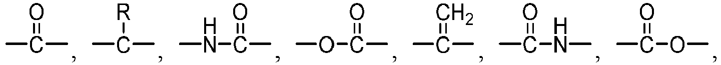 cycloalkyl optionally substituted by oxo, or heterocyclyl optionally substituted by oxo;
A is hydrogen, hydroxy, -COOH, -N(R')₂, straight or branched C₁₋₂₄ alkyl optionally substituted by hydroxy, straight or branched C₂₋₂₄ alkenyl, straight or branched C₂₋₂₄ heteroalkenyl optionally substituted by one or more substituents selected from hydroxy or amine, aryl optionally substituted by alkoxy, aminosulfonyl, or hydroxy, or heterocyclyl optionally substituted by oxo;
B is hydroxy, -CHO, -COOH, -C(O)R', -OC(O)R', straight or branched C₁₋₂₄ alkyl, straight or branched C₂₋₂₄ alkenyl;
R is hydrogen, straight or branched C₁₋₂₄ alkyl;
each R' is independently straight or branched C₁₋₂₄ alkyl.

10. A kit, comprising:
one or more compounds positioned in a first container, or pharmaceutically acceptable salts, hydrates, or solvates thereof, and a nucleic acid positioned in a second container, wherein the one or more compounds are selected from the group consisting of lipids 501, 502, 503, 505, 519, 520, 521, 522, 525, 526, 527, 528, 529, 530, 537, 542, 543, 572, 573, 574, 578, 593, 594, 595, 596, 597, 598, 599, 600, 601, 602, 603, 604, 605, 606, 607, 608, 609, 610, 611, 612, 613, 614, 615, 616, 617, 618, 619, 620, 621, 622, 623, 624, 625, 626, 627, 628, 635, 636, 802, 803, 804, 805, 806, 807, 808, 809, 810, 811, 812, 813, 814, 815, 816, 817, 818, 819, 820, 821, 822, 823, 824, 825, 826, 827, 828, 829, and 830.
